# EUROPEAN PATENT APPLICATION

(11) **EP 2 511 265 A1**
(43) Date of publication of application: **17.10.2012**
(21) Application number: 10836015.7
(22) Date of filing: 09.12.2010
(51) Int. Cl.: C07D 213/75, A61K 31/415, A61K 31/4155, A61K 31/4245, A61K 31/426, A61K 31/433, A61K 31/44, A61K 31/4439, A61K 31/455, A61K 31/4965, A61K 31/497, A61K 31/506, A61K 31/5377, A61P 3/00

(54) **BENZAMIDE COMPOUND**

(30) Priority: 11.12.2009 JP 2009281350
(71) Applicant: Astellas Pharma Inc., Tokyo 103-8411 (JP)
(72) Inventor: HAYAKAWA, Masahiko, Tokyo 103-8411 (JP); YOSHIMURA, Seiji, Tokyo 103-8411 (JP); SASUGA, Daisuke, Tokyo 103-8411 (JP); KOIKE, Takanori, Tokyo 103-8411 (JP); NIGAWARA, Takahiro, Tokyo 103-8411 (JP); OKUMURA, Mitsuaki, Tokyo 103-8411 (JP); MAKI, Keisuke, Tokyo 103-8411 (JP)
(74) Representative: Gille Hrabal
(86) International application number: PCT/JP2010/072077
(87) International publication number: WO 2011/071095

(57) **Abstract**

[Problem]

A compound which is useful as a GK activator is provided.

[Means for Solution]

The present inventors have conducted studies on compounds having a GK activating action, which are promising as active ingredients of pharmaceutical compositions for the treatment of diabetes, type 2 diabetes mellitus, obesity, metabolic syndrome and related diseases caused by the aforementioned diseases, and as a result, they have confirmed that a benzamide compound of the present invention has an excellent GK activating action, thereby completing the present invention. That is, the benzamide compound of the present invention has a GK activating action and can be used as an agent for preventing and/or treating diabetes, type 2 diabetes mellitus, obesity, metabolic syndrome, and related diseases caused by the aforementioned diseases.

## Description

### Technical Field

The present invention relates to a benzamide compound which is useful as an active ingredient of a pharmaceutical composition, for example, a pharmaceutical composition for treating diabetes.

### Background Art

GK (glucokinase (ATP: D-hexose 6-phosphotransferase, EC2.7.1.1)) is an enzyme which is expressed in the pancreas and the liver and phosphorylates hexose, and its presence in the brain has also been revealed in recent years. This enzyme belongs to a hexokinase family and is also called hexokinase IV as an alias. As compared with other hexokinases, GK has characteristics such as 1) it has low affinity for glucose as its substrate and shows a Km value close to the blood glucose concentration, 2) it is not inhibited by glucose-6-phosphate which is its enzyme reaction product, 3) it has a molecular weight of 50 kDa which is about half of those of other hexokinases, and the like. The human glucokinase gene is positioned at the 7^{th} chromosome 7p13 as a single gene and controlled by 30 kb or more distant tissue-specific different promoters in pancreatic β cells and hepatic cells and uses a different first exon but the other exons 2 to 10 are common. Accordingly, between the pancreatic and hepatic GK proteins, only the N-terminal 15 residues are different.

Accompanied by an increase in the blood glucose level, the glucose concentration in the pancreatic β cells quickly reaches its equilibrium via a glucose transporting carrier GLUT 2, and GK detects a change in the intracellular glucose concentration and activates the glycolytic system. As a result, the ATP/ADP ratio in the pancreatic β cells increases and the KATP channel is closed, and a voltage-dependent Ca channel detects this and the intracellular calcium concentration is thereby increased and release of insulin occurs. That is, GK acts as a glucose sensor in the pancreatic β cells and plays an important role in the control of insulin secretion. GK also acts as a glucose sensor in the liver, responds to the increase in the blood glucose level and converts glucose into glucose-6-phosphate. As a result, production of glycogen increases, and the glycolytic system is also activated and the gluconeogenesis in the liver is thereby inhibited.
In patients whose glucose phosphorylation ability has been reduced due to gene mutation of GK, hyperglycemia occurs frequently, and juvenile diabetes is generated (MODY 2). On the other hand, in patients who show a low value of the Km value of GK activity due to a gene mutation, hypoglycemia is recognized after meals and at the time of fasting. That is, GK also acts as a glucose sensor in humans and thereby plays an important role in maintaining normal blood glucose levels. From these facts, it is expected that an agent capable of activating GK will become an excellent agent for treating type 2 diabetes mellitus, which corrects hyperglycemia after meals by accelerating glucose-dependent insulin secretion from the pancreatic β cells, and at the same time, inhibits release of glucose from the liver. Further, there also is a possibility that excess acceleration of insulin secretion does not occur due to acceleration of glucose uptake into the liver under a hyperglycemic state after meals, and therefore that the pancreatic secondary failure as a conventional problem with sulfonylurea (SU) agents can be avoided. In addition, it has been reported in recent years that apoptosis is induced when a mouse cultured pancreatic cell (MIN6N8) is cultured under high glucose conditions. In addition, since apoptosis of the MIN6N8 was inhibited when glucokinase was over-expressed in this cell (Diabetes 54:2) 2602-2611 (2005)), it is expected that a GK activating agent will show a pancreas protective action.

The GK which exists in the brain is a pancreas type and frequently expressed in the nerve of a feeding center VMH (ventromedial hypothalamus). Glucose-sensitive nerves are classified into GE (Glucose Exited)-neurons, which is excitatory for glucose and GI (Glucose Inhibited)-neurons, which is suppressive for glucose. The presence of mRNA and protein of GK is found in about 70% of the GE-neurons and about 40% of the GI-neurons.
In these glucose-sensitive nerves, GK detects increase of the intracellular glucose and activates the glycolytic system, and thus, the intracellular ATP/ADP ratio increases. As a result, the KATP channel is closed in the GE-neuron, frequency of action potential of the neuron is increased and a neurotransmitter is released. On the other hand, it is considered that a Cl-channel is involved in the GI-neuron. In a rat in which expression of GK mRNA is increased in the VMH, a compensatory action for the glucose-deficient state is reduced.
Receptors for leptin and insulin involved in the feeding behavior are also present in the glucose-sensitive nerves. In the GE-neuron under a high glucose condition, leptin and insulin open the KATP channel and reduce the frequency of action potential. In addition, the NPY (Neuropeptide Y)-neuron which functions to promote appetite at the ARC (arcuate nucleus) is suppressive for glucose and the POMC (Proopiomelanocortin)-neuron which functions to suppress appetite is excitatory for glucose (Diabetes 53:2521-2528 (2004)). From these facts, suppression of feeding behavior is expected by activating GK of the central nervous system, which is effective for the treatment of obesity or metabolic syndrome.

A large number of compounds having the GK activation action have been reported, and there is also report of a compound whose clinical efficacy has been confirmed. However, a novel GK activator having an excellent profile regarding reduction in various side effects (actions for hERG or CYP) or solubility is in great demand.

Benzamide derivatives having a GK activation action have been reported in Patent Documents 1 to 22, but there is no specific disclosure of the compound of the present invention.

[Patent Document 1] Pamphlet of International Publication WO 2009/041475
[Patent Document 2] Pamphlet of International Publication WO 2004/076420
[Patent Document 3] Pamphlet of International Publication WO 2008/075073
[Patent Document 4] Pamphlet of International Publication WO 2008/050117
[Patent Document 5] Pamphlet of International Publication WO 2008/050101
[Patent Document 6] Pamphlet of International Publication WO 2007/060448
[Patent Document 7] Pamphlet of International Publication WO 2007/017649
[Patent Document 8] Pamphlet of International Publication WO 2007/007042
[Patent Document 9] Pamphlet of International Publication WO 2007/007040
[Patent Document 10] Pamphlet of International Publication WO 2006/040529
[Patent Document 11] Pamphlet of International Publication WO 2006/040528
[Patent Document 12] Pamphlet of International Publication WO 2006/040527
[Patent Document 13] Pamphlet of International Publication WO 2005/121110
[Patent Document 14] Pamphlet of International Publication WO 2005/080360
[Patent Document 15] Pamphlet of International Publication WO 2005/080359
[Patent Document 16] Pamphlet of International Publication WO 2005/056530
[Patent Document 17] Pamphlet of International Publication WO 2005/054233
[Patent Document 18] Pamphlet of International Publication WO 2005/054200
[Patent Document 19] Pamphlet of International Publication WO 2005/044801
[Patent Document 20] Pamphlet of International Publication WO 2003/015744
[Patent Document 21] Pamphlet of International Publication WO 2007/007041
[Patent Document 22] Pamphlet of International Publication WO 2010/092387

### Disclosure of Invention

### Problems to Be Solved by the Invention

A benzamide compound which is useful as an active ingredient of a pharmaceutical composition, for example, a pharmaceutical composition for treating diabetes, is provided.

### Means for Solving the Problems

The present inventors have made extensive studies on compounds having a GK activating action, and as a result, they have found that the benzamide compound of the present invention has a GK activating action, thereby completing the present invention.

That is, the present invention relates to a compound of the formula (I) or a salt thereof as well as a pharmaceutical composition containing a compound of the formula (I) or a salt thereof and pharmaceutically acceptable excipients. 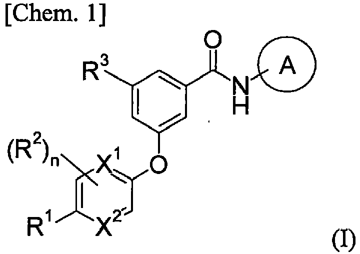 [the symbols in the formula have the following meanings:
Ring A: nitrogen-containing heteroaryl which may be substituted,
X¹ and X²: the same as or different from each other, each representing C(H), C(R²), or N,
R¹: -N(halogeno-lower alkyl)-C(O)-R¹¹, -N(lower alkylene-cycloalkyl)-C(O)-R¹¹, -N(lower alkylene-cycloalkyl)-CO₂R¹¹, -N(lower alkylene-cycloalkyl)-C(O)N(R⁰)(R¹¹), -N(lower alkylene-cycloalkyl)-S(O)₂-R¹¹, or R¹²,
R⁰: the same as or different from each other, each representing -H, or lower alkyl,
R¹¹: lower alkyl, halogeno-lower alkyl, lower alkylene-OR⁰, lower alkylene-O-lower alkylene-OR⁰, lower alkylene-OC(O)-lower alkyl, lower alkylene-CN, lower alkylene-N(R⁰)₂, lower alkylene-cycloalkyl, cycloalkyl, or a heterocyclic group,
provided that the cycloalkyl and the heterocyclic group in R¹¹ may be each substituted,
R¹²: 1,2,4-oxadiazol-3-yl, 1,2,4-oxadiazol-5-yl, or 1,3,4-oxadiazol-2-yl, each of which may be substituted with a group selected from Group G,
provided that when both of X¹ and X² are not N, 1,2,4-oxadiazol-3-yl and 1,2,4-oxadiazol-5-yl in R¹² are substituted with a group selected from Group G,
Group G: lower alkylene-CN, lower alkylene-OR⁰, lower alkylene-N(R⁰)₂, lower alkylene-C(O)N(R⁰)₂, -C(O)N(R⁰)₂, -C(O)N(R⁰)-lower alkylene-OR⁰, -C(O)N(R⁰)-cycloalkyl, -C(O)N(R⁰)-heterocyclic group, -C(O)-heterocyclic group, -N(R⁰)₂, -S(O)ₚ-lower alkyl, and cycloalkyl,
provided that the cycloalkyl and the heterocyclic group in Group G may be substituted,
R²: the same as or different from each other, each representing halogen, lower alkyl, -O-lower alkyl, or -O-halogeno-lower alkyl,
n and p: the same as or different from each other, each representing 0, 1, or 2,
R³: -H, halogen, lower alkyl, halogeno-lower alkyl, -N(R⁰)C(O)-lower alkyl, -N(lower alkylene-cycloalkyl)-C(O)-lower alkyl, or -O-R³¹, and
R³¹: -H, lower alkyl, halogeno-lower alkyl, lower alkylene-OR⁰, lower alkylene-N(R⁰)₂, lower alkylene-S(O)₂-lower alkyl, lower alkylene-C(O)N(R⁰)-S(O)₂-lower alkyl, lower alkylene-cycloalkyl, lower alkylene-aryl, cycloalkyl, or heterocyclic group,
provided that the cycloalkyl, aryl, and heterocyclic groups in R³¹ may be each substituted].

Moreover, unless specified otherwise, in the case where the symbols of the chemical formulae in the present specification are also used in other chemical formulae, the same symbols denote the same meanings.

Furthermore, the present invention relates to a GK activator containing a compound of the formula (I) or a salt thereof. Further, in another embodiment, the present invention relates to a pharmaceutical composition for preventing and/or treating diabetes, type 2 diabetes mellitus, obesity, or metabolic syndrome, which contains a compound of the formula (I) or a salt thereof. In addition, the pharmaceutical composition includes an agent for preventing and/or treating diabetes, type 2 diabetes mellitus, obesity, or metabolic syndrome, which contains a compound of the formula (I) or a salt thereof.
Furthermore, the present invention relates to use of a compound of the formula (I) or a salt thereof for preparation of a GK activator, or a pharmaceutical composition for preventing or treating diabetes, type 2 diabetes mellitus, obesity, or metabolic syndrome, a compound of the formula (I) or a salt thereof to be used for preventing or treating diabetes, type 2 diabetes mellitus, obesity, or metabolic syndrome, and a method for preventing and/or treating diabetes, type 2 diabetes mellitus, obesity, or metabolic syndrome including administering an effective amount of a compound of the formula (I) or a salt thereof to a subject. Further, the "subject" is a human or another animal in need of such prevention or treatment, and in a certain embodiment, a human in need of such prevention or treatment.
That is, the present invention also relates to
(1) a pharmaceutical composition comprising a compound of the formula (I) or a salt thereof and pharmaceutically acceptable excipients;
(2) the pharmaceutical composition according to (1), which is a GK activator;
(3) the pharmaceutical composition according to (1), which is a drug for preventing and/or treating diabetes;
(4) the pharmaceutical composition according to (3), which is a drug for preventing and/or treating type 2 diabetes mellitus;
(5) the pharmaceutical composition according to (1), which is a drug for preventing and/or treating obesity;
(6) the pharmaceutical composition according to (1), which is a drug for preventing and/or treating metabolic syndrome;
(7) use of a compound of the formula (I) or a pharmaceutically acceptable salt thereof for the preparation of a GK activator, or a drug for preventing and/or treating diabetes, obesity, or metabolic syndrome;
(8) use of a compound of the formula (I) or a pharmaceutically acceptable salt thereof for preventing and/or treating diabetes, obesity, or metabolic syndrome;
(9) a method for preventing and/or treating diabetes, obesity, or metabolic syndrome, comprising administering to a patient a therapeutically effective amount of a compound of the formula (I) or a salt thereof;
(10) a compound of the formula (I) or a pharmaceutically acceptable salt thereof for preventing and/or treating diabetes, obesity, or metabolic syndrome.

### Effects of the Invention

A compound of the formula (I) or a salt thereof has a GK activating action, and can be used as an active ingredient of a pharmaceutical composition for preventing and/or treating diabetes, particularly type 2 diabetes mellitus. Further, it can be used as an active ingredient of a pharmaceutical composition for preventing and/or treating nephropathy, retinopathy, neuropathy, peripheral circulatory disorder, cerebrovascular disorder, ischemic heart disease, and arteriosclerosis that are complications of diabetes mellitus. It can further be used as an active ingredient of a pharmaceutical composition for preventing and/or treating obesity or metabolic syndrome by suppressing overeating.

### Embodiments for Carrying Out the Invention

Hereinafter, the present invention will be described in detail.

In the present specification, the "lower alkyl" preferably refers to linear or branched alkyl having 1 to 6 carbon atoms (hereinafter simply referred to as C₁₋₆), specifically methyl, ethyl, n-propyl, isopropyl, n-butyl, isobutyl, sec-butyl, tert-butyl, n-pentyl, n-hexyl, or the like, more preferably C₁₋₄ alkyl, and particularly preferably methyl, ethyl, normal propyl, isopropyl, or tert-butyl.

The "lower alkylene" preferably refers to linear or branched C₁₋₆ alkylene, specifically, methylene, ethylene, trimethylene, tetramethylene, pentamethylene, hexamethylene, propylene, methylmethylene, ethylethylene, 1,2-dimethylethylene, a 1,1,2,2-tetramethylethylene group, or the like, more preferably C₁₋₄ alkylene, and still more preferably methylene, ethylene, or trimethylene.

The "halogen" means F, Cl, Br, or I.
The "halogeno-lower alkyl" refers to C₁₋₆ alkyl substituted with one or more halogen atoms, preferably a lower alkyl substituted with 1 to 5 halogen atoms, and more preferably fluoromethyl, difluoromethyl, or trifluoromethyl.

The "cycloalkyl" refers to a C₃₋₁₀ saturated hydrocarbon ring group, which may have a bridge. It is specifically cyclopropyl, cyclobutyl, cyclopentyl, cyclohexyl, cycloheptyl, cyclooctyl, an adamantyl group, or the like, preferably C₃₋₈ cycloalkyl, more preferably cyclopropyl, cyclobutyl, cyclopentyl, or cyclohexyl, and still more preferably cyclopropyl.

The "aryl" refers a C₆₋₁₄ monocyclic to tricyclic aromatic hydrocarbon ring group, more preferably phenyl or naphthyl, and still more preferably phenyl.

"Heteroaryl" group means a 5- to 15-membered, preferably 5- to 10-membered, monocyclic to tricyclic aromatic heterocyclic group containing 1 to 4 hetero atoms selected from oxygen, sulfur, and nitrogen. Sulfur or nitrogen which is a ring atom may be oxidized to form an oxide. Specific examples of the heteroaryl group include pyrrolyl, pyrazolyl, imidazolyl, triazolyl, tetrazolyl, furyl, oxazolyl, isoxazolyl, oxadiazolyl, thienyl, thiazolyl, isothiazolyl, thiadiazolyl, pyridyl, pyridazinyl, pyrimidinyl, pyrazinyl, triazinyl, indolyl, isoindolyl, indazolyl, benzimidazolyl, benzofuryl, benzoxazolyl, benzothienyl, benzothiazolyl, [1,3]thiazolo[5,4-b]pyridinyl, quinolyl, isoquinolyl, quinazolinyl, quinoxalinyl, cinnolinyl, phthalazinyl, naphthylizinyl, carbazolyl, and the like, preferably a 5- to 6-membered monocyclic heteroaryl, and more preferably pyrazolyl, thiazolyl, thiadiazolyl, pyridyl, and pyrazinyl.

The "nitrogen-containing heteroaryl" group means heteroaryl having at least one nitrogen atom among the aforementioned "heteroaryl". Specific examples thereof include pyrrolyl, pyrazolyl, imidazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, triazinyl, indolyl, isoindolyl, indazolyl, benzimidazolyl, benzoxazolyl, benzothiazolyl, [1,3]thiazolo[5,4-b]pyridinyl, quinolyl, isoquinolyl, quinazolinyl, quinoxalinyl, cinnolinyl, phthalazinyl, naphthylizinyl, carbazolyl, and the like, preferably a 5- to 6-membered monocyclic nitrogen-containing heteroaryl, and more preferably pyrazolyl, thiazolyl, thiadiazolyl, pyridyl, and pyrazinyl.

The "heterocyclic" group refers to a 3- to 15-membered, preferably 5- to 10-membered, monocyclic to tricyclic heterocyclic group containing 1 to 4 hetero atoms selected from oxygen, sulfur, and nitrogen, which contains a saturated ring, an aromatic ring, and a partially hydrogenated ring group thereof and may have a bridge. Sulfur or nitrogen which is a ring atom may be oxidized to form an oxide or a dioxide. Specific examples of the heterocyclic group include aziridinyl, azetidinyl, pyrrolidinyl, imidazolidinyl, piperidinyl, piperazinyl, morpholinyl, azepanyl, diazepanyl, tetrahydrofuranyl, tetrahydropyranyl, dioxolanyl, pyrrolyl, pyrazolyl, imidazolyl, triazolyl, tetrazolyl, furyl, oxazolyl, isoxazolyl, oxadiazolyl, thienyl, thiazolyl, isothiazolyl, thiadiazolyl, pyridyl, pyridazinyl, pyrimidinyl, pyrazinyl, triazinyl, indolyl, isoindolyl, indazolyl, benzimidazolyl, benzofuryl, benzoxazolyl, benzothienyl, benzothiazolyl, [1,3]thiazolo[5,4-b]pyridinyl, quinolyl, isoquinolyl, quinazolinyl, quinoxalinyl, cinnolinyl, phthalazinyl, naphthylizinyl, carbazolyl, indolinyl, tetrahydroquinolyl, tetrahydroisoquinolyl, methylenedioxyphenyl, an ethylenedioxyphenyl group, and the like, preferably a 5- to 9-membered monocyclic to bicyclic heterocyclic group, more preferably a 5- to 6-membered monocyclic heterocyclic group, and still more preferably pyrazolyl, isoxazolyl, oxadiazolyl, thiazolyl, thiadiazolyl, pyridyl, pyrimidinyl, pyrazinyl, tetrahydrofuranyl, tetrahydropyranyl, dioxolanyl, pyrrolidinyl, imidazolidinyl, and piperidinyl.

The expression "which may be substituted" represents "which is not substituted" or "which is substituted with 1 to 5 substituents, which are the same as or different from each other". Further, the expression "which is substituted" represents "which has 1 to 5 substituents, which are the same as or different from each other". If there are a plurality of substituents, the substituents may be the same as or different from each other.

The substituent of the "nitrogen-containing heteroaryl" which may be substituted in the ring A is preferably a group selected from Group G¹, more preferably lower alkyl which may be substituted with a hydroxyl group, and still more preferably lower alkyl.
Group G¹: halogen, lower alkyl which may be substituted with -OR⁰, halogeno-lower alkyl, -OR⁰, -O-halogeno-lower alkyl, -CO₂R⁰, or a heterocyclic group,
provided that the heterocyclic group in the group G¹ may be substituted with a group selected from the group consisting of lower alkyl, halogeno-lower alkyl, -OR⁰, -O-halogeno-lower alkyl, and oxo.

The substituent of the "cycloalkyl" and the "heterocyclic group", each of which may be substituted, in R¹¹ is preferably a group selected from the group consisting of lower alkyl, halogeno-lower alkyl, -OR⁰, -O-halogeno-lower alkyl, and oxo.

The substituent of the "cycloalkyl" and the "heterocyclic group", each of which may be substituted, in Group G is preferably a group selected from the group consisting of lower alkyl, halogeno-lower alkyl, -OR⁰, -O-halogeno-lower alkyl, and oxo.

The substituent of the "cycloalkyl", "aryl", and the "heterocyclic group", each of which may be substituted, in R³¹ is preferably a group selected from the group consisting of halogen, lower alkyl, -OR⁰, -O-halogeno-lower alkyl, lower alkylene-OR⁰, and lower alkylene-O-lower alkylene-aryl.

Certain embodiments of the compound of the formula (I) or a salt thereof are shown below.

(1) The compound or a salt thereof, wherein Ring A is pyrazol-3-yl, thiazol-2-yl, or 1,2,4-thiadiazol-5-yl, each of which may be substituted with lower alkyl which may be substituted with a hydroxyl group, in another embodiment, the compound or a salt thereof, wherein Ring A is pyrazol-3-yl or 1-methylpyrazol-3-yl, and in a still another embodiment, the compound or a salt thereof, wherein Ring A is 1-methylpyrazol-3-yl.
(2) (2-1) The compound or a salt thereof, wherein R¹ is R¹², in another embodiment, the compound or a salt thereof, wherein R¹ is 1,2,4-oxadiazol-3-yl, 1,2,4-oxadiazol-5-yl, or 1,3,4-oxadiazol-2-yl, each of which is substituted with a group selected from the group consisting of lower alkylene-OR⁰, -C(O)N(R⁰)₂, -C(O)N(R⁰)-lower alkylene-OR⁰, and -C(O)N(R⁰)-cycloalkyl, in a still another embodiment, the compound or a salt thereof, wherein R¹ is 1,2,4-oxadiazol-3-yl, 1,2,4-oxadiazol-5-yl, or 1,3,4-oxadiazol-2-yl, which is substituted with carbamoyl which may be substituted with one or two lower alkyl groups (in which the lower alkyl may be substituted with a hydroxyl group), in a further still another embodiment, the compound or a salt thereof, wherein R¹ is 1,2,4-oxadiazol-3-yl, 1,2,4-oxadiazol-5-yl, or 1,3,4-oxadiazol-2-yl, which is substituted with a group selected from the group consisting of carbamoyl, methylcarbamoyl, dimethylcarbamoyl, and 2-hydroxyethylcarbamoyl, in a further still another embodiment, the compound or a salt thereof, wherein R¹ is 1,2,4-oxadiazol-3-yl, which is substituted with a group selected from the group consisting of carbamoyl, methylcarbamoyl, dimethylcarbamoyl, and 2-hydroxyethylcarbamoyl, in a further still another embodiment, the compound or a salt thereof, wherein R¹ is 1,2,4-oxadiazol-5-yl which is substituted with a group selected from the group consisting of carbamoyl, methylcarbamoyl, dimethylcarbamoyl, and 2-hydroxyethylcarbamoyl, in a further still another embodiment, the compound or a salt thereof, wherein R¹ is 1,3,4-oxadiazol-2-yl which is substituted with a group selected from the group consisting of carbamoyl, methylcarbamoyl, dimethylcarbamoyl, and 2-hydroxyethylcarbamoyl. (2-2) The compound or a salt thereof, wherein R¹ is -N(lower alkylene-cycloalkyl)-C(O)-R¹¹, in another embodiment, the compound or a salt thereof, wherein R¹ is -N(cyclopropylmethyl)-C(O)-R¹¹, in a further still another embodiment, the compound or a salt thereof, wherein R¹ is - N(cyclopropylmethyl)-C(O)-R¹¹, and R¹¹ is lower alkylene-O-lower alkylene-OR⁰ or lower alkylene-N(R⁰)₂, in a further still another embodiment, the compound or a salt thereof, wherein R¹ is -N(cyclopropylmethyl)-C(O)-R¹¹, and R¹¹ is 2-methoxyethoxymethyl or N,N-dimethylaminomethyl, in a further still another embodiment, the compound or a salt thereof, wherein R¹ is -N(cyclopropylmethyl)-C(O)-R¹¹, and R¹¹ is a heterocyclic group which may be substituted with a group selected from the group consisting of lower alkyl, halogeno-lower alkyl, -O-R⁰, -O-halogeno-lower alkyl, and oxo, in a further still another embodiment, the compound or a salt thereof, wherein R¹ is -N(cyclopropylmethyl)-C(O)-R¹¹, and R¹¹ is tetrahydrofuran-2-yl, tetrahydropyran-4-yl, pyrazin-2-yl, or 2-oxoimidazolidin-1-yl, in a further still another embodiment, the compound or a salt thereof, wherein R¹ is -N(cyclopropylmethyl)-C(O)-R¹¹, and R¹¹ is tetrahydrofuran-2-yl or tetrahydropyran-4-yl, in a further still another embodiment, the compound or a salt thereof, wherein R¹ is -N(cyclopropylmethyl)-C(O)-R¹¹, and R¹¹ is pyrazin-2-yl, and in a further still another embodiment, the compound or a salt thereof, wherein R¹ is - N(cyclopropylmethyl)-C(O)-R¹¹, and R¹¹ is 2-oxoimidazolidin-1-yl.

(3)The compound or a salt thereof, wherein n is 0.
(4) The compound or a salt thereof, wherein X¹ is N and X² is N, in another embodiment, the compound or a salt thereof, wherein X¹ is C(H) and X² is N, and in a still another embodiment, the compound or a salt thereof, wherein X¹ is N and X² is C(H).
(5) (5-1) The compound or a salt thereof, wherein R³ is -O-R³¹, in another embodiment, the compound or a salt thereof, wherein R³ is -O-lower alkyl, -O-halogeno-lower alkyl, -O-lower alkylene-OR⁰, -O-(cycloalkyl lower which may be substituted with lower alkylene-OR⁰), or -O-lower alkylene-aryl, in a still another embodiment, the compound or a salt thereof, wherein R³ is -O-(lower alkyl) or -O-(lower alkylene-OR°), in a further still another embodiment, the compound or a salt thereof, wherein R³ is isopropoxy, 1-hydroxypropan-2-yloxy, or 1-methoxypropan-2-yloxy, in a further still another embodiment, the compound or a salt thereof, wherein R³ is isopropoxy, in a further still another embodiment, the compound or a salt thereof, wherein R³ is 1-hydroxypropan-2-yloxy, and in a further still another embodiment, the compound or a salt thereof, wherein R³ is 1-methoxypropan-2-yloxy. (5-2) The compound or a salt thereof, wherein R³ is - N(lower alkylene-cycloalkyl)-C(O)-lower alkyl, in another embodiment, the compound or a salt thereof, wherein R³ is -N(cyclopropylmethyl)-C(O)-lower alkyl, and in a still another embodiment, the compound or a salt thereof, wherein R³ is N-cyclopropylmethyl-N-acetylamino.

(6) The compounds or salts thereof including the combinations of two or more of the groups as described in (1) to (5).

Furthermore, examples of specific embodiments including the combinations of two or more of the groups as described in (1) to (5) as described in (6) include (a) to (f) below.
(a) The compound of the formula (I) or a salt thereof, wherein R¹ is -N(lower alkylene-cycloalkyl)-C(O)-R¹¹ or R¹².
(b) The compound of the formula (I) or a salt thereof as described in (a), wherein Ring A is pyrazol-3-yl, thiazol-2-yl, or 1,2,4-thiadiazol-5-yl, each of which may be substituted with lower alkyl which may be substituted with a hydroxyl group.
(c) The compound of the formula (I) or a salt thereof as described in (b), wherein R³ is -O-lower alkyl, -O-halogeno-lower alkyl, -O-lower alkylene-OR⁰, -O-(cycloalkyl which may be substituted with lower alkylene-OR⁰), -O-lower alkylene-aryl, or -N(lower alkylene-cycloalkyl)-C(O)-lower alkyl.
(d) The compound as described in (c), wherein X¹ is C(H) and X² is N, or X¹ is N and X² is N.
(e) The compound as described in (d), wherein n is 0.
(f) The compound as described in (e), wherein R¹ is 1,2,4-oxadiazol-3-yl, 1,2,4-oxadiazol-5-yl, or 1,3,4-oxadiazol-2-yl, each of which is substituted with a group selected from the group consisting of lower alkylene-OR⁰, -C(O)N(R⁰)₂, -C(O)N(R⁰)-lower alkylene-OR⁰, and -C(O)N(R⁰)-cycloalkyl.
(f) The compound as described in (e), wherein R¹ is N(cyclopropylmethyl)-C(O)-lower alkylene-O-lower alkylene-OR⁰, -N(cyclopropylmethyl)-C(O)-lower alkylene-N(R⁰)₂, or -N(cyclopropylmethyl)-C(O)-(heterocyclic group which may be substituted with a group selected from the group consisting of lower alkyl, halogeno-lower alkyl, -O-R⁰, - O-halogeno-lower alkyl, and oxo).

Examples of the specific compounds included in the compound of the formula (I) or a salt thereof include the following compounds:
3-(5- {3-isopropoxy-5-[(1-methyl-1H-pyrazol-3-yl)carbamoyl]phenoxy}pyrazin-2-yl)-N,N-dimethyl-1,2,4-oxadiazole-5-carboxamide,
N-(cyclopropylmethyl)-N-(5-{3-isopropoxy-5-[(1-methyl-1H-pyrazol-3-yl)carbamoyl]phenoxy}pyrazin-2-yl)-2-oxoimidazolidine-1-carboxamide,
N-(cyclopropylmethyl)-N-(5-{3-isopropoxy-5-[(1-methyl-1H-pyrazol-3-yl)carbamoyl]phenoxy}pyrazin-2-yl)pyrazine-2-carboxamide,
N-(cyclopropylmethyl)-N-(5-{3-isopropoxy-5-[(1-methyl-1H-pyrazol-3-yl)carbamoyl]phenoxy}pyrazin-2-yl)tetrahydro-2H-pyran-4-carboxamide,
5-(5-{3-isopropoxy-5-[(1-methyl-1H-pyrazol-3-yl)carbamoyl]phenoxy}pyrazin-2-yl)-N,N-dimethyl-1,3,4-oxadiazole-2-carboxamide,
3-[(5-{(cyclopropylmethyl)[(2-methoxyethoxy)acetyl]amino}pyrazin-2-yl)oxy]-5-isopropoxy-N-(1-methyl-1H-pyrazol-3-yl)benzamide,
(2R)-N-(cyclopropylmethyl)-N-(5-{3-isopropoxy-5-[(1-methyl-1H-pyrazol-3-yl)carbamoyl]phenoxy}pyrazin-2-yl)tetrahydrofuran-2-carboxamide,
5-(5-{3-isopropoxy-5-[(1-methyl-1H-pyrazol-3-yl)carbamoyl]phenoxy}pyridin-2-yl)-1,2,4-oxadiazole-3-carboxamide,
5-(5- {3-isopropoxy-5-[(1-methyl-1H-pyrazol-3-yl)carbamoyl]phenoxy}pyridin-2-yl)-N-methyl-1,2,4-oxadiazole-3-carboxamide,
5-(5-{3-isopropoxy-5-[(1-methyl-1H-pyrazol-3-yl)carbamoyl]phenoxy}pyridin-2-yl)-N,N-dimethyl-1,2,4-oxadiazole-3-carboxamide,
N-(5-{3-[acetyl(cyclopropylmethyl)amino]-5-[(1-methyl-1H-pyrazol-3-yl)carbamoyl]phenoxy}pyrazin-2-yl)-N-(cyclopropylmethyl)tetrahydro-2H-pyran-4-carboxamide,
5-(5-{3-isopropoxy-5-[(1-methyl-1H-pyrazol-3-yl)carbamoyl]phenoxy}pyridin-2-yl)-N-methyl-1,3,4-oxadiazole-2-carboxamide,
N-(2-hydroxyethyl)-5-(5-{3-isopropoxy-5-[(1-methyl-1H-pyrazol-3-yl)carbamoyl]phenoxy}pyridin-2-yl)-1,2,4-oxadiazole-3-carboxamide,
3-[5-(3-{[(2S)-1-methoxypropan-2-yl]oxy}-5-[(1-methyl-1H-pyrazol-3-yl)carbamoyl]phenoxy)pyridin-2-yl]-N,N-dimethyl-1,2,4-oxadiazole-5-carboxamide,
5-(5-{3-isopropoxy-5-[(1-methyl-1H-pyrazol-3-yl)carbamoyl]phenoxy}pyrazin-2-yl)-N-methyl-1,2,4-oxadiazole-3-carboxamide,
N-(cyclopropylmethyl)-N-[5-(3-{[(2S)-1-hydroxypropan-2-yl]oxy}-5-[(1-methyl-1H-pyrazol-3-yl)carbamoyl]phenoxy)pyrazin-2-yl]tetrahydro-2H-pyran-4-carboxamide,
5-[5-(3- {[(2S)-1-methoxypropan-2-yl]oxy}-5-[(1-methyl-1H-pyrazol-3-yl)carbamoyl]phenoxy)pyridin-2-yl]-1,2,4-oxadiazole-3-carboxamide,
5-[5-(3-{[(2S)-1-hydroxypropan-2-yl]oxy}-5-[(1-methyl-1H-pyrazol-3-yl)carbamoyl]phenoxy)pyridin-2-yl]-1,2,4-oxadiazole-3-carboxamide,
3-[(5-{(cyclopropylmethyl)[(2-methoxyethoxy)acetyl]amino}pyrazin-2-yl)oxy]-5-{[(2S)-1-hydroxypropan-2-yl]oxy}-N-(1-methyl-1H-pyrazol-3-yl)benzamide,
3-({5-[(cyclopropylmethyl)(N,N-dimethylglycyl)amino]pyrazin-2-yl}oxy)-5-{[(2S)-1-methoxypropan-2-yl]oxy}-N-(1-methyl-1H-pyrazol-3-yl)benzamide,
(2R)-N-(cyclopropylmethyl)-N-[5-(3-{[(2S)-1-hydroxypropan-2-yl]oxy}-5-[(1-methyl-1H-pyrazol-3-yl)carbamoyl]phenoxy)pyrazin-2-yl]tetrahydrofuran-2-carboxamide, and
salts thereof.

The compound of the formula (I) may exist in the form of tautomers or geometrical isomers depending on the kind of substituents. In the present specification, the compound of the formula (I) shall be described in only one form of isomer, yet the present invention includes other isomers, isolated forms of the isomers, or a mixture thereof.
In addition, the compound of the formula (I) may have asymmetric carbon atoms or axial asymmetry in some cases, and it may exist in the form of optical isomers based thereon. The present invention includes both isolated optical isomers of the compound of the formula (I) and a mixture thereof.

Moreover, the present invention also includes a pharmaceutically acceptable prodrug of the compound represented by the formula (I). The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1985) and "Pharmaceutical Research and Development" (Hirokawa Publishing Company, 1990), Vol. 7, Drug Design, 163-198.

Furthermore, the salt of the compound of the formula (I) is a pharmaceutically acceptable salt of the compound of the formula (I) and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or with organic bases such as methylamine, ethylamine, ethanolamine, lysine, ornithine, and the like, salts with various amino acids and amino acid derivatives such as acetylleucine and the like, or ammonium salts, etc.

In addition, the present invention also includes various hydrates or solvates, and polymorphic crystalline substances of the compound of the formula (I) and salts thereof. In addition, the present invention also includes compounds labeled with various radioactive or non-radioactive isotopes.

### (Preparation Methods)

The compound of the formula (I) and a salt thereof can be prepared using the characteristics based on the basic structure or the type of substituents thereof and by applying various known synthesis methods. During the preparation, replacing the relevant functional group with a suitable protective group (a group that can be easily converted into the relevant functional group) at the stage from starting material to an intermediate may be effective depending on the type of the functional group in the preparation technology in some cases. The protective group for such a functional group may include, for example, the protective groups described in "Greene's Protective Groups in Organic Synthesis (4th Ed., 2006)", P. G M. Wuts and T. W. Greene, and one of these may be suitably selected and used depending on the reaction conditions. In this kind of method, a desired compound can be obtained by introducing the protective group, by carrying out the reaction and by eliminating the protective group as necessary.
In addition, the prodrug of the compound of the formula (I) can be prepared by introducing a specific group at the stage from a starting material to an intermediate, just as in the case of the above-mentioned protective group or by further carrying out the reaction using the obtained compound of the formula (I). The reaction can be carried out using methods known to those skilled in the art, such as ordinary esterification, amidation, dehydration, and the like.

Hereinbelow, the representative preparation methods for the compound of the formula (I) will be described. Further, the preparation methods of the present invention are not limited to the examples as shown below.
Furthermore, a desired functional group can be introduced by carrying out functional group conversion that is apparent to a person skilled in the art, such as amidation, alkylation, hydrolysis, esterification, and the like, with the prepared compounds or at the stage for a preparation intermediate, or by carrying out the process in any order apparent to a person skilled in the art. For example, in the following first production process, R¹ can be introduced into the compound of the present invention by preparing 1,2,4-oxadiazol-3-yl, 1,2,4-oxadiazol-5-yl, or 1,3,4-oxadiazol-2-yl, each of which may be substituted with lower alkyl ester, and then converting lower alkyl ester into amide. Further, for example, in the following first production process, R¹ can be introduced into the compound of the present invention by preparing a secondary amino substituted with halogeno-lower alkyl or lower alkylene-cycloalkyl, and then introducing carbonyl or sulfonyl into amino. In addition, for example, in the following first production process, an amide substituent on a substituted benzene ring of R³ can be introduced into the compound of the present invention by preparing carboxylic acid or lower alkyl ester, and then converting carboxylic acid or lower alkyl ester into amide.

### (Production Process 1)

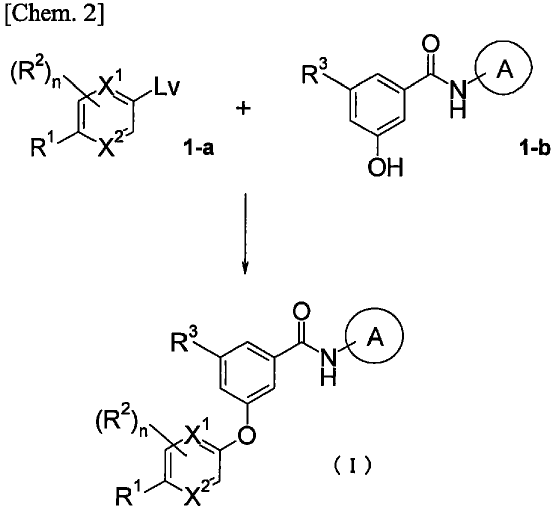

(wherein Lv represents a leaving group).

The present preparation is a method for preparing the compound (I) of the present invention by the reaction of a compound 1-a with a compound 1-b.
Here, examples of the leaving group include halogen atoms such as fluorine, chlorine, bromine, and the like; lower alkanesulfonyloxy which may be substituted, such as methanesulfonyloxy, ethanesulfonyloxy, trifluoromethanesulfonyloxy, and the like; benzenesulfonyloxy which may be substituted, such as benzenesulfonyloxy, 4-methylbenzenesulfonyloxy, 4-bromobenzenesulfonyloxy, 4-nitrobenzenesulfonyloxy, and the like; etc.

In this reaction, the compound 1-a and the compound 1-b in equivalent amounts, or either thereof in an excess amount are used, and a mixture thereof is stirred in a range of from cooling to heating to reflux, in a certain embodiment at 0°C to 80°C, usually for about 0.1 hours to 5 hours, in a solvent which is inert to the reaction or without a solvent. The solvent as used herein is not particularly limited, but examples thereof include aromatic hydrocarbons such as benzene, toluene, xylene, and the like, ethers such as diethyl ether, tetrahydrofuran (THF), dioxane, dimethoxyethane, and the like, halogenated hydrocarbons such as dichloromethane, 1,2-dichloroethane, chloroform, and the like, N,N-dimethylformamide (DMF), dimethylsulfoxide (DMSO), ethyl acetate, acetonitrile, and a mixture thereof. It may be in some cases advantageous for smooth progress of the reaction to carry out the reaction in the presence of organic bases such as triethylamine, N,N-diisopropylethylamine, N-methylmorpholine, and the like, or inorganic bases such as potassium carbonate, sodium carbonate, potassium hydroxide, and the like.

Further, in the case where R¹ in the compound 1-a or the compound (I) is 1,2,4-oxadiazol-3-yl, 1,2,4-oxadiazol-5-yl, or 1,3,4-oxadiazol-2-yl, each of which may be substituted, preparation can be conducted using a cyclization reaction that is apparent to a person skilled in the art from the corresponding carboxylic acid derivative. Specifically, the methods described in Preparation Examples or Examples as described later may be exemplified, and modified methods thereof, methods equivalent thereto, or the like may also be employed. That is, conversion from the carboxylic acid derivative into 1,2,4-oxadiazol-3-yl, 1,2,4-oxadiazol-5-yl, or 1,3,4-oxadiazol-2-yl may be carried out before the step of the first production process or after the step of the first production process.

In the preparation method, the starting compound can be prepared by using, for example, the method described in Preparation Examples as described later, a known method, or a modified method thereof.

The compounds of the formula (I) can be isolated and purified as free compounds, salts, hydrates, solvates, or polymorphic crystalline substances thereof. The salts of the compound of the formula (I) can be prepared by carrying out the treatment of a conventional salt forming reaction.
Isolation and purification are carried out by employing ordinary chemical operations such as extraction, fractional crystallization, various types of fractional chromatography, and the like.
Various isomers can be prepared by selecting an appropriate starting compound or separated by using the difference in the physicochemical properties between the isomers. For example, the optical isomers can be obtained by means of a general optical resolution method for racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) was confirmed by the tests shown below.
Test Example 1 Measurement of Glucokinase (GK) Activation
The present test was carried out in accordance with the method described in Science 301: 370-373, 2003 with partial modification, and is summarized as below.
First, cloning of GK (GenBank No. AK122876) was carried out. PCR was carried out using 5'-TAGAATTCATGGCGATGGATGTCACAAG-3' (SEQ ID NO: 1) and 5'-ATCTCGAGTCACTGGCCCAGCATACAG-3' (SEQ ID NO: 2) as primers, and pME18S-FL3-Glucokinase isoform 2 as a template. The resulting reaction product was TA-cloned into a pGEM-T easy vector, and then digested with EcoRI and XhoI, and the fragment was ligated to a pGEX-SX-1 vector digested in the same manner. Using this plasmid DNA (pGEX-human Glucokinase 2), a recombinant human liver type GK (GST-hGK2) fused with GST (Glutathione S-transferase) was expressed in E. coli (strain BL21), and purified by Glutathione Sepharose.
The reaction of GK was carried out at 27°C on a 96-well plate. First, 1 µL of a test compound diluted with dimethylsulfoxide (DMSO) (final concentration of 10 µM) was added to a 89 µL of an enzyme mixed liquid (25 mM HEPES pH 7.4, 25 mM KCl, 2 mM MgCl₂, 1 mM ATP, 0.1 % BSA, 1 mM DTT, 1 mM NADP (nicotinamide adenine dinucleotide phosphate), 2.5 U/mL glucose-6-phosphate dehydrogenase, GST-hGK2 adjusted to ΔOD_{Cont} as described later to be about 0.05 to 0.10, all of which are final concentrations). Subsequently, 10 µL of a substrate solution (final concentration of 5 mM glucose) was added thereto to initiate the reaction. In order to quantify a final product NADPH, an absorbance at a wavelength of 340 nm was measured, and the GK activation (%) of the test compound was calculated from the increase (ΔOD) in the absorbance during the first 10 minutes after the initiation of the reaction, using the following formula.
GK activation (%)=[(ΔOD_{Test})-(ΔOD_{Cont})]/(ΔOD_{Cont})×100
ΔOD_{Test}: ΔOD at the test compound
ΔOD_{Cont}: ΔOD at DMSO
The results of the measurement are shown in Table 1. Further, Ex indicates Example compound Number.

**[Table 1]**

| Ex | GK activation (%) | Ex | GK activation (%) | Ex | GK activation (%) |
|---|---|---|---|---|---|
| 1 | 295 | 29 | 287 | 119 | 376 |
| 2 | 338 | 67 | 498 | 128 | 338 |
| 3 | 380 | 74 | 298 | 130 | 502 |
| 9 | 319 | 75 | 276 | 138 | 316 |
| 10 | 380 | 98 | 498 | 156 | 237 |
| 23 | 347 | 101 | 345 | 158 | 237 |
| 24 | 269 | 110 | 338 | 162 | 306 |

### Test Example 2 Oral Glucose Loading Test in Normal Mice

The test compound was dissolved in a solvent (5% Cremophor, 5% aqueous DMSO solution), 10 mg/kg of the test compound was orally administered to ICR mice after fasting overnight, and after 30 minutes, oral glucose loading was carried out. Immediately before the administration of the test compound, immediately before the glucose loading, and 0.25, 0.5, 1, and 2 hours after the glucose loading, blood was collected and blood glucose levels were measured. An AUC lowering rate (%) of the blood glucose levels from immediately before the glucose loading to 2 hours after the glucose loading with respect to that of the solvent control was calculated.
The results are shown in Table 2.

**[Table 2]**

| Ex | Blood glucose level lowering rate (%) | Ex | Blood glucose level lowering rate (%) | Ex | Blood glucose level lowering rate (%) |
|---|---|---|---|---|---|
| 1 | 37 | 29 | 49 | 119 | 40 |
| 2 | 59 | 67 | 57 | 128 | 47 |
| 3 | 64 | 74 | 45 | 130 | 65 |
| 9 | 46 | 75 | 48 | 138 | 61 |
| 10 | 61 | 98 | 54 | 156 | 18 |
| 23 | 52 | 101 | 48 | 158 | 29 |
| 24 | 49 | 110 | 43 | 162 | 25 |

### Test Example 3 Oral Glucose Loading Test in High Fat Diet Loading Mice

The test compound is dissolved in a solvent (5% Cremophor, 5% aqueous DMSO solution), and orally administered to C57BL/6 mice after loading a high fat diet for about 30 days and then fasting overnight, and after 30 minutes, oral glucose loading was carried out. Immediately before the administration of the test compound, immediately before the glucose loading, and 0.25, 0.5, 1, and 2 hours after the glucose loading, blood is collected and blood glucose levels were measured. An AUC lowering rate (%) of the blood glucose levels from immediately before the glucose loading to 2 hours after the glucose loading with respect to that of the solvent control is calculated.
As described above, a glucose level lowering action of the compound of the present invention on the high fat diet loading mice can be evaluated.

### Test Example 4 Oral Glucose Loading Test in Normal Rats

The test compound was dissolved in a solvent (5% Cremophor, 5% aqueous DMSO solution), 3 mg/kg of the test compound was orally administered to SD rats after fasting overnight, and after 30 minutes, oral glucose loading was carried out. Immediately before the administration of the test compound, immediately before the glucose loading, and 0.5, 1, and 2 hours after the glucose loading, blood was collected and blood glucose levels were measured. An AUC lowering rate (%) of the blood glucose levels from immediately before the glucose loading to 2 hours after the glucose loading with respect to that of the solvent control was calculated.
The results are shown in Table 3.

**[Table 3]**

| Ex | Blood glucose level lowering rate (%) |
|---|---|
| 1 | 28 |
| 2 | 39 |
| 9 | 19 |
| 23 | 45 |
| 29 | 33 |

From the above test results, it was confirmed that the compound of the formula (I) has a GK activation action and a blood glucose level lowering action. In addition, the compound can be used as an active ingredient of a pharmaceutical composition for preventing and/or treating diabetes, type 2 diabetes mellitus, obesity, or metabolic syndrome, and the like.

A pharmaceutical composition containing one or more kinds of the compound of the formula (I) or a salt thereof as an active ingredient can be prepared using excipients that are usually used in the art, that is, excipients for pharmaceutical preparation, carriers for pharmaceutical preparation, and the like according to the methods usually used.
Administration can be accomplished either by oral administration via tablets, pills, capsules, granules, powders, solutions, and the like, or parenteral administration, such as injections such as intraarticular, intravenous, and intramuscular injections, suppositories, ophthalmic solutions, eye ointments, transdermal solutions, ointments, transdermal patches, transmucosal solutions, transmucosal patches, inhalers, and the like.

The solid composition for oral administration is used in the form of tablets, powders, granules, or the like. In such a solid composition, one or more active ingredient(s) are mixed with at least one inactive excipient. In a conventional method, the composition may contain inactive additives, such as a lubricant, a disintegrating agent, a stabilizer, or a solubilization assisting agent. If necessary, tablets or pills may be coated with sugar or a film of a gastric-soluble or enteric coating substance.
The liquid composition for oral administration contains pharmaceutically acceptable emulsions, solutions, suspensions, syrups, elixirs, or the like, and also contains generally used inert diluents, for example, purified water or ethanol (EtOH). In addition to the inert diluent, the liquid composition may also contain adjuvants, such as a solubilization assisting agent, a moistening agent, and a suspending agent, sweeteners, flavors, aromatics, or antiseptics.

The injections for parenteral administration include sterile aqueous or non-aqueous solutions, suspensions, or emulsions. The aqueous solvent includes, for example, distilled water for injection or physiological saline. Examples of the non-aqueous solvent include alcohols such as ethanol. Such a composition may further contain a tonicity agent, an antiseptic, a moistening agent, an emulsifying agent, a dispersing agent, a stabilizer, or a solubilization assisting agent. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, poultices, sprays, lotions, eye drops, eye ointments, and the like. The agents contain generally used ointment bases, lotion bases, aqueous or non-aqueous solutions, suspensions, emulsions, and the like.

As the transmucosal agents such as an inhaler, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizer, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a known device or sprayer, such as a measured administration inhalation device, and the like. A dry powder inhaler or the like may be for single or multiple administration use, and a dry powder or a powder-containing capsule may be used. Alternatively, this may be in a form of an appropriate ejection agent such as a pressurized aerosol spray which uses a suitable gas such as chlorofluoroalkane, hydrofluoroalkane, carbon dioxide, and the like.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 separate portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the sexuality, and the like into consideration.

Although varying depending on administration routes, dosage forms, administration sites, or the types of excipients and additives, the pharmaceutical composition of the present invention contains 0.01 to 100% by weight, and in a certain embodiment, 0.01 to 50% by weight of one or more kinds of the compound of the formula (I) or a salt thereof, which is an active ingredient.

The compound of the formula (I) or a salt thereof can be used in combination with various agents for treating or preventing the diseases, in which the compound of the formula (I) or a salt thereof is considered effective, as described above. The combined use may be administered simultaneously or separately and continuously, or at a desired time interval. The preparations to be administered simultaneously may be prepared individually or may be a pharmaceutical composition including various agents for treating or preventing the diseases, in which the compound of the formula (I) or a salt thereof is considered effective, as described above, and the compound of the formula (I) or a salt thereof.

### Examples

Hereinbelow, the preparation methods for the compound of the formula (I) or a salt thereof will be described in more detail with reference to Examples, but the present invention is not limited to the compounds described in the Examples as described below. Furthermore, the production processes for the starting compounds will be described in Preparation Examples. Further, the preparation methods for the compound of the formula (I) are not limited to the preparation methods of the specific Examples as below, and the compound of the formula (I) can be prepared by any combination of the preparation methods or the methods that are apparent to a person skilled in the art.

### Preparation Example 1

To a mixture of 3-hydroxy-5-isopropoxy-N-(1-methyl-1H-pyrazol-3-yl)benzamide (492 mg), potassium carbonate (445 mg), and N-methylpyrrolidone (NMP) (4 mL) was added a solution of 5-chloropyrazine-2-carbonitrile in NMP (3 mL) under ice-cooling, followed by stirring at room temperature for 4 hours and at 50°C for 20 minutes. Diethyl ether (20 mL) and water (20 mL) were added thereto to carry out liquid separation, the organic layer was washed with water (20 mL) and saturated brine (20 mL) in this order, and the aqueous layer was extracted again with diethyl ether (20 mL). The combined organic layer was dried over anhydrous magnesium sulfate and then filtered, and the filtrate was concentrated under reduced pressure. The residue was purified by silica gel column chromatography (n-hexane-ethyl acetate=1:1 to 1:2) to obtain 3-[(5-cyanopyrazin-2-yl)oxy]-5-isopropoxy-N-(1-methyl-1H-pyrazol-3-yl)benzamide (632 mg) as a light yellow oil.

### Preparation Example 2

To a solution of 3-[(5-cyanopyrazin-2-yl)oxy]-5-isopropoxy-N-(1-methyl-1H-pyrazol-3-yl)benzamide (615 mg) in methanol (5 mL) was added a 50% aqueous hydroxylamine solution (200 µL) under ice-cooling, followed by stirring at room temperature for 1 hour. Water (5 mL) was added thereto, and the insoluble material was collected by filtration, washed with methanol, and dried under reduced pressure to obtain 3- {[5-(N'-hydroxycarbamimidoyl)pyrazin-2-yl]oxy}-5-isopropoxy-N-(1-methyl-1H-pyrazol-3-yl)benzamide (618 mg) as a white solid.

### Preparation Example 3

3-{[5-(N'-Hydroxycarbamimidoyl)pyrazin-2-yl]oxy}-5-isopropoxy-N-(1-methyl-1H-pyrazol-3-yl)benzamide (316 mg) was suspended in a mixed solvent of dioxane (2 mL)-chloroform (2 mL), and triethylamine (260 µL) and ethylchloro(oxo)acetate (120 µL) were added thereto in an ice bath, followed by stirring at room temperature for 45 minutes and at 80°C for 4 hours. The reaction mixture was cooled to room temperature, and triethylamine (64 µL) and ethylchloro(oxo)acetate (30 µL) were added thereto, followed by stirring at 80°C for 2 hours and at 100°C for 1 hour. The reaction mixture was cooled to room temperature, and ethyl acetate and water were added thereto to carry out liquid separation. The organic layer was washed with a saturated aqueous sodium hydrogen carbonate solution and saturated brine in this order, dried over anhydrous magnesium sulfate, and then filtered, and the filtrate was concentrated under reduced pressure. The residue was purified by silica gel column chromatography (n-hexane-ethyl acetate=1:1 to 1:2) to obtain ethyl 3-(5-{3-isopropoxy-5-[(1-methyl-1H-pyrazol-3-yl)carbamoyl]phenoxy}pyrazin-2-yl)-1,2,4-oxadiazole-5-carboxylate (123 mg) as a white amorphous substance.

### Preparation Example 4

Methyl 3-(4-cyanophenoxy)benzoate (1.83 g), methanol (20 mL), and THF (20 mL) were mixed, and a 1 M aqueous sodium hydroxide solution (8.70 mL) was added thereto, followed by stirring for 1 hour and leaving to stand overnight. The reaction mixture was concentrated under reduced pressure, and water and 1 M hydrochloric acid was added thereto, followed by extraction with ethyl acetate. The organic layer was washed with water and a saturated aqueous sodium chloride solution, and dried over anhydrous magnesium sulfate, and the solvent was evaporated under reduced pressure to obtain 3-(4-cyanophenoxy)benzoic acid (1.55 g) as a white solid.

### Preparation Example 5

3-(4-Cyanophenoxy)benzoic acid (1.55 g), 1-methyl-1H-pyrazole-3-amine (1.26 g), 1-ethyl-3-(3-dimethylaminopropyl)carbodiimide hydrochloride (EDCI) (1.86 g), 1-hydroxybenzotriazole (HOBt) (1.31 g), and DMF (30 mL) were mixed, followed by stirring at room temperature for 3 hours. To the reaction mixture was added water, followed by extraction with ethyl acetate. The organic layer was washed with water, a saturated aqueous sodium hydrogen carbonate solution, 1 M hydrochloric acid, and a saturated aqueous sodium chloride solution, and dried over anhydrous magnesium sulfate, and the solvent was evaporated under reduced pressure. The obtained residue was purified by silica gel column chromatography to obtain 3-(4-cyanophenoxy)-N-(1-methyl-1H-pyrazol-3-yl)benzamide (1.99 g) as a white amorphous substance.

### Preparation Example 6

To a mixture of 3-(benzyloxy)-5-(methoxycarbonyl)benzoic acid (114 g) and tert-butyl alcohol (700 mL) were added triethylamine (70 mL) and diphenylazide phosphate (100 mL), followed by heating to 105°C and stirring for 2 hours. After leaving to stand for cooling at room temperature, ethyl acetate (700 mL) was added thereto, and the organic layer was washed sequentially with water (700 mL), 1 M hydrochloric acid (700 mL), a saturated aqueous sodium hydrogen carbonate solution (700 mL), and saturated brine (500 mL), dried over anhydrous magnesium sulfate, and then concentrated. To the residue was added hexane/diethyl ether=1/2 (150 mL), followed by stirring for a while, and then the resulting precipitate was collected by filtration and dried to obtain methyl 3-(benzyloxy)-5-[(tert-butoxycarbonyl)amino]benzoate (94.6 g) as a colorless solid.

### Preparation Example 7

To a solution of methyl 3-(benzyloxy)-5-[(tert-butoxycarbonyl)amino]benzoate (50 g) in dimethylformamide (250 mL) was added tert-butoxypotassium (19 g) under ice-cooling, followed by stirring for a while (yellow solution). Then, bromomethylcyclopropane (18 mL) was added thereto at an internal temperature of 15°C or lower, followed by stirring for 20 minutes, and then stirring at room temperature for 3 hours. Water (800 mL) and ethyl acetate (400 mL) were added thereto, and the organic layer was washed with water (400 mL), a saturated aqueous sodium hydrogen carbonate solution (500 mL), 1 M hydrochloric acid (500 mL), and saturated brine (400 mL), and dried over anhydrous magnesium sulfate. Then, the solvent was evaporated under reduced pressure to obtain methyl 3-(benzyloxy)-5-[(tert-butoxycarbonyl)(cyclopropylmethyl)amino]benzoate (57.5 g) as a yellow oily substance.

### Preparation Example 8

To a solution of methyl 3-(benzyloxy)-5-[(tert-butoxycarbonyl)(cyclopropylmethyl)amino]benzoate (1.02 g) in ethanol (15 mL)-THF (15 mL) were added cyclohexene (5.0 mL) and 20% palladium hydroxide-carbon powder (30 wt%, 232 mg), followed by heating to reflux for 1.5 hours. After filtration using Celite, the solvent was evaporated under reduced pressure. The residue was purified by silica gel column chromatography (hexane:ethyl acetate=83:17 to 67:33) to obtain methyl 3-[(tert-butoxycarbonyl)(cyclopropylmethyl)amino]-5-hydroxybenzoate (765 mg) as a colorless liquid.

### Preparation Example 9

A mixture of methyl 3-[(tert-butoxycarbonyl)(cyclopropylmethyl)amino]-5-hydroxybenzoate (1.38 g), potassium carbonate (1.19 g), 4-fluorobenzonitrile (624 mg), and NMP (15 mL) was heated and stirred at 100°C for 2 hours and 30 minutes. The reaction mixture was cooled to room temperature, and diethyl ether (75 mL) and water (75 mL) were added thereto to carry out liquid separation. The organic layer was washed with a 0.2 M aqueous sodium hydroxide solution (75 mL), water (75 mL), and saturated brine (75 mL) in this order, dried over anhydrous magnesium sulfate, and then filtered, and the filtrate was concentrated under reduced pressure to obtain methyl 3-[(tert-butoxycarbonyl)(cyclopropylmethyl)amino]-5-(4-cyanophenoxy)benzoate (1.38 g).

### Preparation Example 10

To a solution of methyl 3-[(tert-butoxycarbonyl)(cyclopropylmethyl)amino]-5-(4-cyanophenoxy)benzoate (50 mg) in dichloromethane (1 mL) was added trifluoroacetic acid (180 µL), followed by stirring at room temperature for 2 hours. A saturated aqueous sodium hydrogen carbonate solution was added thereto until bubbles were not generated, followed by extraction with ethyl acetate (15 mL). The organic layer was washed with a saturated aqueous sodium hydrogen carbonate solution (15 mL) and saturated brine (15 mL) in this order, dried over anhydrous magnesium sulfate, and then filtered, and the filtrate was concentrated under reduced pressure to obtain methyl 3-(4-cyanophenoxy)-5-[(cyclopropylmethyl)amino]benzoate (33 mg).

### Preparation Example 11

To a mixed liquid of methyl 3-(4-cyanophenoxy)-5-[(cyclopropylmethyl)amino]benzoate (980 mg), chloroform (1 mL), and pyridine (1 mL) was added acetic anhydride (1 mL), followed by stirring at room temperature for 17 hours. To the reaction mixture were added diethyl ether (20 mL) and water (20 mL) to carry out liquid separation, and the organic layer was washed with 0.2 M hydrochloric acid (20 mL), water (20 mL) and saturated brine (20 mL) in this order, and dried over anhydrous magnesium sulfate. The filtrate was concentrated under reduced pressure to obtain methyl 3-[acetyl(cyclopropylmethyl)amino]-5-(4-cyanophenoxy)benzoate (1.13 g).

### Preparation Example 12

5-Fluoropyridine-2-carboxylic acid hydrochloride (13.15 g) and dichloromethane (130 mL) were mixed, and oxalyl chloride (9.85 mL) and DMF (0.570 mL) were added thereto, followed by stirring for 1 hour. The reaction mixture was concentrated under reduced pressure, and toluene was added thereto, followed by concentration under reduced pressure. The obtained residue and dichloromethane (260 mL) were mixed, and ethyl (2E)-amino(hydroxyimino)acetate (10.78 g) was added thereto under ice-cooling, followed by stirring for 30 minutes. The reaction mixture was diluted with chloroform, washed with water, a saturated aqueous sodium hydrogen carbonate solution, and a saturated aqueous sodium chloride solution, and dried over anhydrous magnesium sulfate, and the solvent was evaporated under reduced pressure. The obtained residue was washed with ethyl acetate and collected by filtration to obtain a beige solid.
The obtained solid and NMP (130 mL) were mixed, and 4-methylbenzenesulfonic acid hydrate (705 mg) was added thereto, followed by stirring at an oil bath temperature of 130°C for 1 hour. 4-Methylbenzenesulfonic acid hydrate (705 mg) was added thereto, followed by stirring at an oil bath temperature of 130°C for 1 hour. The reaction mixture was cooled to room temperature and diluted with ethyl acetate, washed with water, a 1 M aqueous sodium hydroxide solution, and a saturated aqueous sodium chloride solution, and dried over anhydrous magnesium sulfate, and the solvent was evaporated under reduced pressure. The obtained residue was washed with diethyl ether and collected by filtration to obtain ethyl 5-(5-fluoropyridin-2-yl)-1,2,4-oxadiazole-3-carboxylate (7.95 g) as a white solid.

### Preparation Example 13

3-Hydroxy-5-isopropoxy-N-(1-methyl-1H-pyrazol-3-yl)benzamide (4.00 g), ethyl 5-(5-fluoropyridin-2-yl)-1,2,4-oxadiazole-3-carboxylate (3.45 g), potassium carbonate (4.02 g), and DMF (40 mL) were mixed, followed by stirring at an oil bath temperature of 60°C for 1 hour and stirring at an oil bath temperature of 80°C for 1.5 hours. Ethyl 5-(5-fluoropyridin-2-yl)-1,2,4-oxadiazole-3-carboxylate (345 mg) was added thereto, followed by stirring at an oil bath temperature of 80°C for 1 hour. The reaction mixture was cooled to room temperature and diluted with ethyl acetate, washed with water and a saturated aqueous sodium chloride solution, dried over anhydrous magnesium sulfate, and concentrated under reduced pressure. The obtained residue was purified by silica gel column chromatography (hexane:ethyl acetate=7:3 to 3:7) to obtain ethyl 5-(5-{3-isopropoxy-5-[(1-methyl-1H-pyrazol-3-yl)carbamoyl]phenoxy}pyridin-2-yl)-1,2,4-oxadiazole-3-carboxylate (6.35 g) as a white amorphous substance.

### Preparation Example 14

3-Hydroxy-5-isopropoxy-N-(1-methyl-1H-pyrazol-3-yl)benzamide (2.41 g), ethyl 5-fluoropyridine-2-carboxylate (1.61 g), and potassium carbonate (3.03 g), NMP (50 mL) were mixed, followed by stirring at 110°C for 5 hours. The reaction mixture was cooled to room temperature, and water was added thereto, followed by extraction with ethyl acetate. The organic layer was washed with a saturated aqueous sodium hydrogen carbonate solution and saturated brine, and dried over anhydrous magnesium sulfate, and the solvent was evaporated under reduced pressure to obtain a light yellow oily substance. The obtained light yellow oily substance, methanol (30 mL), and a 5 M aqueous sodium hydroxide solution (3.5 mL) were mixed, followed by stirring at 60°C for 1 hour. 1 M hydrochloric acid was added thereto under ice-cooling to adjust the pH to 4, and water (120 mL) was added thereto. While washing with a solvent (methanol:water=4:1), the precipitated solid was collected by filtration to obtain 5-{3-isopropoxy-5-[(1-methyl-1H-pyrazol-3-yl)carbamoyl]phenoxy}pyridine-2-carboxylic acid (3.24 g) as a white solid.

### Preparation Example 15

5-{3-Isopropoxy-5-[(1-methyl-1H-pyrazol-3-yl)carbamoyl]phenoxy}pyridine-2-carboxylic acid (3.24 g) and DMF (30 mL) were mixed, and EDCI (2.02 g), 1-hydroxy-7-azabenzotriazole (1.43 g), tert-butyl hydrazinecarboxylate (1.51 g), and triethylamine (3.2 mL) were added thereto, followed by stirring at room temperature overnight. To the reaction mixture was added water, followed by extraction with ethyl acetate. The organic layer was washed with a saturated aqueous sodium hydrogen carbonate solution and saturated brine, and dried over anhydrous magnesium sulfate, and the solvent was evaporated under reduced pressure to obtain a light yellow oily substance. To a solution of the obtained light yellow oily substance in dichloromethane (30 mL) was added trifluoroacetic acid (13 mL), followed by stirring at room temperature for 2 hours. The reaction mixture was concentrated under reduced pressure. A saturated aqueous sodium hydrogen carbonate solution was added thereto to adjust the pH to 8, followed by addition of saturated brine and extraction with a solvent (chloroform:isopropyl alcohol=4:1) twice. The organic layer was dried over anhydrous magnesium sulfate and the solvent was evaporated under reduced pressure to obtain 3-{[6-hydrazinocarbonyl)pyridin-3-yl]oxy}-5-isopropoxy-N-(1-methyl-1H-pyrazol-3-yl)benzamide (3.43 g) as a light yellow amorphous substance.

### Preparation Example 16

3- {[6-Hydrazinocarbonyl)pyridin-3-yl]oxy}-5-isopropoxy-N-(1-methyl-1H-pyrazol-3-yl)benzamide (1.0 g) and THF (10 mL) were mixed, triethylamine (0.82 mL) was added thereto, followed by addition of ethyloxalyl chloride (0.33 mL) under ice-cooling and stirring for 1 hour under ice-cooling. To the reaction mixture were added water and saturated brine under ice-cooling, followed by extraction with a solvent (chloroform:isopropyl alcohol=4:1). The organic layer was dried over anhydrous magnesium sulfate and the solvent was evaporated under reduced pressure. The obtained residue was purified by silica gel column chromatography (hexane:ethyl acetate=1:1, chloroform:methanol=1:0 to 10:1) to obtain ethyl {2-[(5-{3-isopropoxy-5-[(1-methyl-1H-pyrazol-3-yl)carbamoyl]phenoxy}pyridin-2-yl)carbonyl]hydrazino}(oxo)acetate (1.16 g) as a light yellow amorphous substance.

### Preparation Example 17

Into a solution of ethyl {2-[(5-{3-isopropoxy-5-[(1-methyl-1H-pyrazol-3-yl)carbamoyl]phenoxy}pyridin-2-yl)carbonyl]hydrazino}(oxo)acetate (1.16 g) in dichloromethane (30 mL) was mixed pyridine (0.74 mL), and anhydrous trifluoromethanesulfonic anhydride (0.75 mL) was added thereto under ice-cooling and stirring for 30 minutes under ice-cooling. To the reaction mixture was added water under ice-cooling, followed by extraction with ethyl acetate. The organic layer was washed with a saturated aqueous ammonium chloride solution, a saturated aqueous sodium hydrogen carbonate solution and saturated brine, and dried over anhydrous magnesium sulfate, and the solvent was evaporated under reduced pressure. The obtained residue was purified by silica gel column chromatography (hexane:ethyl acetate=2:1 to 1:2) to obtain ethyl 5-(5-{3-isopropoxy-5-[(1-methyl-1H-pyrazol-3-yl)carbamoyl]phenoxy}pyridin-2-yl)-1,3,4-oxadiazole-2-carboxylate (572 mg) as a yellowish green amorphous substance.

### Preparation Example 18

To a solution of 5-chloropyrazine-2-carboxylic acid (2 g) in dichloromethane (20 mL) were added DMF (0.05 mL) and oxalyl chloride (1.3 mL), followed by stirring at room temperature for 1 hour. The reaction mixture was concentrated under reduced pressure and azeotroped with toluene. To the residue was added dichloromethane (20 mL), followed by ice-cooling, and triethylamine (4 mL) and ethyl (2E)-amino(hydroxy)iminoacetate (2 g) were added thereto, followed by stirring at the same temperature for 15 minutes. To the reaction mixture was added a saturated aqueous sodium hydrogen carbonate solution, followed by extraction with chloroform. The solid remaining in the aqueous layer was collected by filtration and dried under reduced pressure to obtain ethyl (2E)-amino({[(5-chloropyrazin-2-yl)carbonyl]oxy}imino)acetate (1.4 g) as a colorless solid. Further, the organic layer was washed with a saturated aqueous sodium chloride solution, dried over anhydrous magnesium sulfate, and then concentrated under reduced pressure to obtain ethyl (2E)-amino({[(5-chloropyrazin-2-yl)carbonyl]oxy}imino)acetate (1.9 g) as a yellow solid.

### Preparation Example 19

A mixture of ethyl (2E)-amino({[(5-chloropyrazin-2-yl)carbonyl]oxy}imino)acetate (1.87 g), 4-methylbenzenesulfonic acid (172 mg), and dioxane (100 mL) was stirred at 100°C for 10 hours. To the reaction mixture were added ethyl acetate and water to carry out liquid separation, and the organic layer was washed with a saturated aqueous sodium hydrogen carbonate solution, and saturated brine in this order, dried over anhydrous magnesium sulfate, and then filtered. The filtrate was concentrated under reduced pressure to obtain ethyl 5-(5-chloropyrazin-2-yl)-1,2,4-oxadiazole-3-carboxylate (1.713 g) as a white solid.

### Preparation Example 20

3-(Benzyloxy)-5-hydroxy-N-(1-methyl-1H-pyrazol-3-yl)benzamide (2.52 g), DMF (25 mL), ethyl 1-bromocyclobutane carboxylate (1.52 mL), and potassium carbonate (2.59 g) were mixed, followed by stirring at an oil bath temperature of 80°C for 3 hours. Furthermore, ethyl 1-bromocyclobutane carboxylate (1.52 mL) was added thereto, followed by stirring at an oil bath temperature of 120°C for 8 hours. Further, ethyl 1-bromocyclobutane carboxylate (1.52 mL) and potassium carbonate (2.59 g) were added thereto, followed by stirring at an oil bath temperature of 120°C for 24 hours. The reaction mixture was cooled to room temperature, and water was added thereto, followed by dilution with ethyl acetate. The organic layer was washed with water, 1 M hydrochloric acid, a 1 M aqueous sodium hydroxide solution, and a saturated aqueous sodium chloride solution, and dried over anhydrous magnesium sulfate, and the solvent was evaporated under reduced pressure. The obtained residue was purified by silica gel column chromatography (hexane:ethyl acetate=7:3 to 2:8) to obtain a yellow amorphous substance.
The obtained amorphous substance and ethanol (50 mL) were mixed, and a 1 M aqueous sodium hydroxide solution (16 mL) was added thereto, followed by stirring at room temperature for 2 hours. Further, a 1 M aqueous sodium hydroxide solution (8 mL) was added thereto, followed by stirring for 2 hours. The reaction mixture was concentrated under reduced pressure, and 1 M hydrochloric acid was added thereto, followed by extraction with ethyl acetate. The organic layer was washed with water and a saturated aqueous sodium chloride solution, and dried over anhydrous magnesium sulfate, and the solvent was evaporated under reduced pressure. The obtained solid was washed with diisopropyl ether and collected by filtration to obtain 1-{3-(benzyloxy)-5-[(1-methyl-1H-pyrazol-3-yl)carbamoyl]phenoxy}cyclobutane carboxylic acid (2.00 g) as a yellowish white solid.

### Preparation Example 21

1-{3-(Benzyloxy)-5-[(1-methyl-1H-pyrazol-3-yl)carbamoyl]phenoxy}cyclobutane carboxylic acid (2.00 g) and THF (40 mL) were mixed, and CDI (924 mg) was added thereto, followed by stirring at an oil bath temperature of 50°C for 1 hour. The reaction mixture was ice-cooled, and water (4.2 mL) and sodium borohydride (539 mg) were added thereto, followed by stirring for 30 minutes. To the reaction mixture was added water, followed by extraction with ethyl acetate. The organic layer was washed with a saturated aqueous sodium chloride solution and dried over anhydrous magnesium sulfate, and the solvent was evaporated under reduced pressure. The obtained residue was purified by silica gel column chromatography (hexane:ethyl acetate=7:3 to 2:8) to obtain 3-(benzyloxy)-5- {[1-(hydroxymethyl)cyclobutyl]oxy}-N-(1-methyl-1H-pyrazol-3-yl)benzamide (1.58 g) as a white amorphous substance.

### Preparation Example 22

3-(Benzyloxy)-5-{[1-(hydroxymethyl)cyclobutyl]oxy}-N-(1-methyl-1H-pyrazol-3-yl)benzamide (1.57 g), DMF (20 mL), 1H-imidazole (603 mg), and tert-butyl (chloro)dimethylsilane (1.28 g) were mixed, followed by stirring at room temperature for 1 hour. The reaction mixture was diluted with ethyl acetate, washed with water, a saturated aqueous ammonium chloride solution, a saturated aqueous sodium hydrogen carbonate solution, and a saturated aqueous sodium chloride solution, and dried over anhydrous magnesium sulfate, and the solvent was evaporated under reduced pressure. The obtained residue was purified by silica gel column chromatography (hexane:ethyl acetate=1:0 to 7:3) to obtain 3-(benzyloxy)-5-{[1-({[tert-butyl (dimethyl)silyl]oxy}methyl)cyclobutyl]oxy}-N-(1-methyl-1H-pyrazol-3 -yl)benzamide (1.81 g) as a colorless oily substance.

### Preparation Example 23

3-(Benzyloxy)-5-{[1-({[tert-butyl (dimethyl)silyl]oxy}methyl)cyclobutyl]oxy}-N-(1-methyl-1H-pyrazol-3-yl)benzamide (1.81 g), 10% palladium/carbon (360 mg), and ethyl acetate (25 m) were mixed, followed by stirring overnight under a hydrogen atmosphere of 0.4 MPa. After filtration using Celite, the filtrate was concentrated under reduced pressure. The obtained solid was washed with hexane and collected by filtration to obtain 3-{[1-({[tert-butyl (dimethyl)silyl]oxy}methyl)cyclobutyl]oxy}-5-hydroxy-N-(1-methyl-1H-pyrazol-3-yl)benzamide (1.29 g) as a white solid.

### Preparation Example 24

Ethyl 5-[5-(3-{[1-({[tert-butyl (dimethyl)silyl]oxy}methyl)cyclobutyl]oxy}-5-[(1-methyl-1H-pyrazol-3-yl)carbamoyl]phenoxy)pyrazin-2-yl]-1,2,4-oxadiazole-3-carboxylate (250 mg), and THF (5 mL) were mixed, and a 2 M methylamine/THF solution (0.580 mL) was added thereto under ice-cooling, followed by stirring for 30 minutes, warming to room temperature, and stirring for 15 minutes. Further, a 2 M methylamine/THF solution (0.580 mL) was added thereto, followed by stirring for 15 minutes. Further, a 2 M methylamine/THF solution (0.580 mL) was added thereto, followed by stirring for 15 minutes. Further, a 2 M methylamine/THF solution (0.580 mL) was added thereto, followed by stirring for 15 minutes. The reaction mixture was diluted with ethyl acetate, washed with water and a saturated aqueous sodium chloride solution, and dried over anhydrous magnesium sulfate, and the solvent was evaporated under reduced pressure. The obtained residue was purified by silica gel column chromatography (hexane:ethyl acetate=2:1 to 0:1) to obtain 5-[5-(3-{[1-({[tert-butyl (dimethyl)silyl]oxy} methyl)cyclobutyl]oxy} -5-[(1-methyl-1H-pyrazol-3-yl)carbamoyl]phenoxy)pyrazin-2-yl]-N-methyl-1,2,4-oxadiazole-3-carboxamide (162 mg) as a white amorphous substance.

### Preparation Example 25

Ethyl 5-(3-{[1-(hydroxymethyl)cyclobutyl]oxy}-5-[(1-methyl-1H-pyrazol-3-yl)carbamoyl]phenoxy)pyridine-2-carboxylate (415 mg), ethanol (10 mL), and a 1 M aqueous sodium hydroxide solution (2.7 mL) were added, followed by stirring at room temperature for 3 hours. To the reaction mixture, 1 M hydrochloric acid (2.7 mL) and water were added, followed by extraction with a mixed solution of chloroform/isopropanol (4:1). The organic layer was washed with a saturated aqueous sodium chloride solution and dried over anhydrous magnesium sulfate, and the solvent was evaporated under reduced pressure. The obtained solid was washed with ethyl acetate and collected by filtration to obtain 5-(3-{[1-(hydroxymethyl)cyclobutyl]oxy}-5-[(1-methyl-1H-pyrazol-3-yl)carbamoyl]phenoxy)pyridine-2-carboxylic acid (267 mg) as a white solid.

### Preparation Example 26

tert-Butyl 5- {3-isopropoxy-5-[(1-methyl-1H-pyrazol-3-yl)carbamoyl]phenoxy}pyrazine-2-carboxylate (1.29 g) and dichloromethane (30 mL) were mixed, and trifluoroacetic acid (4.35 mL) was added thereto, followed by stirring at room temperature for 3 hours. The reaction mixture was concentrated under reduced pressure, and toluene was added thereto, followed by concentration under reduced pressure. To the obtained residue was added ethyl acetate, followed by washing with a saturated aqueous sodium hydrogen carbonate solution. The aqueous layer was neutralized by the addition of 1 M hydrochloric acid and extracted with a mixed solution of chloroform/isopropanol (4:1). The organic layer was washed with water and the solvent was evaporated under reduced pressure. The obtained residue was washed with diisopropyl ether and collected by filtration to obtain 5-{3-isopropoxy-5-[(1-methyl-1H-pyrazol-3-yl)carbamoyl]phenoxy}pyrazine-2-carboxylic acid (797 mg) as a white solid.

### Preparation Example 27

5-{3-Isopropoxy-5-[(1-methyl-1H-pyrazol-3-yl)carbamoyl]phenoxy}pyrazine-2-carboxylic acid (1.195 g) and DMF (24 mL) were mixed, and EDCI (750 mg), 1-hydroxy-7-azabenzotriazole (532 mg), triethylamine (1.28 mL), and tert-butylhydrazinecarboxylate (597 mg) were added thereto, followed by stirring at room temperature for 1 hour.
Furthermore, EDCI (750 mg), 1-hydroxy-7-azabenzotriazole (532 mg), triethylamine (1.28 mL), and tert-butylhydrazinecarboxylate (597 mg) were added thereto, followed by stirring at an oil bath temperature of 60°C for 1 hour. The reaction mixture was cooled to room temperature, diluted with ethyl acetate, washed with water, 1 M hydrochloric acid, and a saturated aqueous sodium chloride solution, and dried over anhydrous magnesium sulfate, and the solvent was evaporated under reduced pressure. The obtained residue was purified by silica gel column chromatography (hexane:ethyl acetate=7:3 to 3:7) to obtain tert-butyl 2-[(5-{3-isopropoxy-5-[(1-methyl-1H-pyrazol-3-yl)carbamoyl]phenoxy} pyrazin-2-yl)carbonyl]hydrazinecarboxylate (710 mg) as a white amorphous substance.

### Preparation Example 28

tert-Butyl 2-[(5-{3-isopropoxy-5-[(1-methyl-1H-pyrazol-3-yl)carbamoyl]phenoxy}pyrazin-2-yl)carbonyl]hydrazinecarboxylate (705 mg) and ethyl acetate (15 mL) were mixed, and a 4 M hydrogen chloride/ethyl acetate solution (7 mL) was added thereto, followed by stirring for 30 minutes. The reaction mixture was concentrated under reduced pressure, and dichloromethane (15 mL) and trifluoroacetic acid (3.17 mL) were added thereto, followed by stirring for 1 hour. Further, trifluoroacetic acid (2 mL) was added thereto, followed by stirring for 2 hours. To the reaction mixture was added toluene, followed by concentration under reduced pressure. The residue was diluted with ethyl acetate, and water and a saturated aqueous sodium hydrogen carbonate solution were added thereto to carry out liquid separation. The organic layer was dried over anhydrous magnesium sulfate and concentrated under reduced pressure. The obtained residue was purified by silica gel column chromatography (ethyl acetate:methanol=1:0 to 19:1) to obtain 3-{[5-(hydrazinocarbonyl)pyrazin-2-yl]oxy}-5-isopropoxy-N-(1-methyl-1H-pyrazol-3-yl)benzamide (397 mg) as a white solid.

### Preparation Example 29

Ethyl 5-[3-isopropoxy-5-(1H-pyrazol-3-ylcarbamoyl)phenoxy]pyridine-2-carboxylate (941 mg), methanol (20 mL), and a 5 M aqueous sodium hydroxide solution (1.4 mL) were mixed, followed by stirring at 60°C for 2 hours and adjusting to pH 4 by the addition of 1 M hydrochloric acid under ice-cooling. Water (80 mL) was added thereto, and the precipitated solid was collected by filtration while washing with a solvent (methanol:water=5:1) to obtain 5-[3-isopropoxy-5-(1H-pyrazol-3-ylcarbamoyl)phenoxy]pyridine-2-carboxylic acid (763 mg) as a white solid.

### Preparation Example 30

5-[3-Isopropoxy-5-(1H-pyrazol-3-ylcarbamoyl)phenoxy]pyridine-2-carboxylic acid (763 mg) and DMF (20 mL) were mixed, and EDCI (580 mg), 1-hydroxy-7-azabenzotriazole (413 mg), ethyl (2E)-amino(hydroxyimino)acetate (352 mg), and triethylamine (0.84 mL) were added thereto, followed by stirring at room temperature for 3 days. To the reaction mixture was added water, followed by extraction with ethyl acetate. The organic layer was washed with water and saturated brine, and dried over anhydrous magnesium sulfate, and the solvent was evaporated under reduced pressure. To the obtained residue were added dioxane (50 mL) and pyridinium p-toluenesulfonate (50 mg), followed by heating to reflux for 24 hours. The reaction mixture was cooled to room temperature, and water was added thereto, followed by extraction with ethyl acetate. The organic layer was washed with water, a saturated aqueous sodium hydrogen carbonate solution, and saturated brine, and dried over anhydrous magnesium sulfate, and the solvent was evaporated under reduced pressure. The obtained residue was purified by silica gel column chromatography (hexane:ethyl acetate=1:1 to 1:3) to obtain ethyl 5-{5-[3-isopropoxy-5-(1H-pyrazol-3-ylcarbamoyl)phenoxy]pyridin-2-yl}-1,2,4-oxadiazole-3-carboxylate (670 mg) as a white amorphous substance.

### Preparation Example 31

5-Fluoropyridine-2-carboxylic acid (2.846 g) and DMF (30 mL) were mixed, and EDCI (4.98 g), 1-hydroxy-7-azabenzotriazole (3.53 g), tert-butyl hydrazinecarboxylate (3.73 g), and triethylamine (7.9 mL) were added thereto, followed by stirring at room temperature overnight. To the reaction mixture was added water, followed by extraction with ethyl acetate. The organic layer was washed with a saturated aqueous sodium hydrogen carbonate solution and saturated brine, and dried over anhydrous magnesium sulfate. The solvent was evaporated under reduced pressure and the obtained crude product was purified by silica gel column chromatography (hexane:ethyl acetate=2:1 to 1:1) to obtain tert-butyl 2-[(5-fluoropyridin-2-yl)carbonyl]hydrazinecarboxylate (3.372 g) as a white solid.

### Preparation Example 32

To a solution of tert-butyl 2-[(5-fluoropyridin-2-yl)carbonyl]hydrazinecarboxylate (3.37 g) in dichloromethane (40 mL) was added trifluoroacetic acid (20 mL), followed by stirring at room temperature for 2 hours. The reaction mixture was concentrated under reduced pressure and adjusted to pH=8 by the addition of a saturated aqueous sodium hydrogen carbonate solution, and then saturated brine was added thereto, followed by extraction with solvent (chloroform:isopropyl alcohol=4:1) four times. The organic layer was dried over anhydrous magnesium sulfate and the solvent was evaporated under reduced pressure to obtain 5-fluoropyridine-2-carbohydrazide (1.88 g) as a light yellow solid.

### Preparation Example 33

Into a solution of 5-fluoropyridine-2-carbohydrazide (1 g) in dichloromethane (30 mL) was mixed triethylamine (2.7 mL), and methyl chloroglyoxylate (0.59 mL) was added thereto under ice-cooling, followed by stirring at room temperature for 5 hours. p-Toluenesulfonyl chloride (1.23 g) was added thereto under ice-cooling, followed by stirring at room temperature overnight. To the reaction mixture was added water at room temperature to carry out liquid separation, and the organic layer was concentrated. After concentration, the obtained oily substance was dissolved in ethyl acetate. Together with the aqueous layer, liquid separation was carried out. The organic layer was washed with a saturated aqueous sodium hydrogen carbonate solution and saturated brine. The organic layer was dried over anhydrous magnesium sulfate, filtered, and concentrated. The obtained solid was collected by filtration while washing with a solvent (hexane:ethyl acetate=4:1) to obtain methyl 5-(5-fluoropyridin-2-yl)-1,3,4-oxadiazole-2-carboxylate (1.073 g) as a gray solid.

### Preparation Example 34

To a suspension of methyl 5-(5-fluoropyridin-2-yl)-1,3,4-oxadiazole-2-carboxylate (1.07 g) in THF (5 mL) was added a 2 M methylamine/THF solution (7.2 mL) at room temperature, followed by stirring at room temperature for 2 hours. After concentration, the obtained solid was collected by filtration while washing with a solvent (hexane:ethyl acetate=1:1) to obtain 5-(5-fluoropyridin-2-yl)-N-methyl-1,3,4-oxadiazole-2-carboxamide (968 mg) as an orange solid.

### Preparation Example 35

To a solution of methyl 3-(4-aminophenoxy)-5-isopropoxybenzoate (1.3 g) in dichloroethane (25 mL) were sequentially added cyclopropane carboxaldehyde (302 mg) and sodium triacetoxyborohydride (1.0 g), followed by stirring at room temperature for 2 hours. Then, to the reaction mixture was added an aqueous sodium hydrogen carbonate solution, followed by alkalifying by the addition of sodium hydrogen carbonate. The organic layer was washed with saturated brine and then dried over anhydrous magnesium sulfate. After concentration, the obtained residue was purified by silica gel column chromatography (0 to 10% ethyl acetate/hexane) to obtain methyl 3-{4-[(cyclopropylmethyl)amino]phenoxy}-5-isopropoxybenzoate (1.53 g) as a light brown oil.

### Preparation Example 36

To a solution of methyl 3-{4-[(cyclopropylmethyl)amino]phenoxy}-5-isopropoxybenzoate (1.3 g) in dichloromethane (10 mL) were added pyridine (0.5 mL) and acetic anhydride (0.6 mL), followed by stirring at room temperature for 1 hour. After concentration, the residue was dissolved in ethyl acetate, and the organic layer was washed with 1 M hydrochloric acid, a saturated aqueous sodium hydrogen carbonate solution, and saturated brine. The organic layer was dried over anhydrous magnesium sulfate and then concentrated, and the residue was purified by silica gel column chromatography (0 to 50% ethyl acetate/hexane) to obtain methyl 3-{4-[acetyl(cyclopropylmethyl)amino]phenoxy}-5-isopropoxybenzoate (1.15 g) as a colorless oil.

### Preparation Example 37

To a solution of methyl 3-{4-[acetyl(cyclopropylmethyl)amino]phenoxy}-5-isopropoxybenzoate (212 mg) in methanol (5 mL) was added a 1 M aqueous sodium hydroxide solution (0.8 mL), followed by stirring at room temperature overnight. After concentration under reduced pressure, water (10 mL), 1 M hydrochloric acid (1.9 mL), and ethyl acetate (20 mL) were added thereto, and the organic layer was washed with saturated brine (20 mL) and dried over anhydrous magnesium sulfate. The solvent was evaporated under reduced pressure to obtain 3-{4-[acetyl(cyclopropylmethyl)amino]phenoxy}-5-isopropoxybenzoic acid (170 mg) as a colorless amorphous substance.

### Preparation Example 38

To a solution of methyl 3,5-dihydroxybenzoate (50 g) in DMF (1 L) was added potassium tert-butoxide (70 g), followed by stirring at room temperature for 30 minutes. A solution of 1-fluoro-4-nitrobenzene (30 mL) in DMF (30 mL) was added dropwise thereto under ice-cooling. After leaving to stand at room temperature overnight, 1 M hydrochloric acid (500 mL) was added thereto under ice-cooling. Water (3 L) was added thereto, followed by extraction with ethyl acetate (500 mL, twice). The organic layer was washed with a saturated aqueous sodium hydrogen carbonate solution (1 L) and saturated brine (500 mL), and dried over anhydrous magnesium sulfate, and the solvent was then evaporated under reduced pressure. To the residue was added a solvent (500 mL, hexane:ethyl acetate=2:1), and the precipitate was collected by filtration and dried to obtain methyl 3-hydroxy-5-(4-nitrophenoxy)benzoate (57.87 g) as a light yellow solid.

### Preparation Example 39

To a solution of {1-[(benzyloxy)methyl]cyclobutyl}methanol (3 g), methyl 3-hydroxy-5-(4-nitrophenoxy)benzoate (5.05 g), and tributylphosphine (4.3 mL) in THF (60 mL) was added 1,1'-(azodicarbonyl)dipiperidine (4.40 g) under ice-cooling, followed by stirring at room temperature for 5 hours and at 60°C overnight. After leaving to stand for cooling at room temperature, the precipitated solid was collected by filtration while washing with a solvent (hexane:ethyl acetate=1:1). The filtrate was concentrated and the obtained oily substance was purified by silica gel column chromatography (hexane:ethyl acetate=1:0 to 5:1) to obtain methyl 3-({1-[(benzyloxy)methyl]cyclobutyl}methoxy)-5-(4-nitrophenoxy)benzoate (4.07 g) as a light yellow oily substance.

### Preparation Example 40

To a mixed solution of methyl 3-({1-[(benzyloxy)methyl]cyclobutyl}methoxy)-5-(4-nitrophenoxy)benzoate (4.07 g) in methanol (120 mL) and water (40 mL) were added reduced iron (4.77 g) and ammonium chloride (913 mg) at room temperature, followed by stirring at 80°C for 4 hours. After leaving to stand for cooling at room temperature and filtering using Celite, the filtrate was extracted with ethyl acetate. The organic layer was washed with a saturated aqueous sodium hydrogen carbonate solution and saturated brine, dried over anhydrous magnesium sulfate, and concentrated to obtain methyl 3-(4-aminophenoxy)-5-({l-[(benzyloxy)methyl]cyclobutyl}methoxy)benzoate (3.891 g) as a light yellow oily substance.

### Preparation Example 41

A mixture of methyl 3-(4-acetamidephenoxy)-5-isopropoxybenzoate (150 mg), 1-fluoro-2-iodoethane (152 mg), potassium carbonate (121 mg), and DMF (2 mL) was stirred at 60°C for 1 hour. 60% sodium hydride (17 mg) was added thereto, followed by stirring at 80°C for 4 days. To the reaction mixture was added water, followed by extraction with ethyl acetate. The organic layer was washed with saturated brine and dried over anhydrous sodium sulfate. The solvent was evaporated and the obtained residue was purified by silica gel column chromatography (ethyl acetate:hexane=50:50) to obtain methyl 3-{4-[acetyl(2-fluoroethyl)amino]phenoxy}-5-isopropoxybenzoate (100 mg) as a light yellow amorphous substance.

### Preparation Example 42

A mixture of methyl 3-(4-aminophenoxy)-5-isopropoxybenzoate (2 g), di-tert-butyldicarbonate (1.59 g), triethylamine (1.02 mL), and DMF (20 mL) was stirred at room temperature for 3 hours. To the reaction mixture was added water, followed by extraction with ethyl acetate. The organic layer was washed with saturated brine and dried over anhydrous sodium sulfate, and the solvent was evaporated. To the obtained residue were added ethyl acetate/hexane, and the precipitated solid was collected by filtration to obtain methyl 3-{4-[(tert-butoxycarbonyl)amino]phenoxy}-5-isopropoxybenzoate (2.3 g) as a white solid.

### Preparation Example 43

A mixture of methyl 3-{4-[(tert-butoxycarbonyl)amino]phenoxy}-5-isopropoxybenzoate (1.2 g), (bromomethyl)cyclopropane (484 mg), 60% sodium hydride (132 mg), and DMF (10 mL) was stirred at room temperature for 5 hours. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=20:80 to 50:50) to obtain methyl 3-{4-[(test-butoxycarbonyl)(cyclopropylmethyl)amino]phenoxy}-5-isopropoxybenzoate (1.3 g) as a white solid.

### Preparation Example 44

To a solution of methyl 3-hydroxy-5-(4-nitrophenoxy)benzoate (5.4 g) in DMF (60 mL) were added potassium carbonate (7.2 g) and benzyl 1-bromocyclobutane carboxylate (10 g), followed by stirring at an internal temperature of 70°C for 3 hours. Then, benzyl 1-bromocyclobutane carboxylate (5 g) was added thereto, followed by stirring for 2 hours. Benzyl 1-bromocyclobutane carboxylate (2.3 g) was added thereto again, followed by stirring overnight. After leaving to stand for cooling at room temperature, water (200 mL) and ethyl acetate (200 mL) were added thereto, and the organic layer was washed with water (200 mL), a saturated aqueous sodium hydrogen carbonate solution (200 mL), 1 M hydrochloric acid (200 mL), and saturated brine (200 mL), and dried over anhydrous magnesium sulfate. Then, the solvent was evaporated under reduced pressure. The residue was purified by silica gel column chromatography (hexane/ethyl acetate=95/5→80/20) to obtain methyl 3-({1-[(benzyloxy)carbonyl]cyclobutyl}oxy)-5-(4-nitrophenoxy)benzoate (8.38 g) as a yellow oily substance.

### Preparation Example 45

Methyl 3-(4-aminophenoxy)-5-(benzyloxy)benzoate (6.87 g) and dichloroethane (70 mL) were mixed, and cyclopropane carboxaldehyde (2.07 mL) and acetic acid (7.0 mL) were added thereto, followed by stirring at room temperature for 30 minutes. Sodium triacetoxyborohydride (6.18 g) was added thereto under ice-cooling, followed by stirring for 30 minutes. To the reaction mixture was added water, followed by extraction with chloroform. The organic layer was washed with water, a saturated aqueous sodium hydrogen carbonate solution, and a saturated aqueous sodium chloride solution, and dried over anhydrous magnesium sulfate, and the solvent was evaporated under reduced pressure.
The obtained oily substance and dichloromethane (70 mL) were mixed, and pyridine (4.77 mL) and acetic anhydride (5.58 mL) were added thereto, followed by stirring for 1 hour. The reaction mixture was concentrated under reduced pressure, diluted with ethyl acetate, washed with water and a saturated aqueous sodium chloride solution, dried over anhydrous magnesium sulfate, filtered, and concentrated under reduced pressure. The obtained residue was purified by silica gel column chromatography (hexane:ethyl acetate=4:1 to 2:1) to obtain methyl 3-{4-[acetyl(cyclopropylmethyl)amino]phenoxy}-5-(benzyloxy)benzoate (5.31 g) as a brown oily substance.

### Preparation Example 46

To a solution of methyl 3-{4-[acetyl(cyclopropylmethyl)amino]phenoxy}-5-({1-[(benzyloxy)carbonyl]cyclobutyl}oxy)benzoate (2.75 g) in ethyl acetate (5 mL) and methanol (15 mL) was added 10% palladium/carbon (200 mg), followed by stirring at room temperature for 2 hours under a hydrogen atmosphere of 0.3 MPa. The precipitate was removed using Celite and the filtrate was concentrated under reduced pressure to obtain 1-[3-{4-[acetyl(cyclopropylmethyl)amino]phenoxy}-5-(methoxycarbonyl)phenoxy]cyclobutane carboxylic acid (2.09 g) as a colorless amorphous substance.

### Preparation Example 47

To a solution of 1-[3-{4-[acetyl(cyclopropylmethyl)amino]phenoxy}-5-(methoxycarbonyl)phenoxy]cyclobutane carboxylic acid (2.09 g) in THF (30 mL) were added triethylamine (0.77 mL) and isobutylchlorocarbonate (0.71 mL) under ice-cooling, followed by stirring for 1 hour under ice-cooling, and then the precipitate was separated by filtration. To the filtrate were added sodium borohydride (470 mg) and methanol (3 mL), followed by stirring at room temperature for 1 hour, and then ice and 1 M hydrochloric acid (30 mL) were added thereto. After concentration under reduced pressure, ethyl acetate (200 mL) and water (100 mL) were added thereto, and the organic layer was washed sequentially with a saturated aqueous sodium hydrogen carbonate solution (100 mL) and saturated brine (100 mL), and dried over anhydrous magnesium sulfate. Then, the solvent was evaporated under reduced pressure and the residue was purified by silica gel column chromatography (hexane/ethyl acetate=30/70→10/90) to obtain methyl 3-{4-[acetyl(cyclopropylmethyl)amino]phenoxy}-5-{[1-(hydroxymethyl)cyclobutyl]oxy}benzoate (1.02 g) as a colorless oil.

### Preparation Example 48

To a solution of methyl 3-{4-[acetyl(cyclopropylmethyl)amino]phenoxy}-5-{[1-(hydroxymethyl)cyclobutyl]oxy}benzoate (1.02 g) in DMF (10 mL) were added imidazole (365 mg) and tert-butyl (chloro)dimethylsilane (770 mg), followed by stirring at room temperature overnight. Water (40 mL) and ethyl acetate (40 mL) were added thereto, and the organic layer was washed sequentially with a saturated aqueous sodium hydrogen carbonate solution (30 mL), 1 M hydrochloric acid (30 mL), and saturated brine (30 mL), and dried over anhydrous magnesium sulfate. Then, the solvent was evaporated under reduced pressure. The residue was purified by silica gel column chromatography (hexane/ethyl acetate=80/20-50/50) to obtain methyl 3-{4-[acetyl(cyclopropylmethyl)amino]phenoxy} -5- {[1-({[tert-butyl (dimethyl)silyl]oxy}methyl)cyclobutyl]oxy}benzoate (1.28 g) as a colorless oil.

### Preparation Example 49

A mixture of tert-butyl (cyclopropylmethyl){4-[3-isopropoxy-5-(1,3-thiazol-2-ylcarbamoyl)phenoxy]phenyl}carbamate (470 mg), trifluoroacetic acid (5 mL), and chloroform (5 mL) was stirred at room temperature for 3 hours. After evaporation of the solvent, a saturated aqueous sodium hydrogen carbonate solution was added thereto, followed by extraction with ethyl acetate. The organic layer was washed with saturated brine and dried over anhydrous sodium sulfate. The solvent was evaporated to obtain 3-{4-[(cyclopropylmethyl)amino]phenoxy}-5-isopropoxy-N-1,3-thiazol-2-ylbenzamide (380 mg) as a white amorphous substance.

### Preparation Example 50

To a solution of 3-{4-[acetyl(cyclopropylmethyl)amino]phenoxy}-5-[(1S)-2-methoxy-1-methylethoxy]benzoic acid (202 mg) in dichloromethane (5 mL) were added a 1 M oxalyl chloride/dichloromethane solution (0.54 mL) and DMF (3 droplets) under ice-cooling, followed by stirring at room temperature for 60 minutes. tert-Butyl 3-amino-1H-pyrazole-1-carboxylate (107 mg) and pyridine (0.08 mL) were added thereto under ice-cooling, followed by stirring at room temperature overnight. Water was added thereto, followed by extraction with ethyl acetate, and the organic layer was washed with a saturated aqueous ammonium chloride solution, a saturated aqueous sodium hydrogen carbonate solution, and saturated brine, and dried over anhydrous magnesium sulfate. Then, the solvent was evaporated under reduced pressure and the obtained crude product was purified by silica gel column chromatography (hexane:ethyl acetate=2:1 to 1:2) to obtain tert-butyl 3-[(3-{4-[acetyl(cyclopropylmethyl)amino]phenoxy}-5-[(1S)-2-methoxy-1-methylethoxy]benzoyl)amino]-1H-pyrazole-1-carboxylate (181 mg) as a white amorphous substance.

### Preparation Example 51

To a solution of methyl 3-{4-[(cyclopropylmethyl)amino]phenoxy}-5-[(1S)-2-methoxy-1-methylethoxy]benzoate (1.263 g) in dichloromethane (20 mL) were added triethylamine (1.4 mL) and methanesulfonyl chloride (0.28 mL) under ice-cooling, followed by stirring for 2 hours under ice-cooling. Water was added thereto, followed by extraction with ethyl acetate. The organic layer was washed with 1 M hydrochloric acid, a saturated aqueous sodium hydrogen carbonate solution, and saturated brine, dried over anhydrous magnesium sulfate, and concentrated to obtain methyl 3-{4-[(cyclopropylmethyl)(methylsulfonyl)amino]phenoxy}-5-[(1S)-2-methoxy-1-methylethoxy]benzoate (1.795 g) as a yellow oily substance.

### Preparation Example 52

To a solution of methyl 3-(2-chloro-4-nitrophenoxy)-5-hydroxybenzoate (2.5 g) in DMF (25 mL) were added potassium carbonate (2.51 g) and 2-iodopropane (1.0 mL) at room temperature, followed by stirring 50°C for 5 hours. 2-Iodopropane (1.0 mL) was added thereto again, followed by stirring at 50°C for 4 hours. After leaving to stand for cooling to room temperature, water was added thereto, followed by extraction with ethyl acetate. The organic layer was washed with a saturated aqueous sodium hydrogen carbonate solution and saturated brine, dried over anhydrous magnesium sulfate, and concentrated to obtain methyl 3-(2-chloro-4-nitrophenoxy)-5-isopropoxybenzoate (3.135 g) as a yellow oily substance.

### Preparation Example 53

A mixture of tert-butyl 5-[3-isopropoxy-5-(methoxycarbonyl)phenoxy]pyrazine-2-carboxylate (0.7 g), trifluoroacetic acid (3 mL), and chloroform (3 mL) was stirred at room temperature for 2 hours. The solvent was evaporated and water was added thereto, followed by extraction with ethyl acetate. The organic layer was washed with saturated brine and dried over anhydrous sodium sulfate, and the solvent was evaporated. The residue was solidified with dichloromethane/diethyl ether/hexane to obtain 5-[3-isopropoxy-5-(methoxycarbonyl)phenoxy]pyrazine-2-carboxylic acid (0.4 g) as a white solid.

### Preparation Example 54

A mixture of 5-[3-isopropoxy-5-(methoxycarbonyl)phenoxy]pyrazine-2-carboxylic acid (3.6 g), diphenyl phosphoryl azide (3.49 mL), triethylamine (3.02 mL), and tert-butanol (36 mL) was heated to reflux for 1 hour. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=10:90) to obtain methyl 3-({5-[(tert-butoxycarbonyl)amino]pyrazin-2-yl}oxy)-5-isopropoxybenzoate (2.5 g) as a light yellow oily substance.

### Preparation Example 55

A mixture of methyl 3-({5-[(cyclopropylmethyl)amino]pyrazin-2-yl}oxy)-5-isopropoxybenzoate (0.5 g), acetic anhydride (5 mL), and 4-dimethylaminopyridine (1.03 g) was stirred at 80°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=20:80 to 50:50) to obtain methyl 3-({5-[acetyl(cyclopropylmethyl)amino]pyrazin-2-yl}oxy)-5-isopropoxybenzoate (347 mg) as a light yellow oily substance.

### Preparation Example 56

A mixture of methyl 3-({5-[(cyclopropylmethyl)amino]pyrazin-2-yl}oxy)-5-isopropoxybenzoate (0.2 g), 2-methylpropanoyl chloride (0.45 mL), and 4-dimethylaminopyridine (1.51 g) was stirred at room temperature for 3 hours. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=20:80) to obtain methyl 3-({5-[(cyclopropylmethyl)(isobutyryl)amino]pyrazin-2-yl}oxy)-5-isopropoxybenzoate (0.2 g) as a light yellow oil.

### Preparation Example 57

A mixture of 3-({5-[(cyclopropylmethyl)(isobutyryl)amino]pyrazin-2-yl}oxy)-5-isopropoxybenzoic acid (120 mg), tert-butyl 3-amino-1H-pyrazole-1-carboxylate (160 mg), N-[(dimethylamino)(3H-[1,2,3]triazolo[4,5-b]pyridin-3-yloxy)methylene]-N-methylmethaneaminium hexafluorophosphate (221 mg), triethylamine (0.11 mL), DMF (0.5 mL), and 4-dimethylaminopyridine (35 mg) was stirred at 50°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=20:80) to obtain tert-butyl 3-{[3-({5-[(cyclopropylmethyl)(isobutyryl)amino]pyrazin-2-yl}oxy)-5-isopropoxybenzoyl]amino}-1H-pyrazole-1-carboxylate (80 mg) as a white solid.

### Preparation Example 58

Methyl 3-hydroxy-5-(4-nitrophenoxy)benzoate (20.0 g), potassium carbonate (10.5 g), and DMF (200 mL) were mixed, and (bromomethyl)benzene (9.10 mL) was added thereto, followed by stirring at room temperature for 8 hours. The solid was removed by filtration and water was added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (hexane:ethyl acetate=20:1 to 4:1) to obtain methyl 3-(benzyloxy)-5-(4-nitrophenoxy)benzoate (26.0 g) as a yellow solid.

### Preparation Example 59

Methyl 3-{4-[acetyl(cyclopropylmethyl)amino]phenoxy}-5-(benzyloxy)benzoate (5.31 g) and trifluoroacetic acid (60 mL) were mixed, and 1,2,3,4,5-pentamethylbenzene (8.84 g) was added thereto under ice-cooling, followed by warming to room temperature and stirring for 5 hours. To the reaction mixture was added toluene, followed by concentration under reduced pressure. The obtained residue was diluted with ethyl acetate, and washed with water, and a saturated aqueous sodium chloride solution. The organic layer was dried over anhydrous magnesium sulfate and the solvent was evaporated under reduced pressure. The obtained residue was purified by silica gel column chromatography (hexane:ethyl acetate=9:1 to 1:1) to obtain methyl 3-{4-[acetyl(cyclopropylmethyl)amino]phenoxy}-5-hydroxybenzoate (3.34 g) as a white solid.

### Preparation Example 60

Methyl 3-{4-[acetyl(cyclopropylmethyl)amino]phenoxy}-5-hydroxybenzoate (500 mg), potassium carbonate (292 mg), chloromethyl (methyl)sulfide (0.175 mL), and DMF (5 mL) were mixed, followed by stirring at an oil bath temperature of 60°C for 2 hours and further stirring at an oil bath temperature of 80°C for 1 hour. Further, potassium carbonate (292 mg) and chloromethyl(methyl)sulfide (0.175 mL) were added thereto, followed by stirring at an oil bath temperature of 80°C for 1 hour. The reaction mixture was cooled to room temperature, and water was added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (hexane:ethyl acetate=9:1 to 4:1).
The obtained oily substance and dichloromethane (10 mL) were mixed, and metachloroperbenzoic acid (75%, 1.30 g) was added thereto under ice-cooling, followed by stirring at room temperature for 2 hours. To the reaction mixture was added an aqueous sodium sulfite solution, followed by extraction with ethyl acetate. The organic layer was washed with water, a 1M aqueous sodium hydroxide solution, and a saturated aqueous sodium chloride solution, and dried over anhydrous magnesium sulfate, and the solvent was evaporated under reduced pressure. The obtained residue was purified by silica gel column chromatography (hexane:ethyl acetate=1:1 to 0:1) to obtain methyl 3-{4-[acetyl(cyclopropylmethyl)amino]phenoxy}-5-[(methylsulfonyl)methoxy]benzoate (489 mg) as a colorless oily substance.

### Preparation Example 61

Methyl 3-{4-[acetyl(cyclopropylmethyl)amino]phenoxy}-5-hydroxybenzoate (300 mg), potassium carbonate (175 mg), bromoacetonitrile (152 mg), and DMF (3 mL) were mixed, followed by stirring at an oil bath temperature of 60°C for 1 hour. The reaction mixture was cooled to room temperature, and water was added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (hexane:ethyl acetate=9:1 to 0:1). The obtained residue and methanol (6 mL) were mixed, and a 1 M aqueous sodium hydroxide solution (1.01 mL) was added thereto, followed by stirring at room temperature for 1 hour and leaving to stand for 3 days. The reaction mixture was concentrated under reduced pressure, and water and 1 M hydrochloric acid were added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (chloroform:methanol=10:0 to 19:1) to obtain an oily substance.
The obtained oily substance and 1-methyl-1H-pyrazole-3-amine (164 mg), EDCI (243 mg), HOBt (171 mg), and DMF (6 mL) were mixed, followed by stirring at an oil bath temperature of 60°C for 1 hour. To the reaction mixture was added water, followed by extraction with ethyl acetate. The organic layer was washed with water, 1 M hydrochloric acid, a 1 M aqueous sodium hydroxide solution, and a saturated aqueous sodium chloride solution, and dried over anhydrous magnesium sulfate, and the solvent was evaporated under reduced pressure. The obtained residue was purified by silica gel column chromatography (hexane:ethyl acetate=1:1 to 0:1).
The obtained residue and methanol (6 mL) were mixed, and a 1 M aqueous sodium hydroxide solution (1.01 mL) was added thereto, followed by leaving to stand at room temperature for 30 minutes. The reaction mixture was concentrated under reduced pressure, and water and 1 M hydrochloric acid were added thereto, followed by extraction with ethyl acetate. The organic layer was washed with water and a saturated aqueous sodium chloride solution, and dried over anhydrous magnesium sulfate, and the solvent was evaporated under reduced pressure. The obtained solid was washed with ethyl acetate and filtered to obtain (3-{4-[acetyl(cyclopropylmethyl)amino]phenoxy}-5-[(1-methyl-1H-pyrazol-3-yl)carbamoyl]phenoxy)acetic acid (103 mg) as a white solid.

### Preparation Example 62

To a solution of methyl 3-bromo-5-hydroxybenzoate (11.1 g) in DMF (100 mL) were added potassium carbonate (9.96 g) and tert-butyl 5-chloropyrazine 2-carboxylate (10.8 g) at room temperature, followed by stirring at 60°C for 2 hours. After returning to room temperature, water was added thereto, followed by extraction with ethyl acetate. The organic layer was washed with saturated brine, dried over anhydrous magnesium sulfate, and concentrated to obtain a yellow oily substance (23.53 g). To a solution of the obtained oily substance in dichloromethane (100 mL) was added trifluoroacetic acid (100 mL) at room temperature, followed by stirring at room temperature for 3 hours. After concentration, the obtained solid was collected by filtration while washing with a solvent (hexane:ethyl acetate=1:1) to obtain 5-[3-bromo-5-(methoxycarbonyl)phenoxy]pyrazine-2-carboxylic acid (14.98 g) as a white solid.

### Preparation Example 63

To a solution of methyl 3-bromo-5-({5-[(tert-butoxycarbonyl)(cyclopropylmethyl)amino]pyrazin-2-yl}oxy)benzoate (3 g) in dichloromethane (10 mL) was added trifluoroacetic acid (10 mL) at room temperature, followed by stirring at room temperature for 3 hours. After concentration, a saturated aqueous sodium hydrogen carbonate solution was added thereto, followed by extraction with ethyl acetate. The organic layer was washed with saturated brine, dried over anhydrous magnesium sulfate, and concentrated under reduced pressure. To a solution of the obtained residue in methanol (30 mL) was added a 4 M aqueous sodium hydroxide solution (4.7 mL), followed by stirring at room temperature for 2 hours. 1 M hydrochloric acid was added thereto at room temperature to adjust the pH to 4, and water (150 mL) was added thereto. The obtained solid was collected by filtration while washing with a solvent (water:methanol=5:1) to obtain 3-bromo-5-({5-[(cyclopropylmethyl)amino]pyrazin-2-yl}oxy)benzoic acid (2.22 g) as a light yellow solid.

### Preparation Example 64

A solution of methyl 3-bromo-5-({5-[(tert-butoxycarbonyl)(cyclopropylmethyl)amino]pyrazin-2-yl}oxy)benzoate (1.5 g), potassium carbonate (2.17 g), trimethyboroxine (394 mg), and tetrakistriphenyl phosphine palladium (362 mg) in 1,4-dioxane (20 mL)-water (5 mL) solution was stirred at 110°C for 7 hours. After returning to room temperature, water was added thereto, followed by extraction with ethyl acetate, and the organic layer was washed with saturated brine. The organic layer was dried over anhydrous magnesium sulfate and concentrated. The obtained crude product was purified by silica gel column chromatography (hexane:ethyl acetate=1:0 to 5:1) to obtain methyl 3-({5-[(tert-butoxycarbonyl)(cyclopropylmethyl)amino]pyrazin-2-yl}oxy)-5-methylbenzoate (1.185 g) as a white solid.

### Preparation Example 65

To a solution of tert-butyl 5-[3-hydroxy-5-(methoxycarbonyl)phenoxy]pyrazine-2-carboxylate (8.87 g) in DMF (90 mL) were added potassium carbonate (4.25 g) and benzylbromide (3.7 mL) under ice-cooling, followed by stirring at 60°C for 2 hours. Water was added thereto under ice-cooling, followed by extraction with ethyl acetate. The organic layer was washed with saturated brine, dried over anhydrous magnesium sulfate, and concentrated, and the obtained crude product was purified by silica gel column chromatography (hexane:ethyl acetate=10:1, 5:1, 4:1) to obtain tert-butyl 5-[3-(benzyloxy)-5-(methoxycarbonyl)phenoxy]pyrazine-2-carboxylate as a colorless oily substance (14.509 g, containing about 15% of ethyl acetate in terms of ratio by weight (determined in accordance with ¹H NMR)).

### Preparation Example 66

A solution of methyl 3-(benzyloxy)-5-({5-[(tert-butoxycarbonyl)(cyclopropylmethyl)amino]pyrazin-2-yl}oxy)benzoate (2.1 g), 10% palladium/carbon (420 mg) in ethyl acetate (25 mL) was stirred for 8 hours under a hydrogen atmosphere of 0.3 MPa, and filtered using Celite, and the filtrate was concentrated to obtain methyl 3-({5-[(tert-butoxycarbonyl)(cyclopropylmethyl)amino]pyrazin-2-yl}oxy)-5-hydroxybenzoate (1.722 g) as a white solid.

### Preparation Example 67

To a solution of methyl 3-({5-[(tert-butoxycarbonyl)(cyclopropylmethyl)amino]pyrazin-2-yl}oxy)-5-hydroxybenzoate (753 mg) in DMF (10 mL) were added potassium carbonate (376 mg) and ethyl iodide (0.29 mL) at room temperature, followed by stirring 60°C for 2 hours. After leaving to stand for cooling to room temperature, water was added thereto, followed by extraction with ethyl acetate. The organic layer was washed with saturated brine, dried over anhydrous magnesium sulfate, and concentrated to obtain a light yellow oily substance. To a solution of the obtained oily substance in dichloromethane (10 mL) was added trifluoroacetic acid (2.8 mL) at room temperature, followed by stirring at room temperature for 2 hours and concentrating. A saturated aqueous sodium hydrogen carbonate solution was added thereto, followed by extraction with ethyl acetate. The organic layer was washed with saturated brine, dried over anhydrous magnesium sulfate, and concentrated. To a mixed solution of the obtained residue in methanol (10 mL) and tetrahydrofuran (3 mL) was added a 4 M aqueous sodium hydroxide solution (2.3 mL), followed by stirring at room temperature overnight. 1 M hydrochloric acid was added thereto at room temperature to adjust the pH to 4, and saturated brine was added thereto, followed by extraction with chloroform. The organic layer was dried over anhydrous magnesium sulfate and concentrated to obtain 3-({5-[(cyclopropylmethyl)amino]pyrazin-2-yl}oxy)-5-ethoxybenzoic acid (595 mg) as a light yellow amorphous substance.

### Preparation Example 68

To a mixture of methyl 3-amino-5-(benzyloxy)benzoate hydrochloride (15 g) and chloroform (150 mL) were sequentially added cyclopropane carboxaldehyde (3 g) and sodium triacetoxyborohydride (14 g), followed by stirring for 2 hours. Then, to the reaction mixture was added water, the organic layer was separated, and the obtained organic layer was washed with water, a saturated aqueous sodium hydrogen carbonate solution, and saturated brine. The organic layer was dried over anhydrous magnesium sulfate and concentrated, and the residue was then purified by silica gel column chromatography (5-15% ethyl acetate/hexane) to obtain methyl 3-(benzyloxy)-5-[(cyclopropylmethyl)amino]benzoate (10 g) as a light brown oily substance.

### Preparation Example 69

To methyl 3-(benzyloxy)-5-[(cyclopropylmethyl)amino]benzoate (10 g) were added chloroform (100 mL), acetic anhydride (10 mL), and pyridine (10 mL), followed by stirring for 3 hours. The reaction mixture was diluted with water and ethyl acetate, and the organic layer was washed with water, a saturated aqueous sodium hydrogen carbonate solution, and saturated brine. The residue was purified by silica gel column chromatography (10 to 30% ethyl acetate/hexane) to obtain methyl 3-[acetyl(cyclopropylmethyl)amino]-5-(benzyloxy)benzoate (10.1 g) as a colorless oily substance.

### Preparation Example 70

To a solution of methyl 3-[acetyl(cyclopropylmethyl)amino]-5-(benzyloxy)benzoate (10.1 g) in methanol (50 mL) and THF (50 mL) was added 20% palladium hydroxide (500 mg), followed by stirring for 6 hours under a hydrogen atmosphere of 0.5 MPa. The reaction mixture was filtered using Celite and concentrated to obtain methyl 3-[acetyl(cyclopropylmethyl)amino]-5-hydroxybenzoate (7.3 g).

### Preparation Example 71

A mixture of tetrahydro-2H-pyran-4-carboxylic acid (200 mg), oxalyl chloride (0.13 mL), DMF (0.010 mL), and dichloromethane (0.85 mL) was stirred at room temperature for 2 hours. To the reaction mixture were added methyl 3-[acetyl(cyclopropylmethyl)amino]-5-({5-[(cyclopropylmethyl)amino]pyrazin-2-yl}oxy)benzoate (170 mg) and 4-dimethylaminopyridine (101 mg), followed by stirring at room temperature for 4 hours. To the reaction mixture was added hydrochloric acid, followed by extraction with ethyl acetate. The organic layer was washed with saturated brine and dried over anhydrous sodium sulfate. The solvent was evaporated and the obtained residue was purified by silica gel column chromatography (ethyl acetate:hexane=20:80) to obtain a light yellow oily substance. A mixture of the obtained oily substance, a 1 M aqueous sodium hydroxide solution (1 mL), methanol (1 mL), and tetrahydrofuran (1 mL) was stirred at room temperature for 2 hours. To the reaction mixture was added 1 M hydrochloric acid to adjust the pH to 4, followed by extraction with ethyl acetate. The organic layer was washed with saturated brine, and dried over anhydrous sodium sulfate. The solvent was evaporated to obtain 3-[acetyl(cyclopropylmethyl)amino]-5-({5-[(cyclopropylmethyl)(tetrahydro-2H-pyran-4-ylcarbonyl)amino]pyrazin-2-yl}oxy)benzoic acid (175 mg) as a white amorphous substance.

### Preparation Example 72

To a solution of methyl 3-(4-aminophenoxy)-5-[(1S)-2-methoxy-1-methylethoxy]benzoate (6.22 g) in dichloroethane (70 mL) were added cyclopropane carboxaldehyde (2.0 mL) and acetic acid (6.7 mL) at room temperature, followed by stirring at room temperature for 30 minutes. Sodium triacetoxyborohydride (5.89 g) was added thereto, followed by stirring at room temperature overnight. Water was added thereto at room temperature, followed by extraction with ethyl acetate. The organic layer was washed with a saturated aqueous sodium hydrogen carbonate solution and saturated brine, dried over anhydrous magnesium sulfate, and concentrated under reduced pressure to obtain a light yellow oily substance. To a solution of tetrahydro-2H-pyran-4-carboxylic acid (2.44 g) in dichloromethane (50 mL) were added oxalyl chloride (1.66 mL) and DMF (3 droplets) under ice-cooling, followed by stirring at room temperature for 2 hours. Then, a solution of the previously obtained light yellow oily substance in dichloromethane (30 mL) and pyridine (3 mL) was added thereto under ice-cooling, followed by stirring at room temperature for 2 hours. Water was added thereto under ice-cooling, followed by extraction with ethyl acetate. The organic layer was washed with 1 M hydrochloric acid, a saturated aqueous sodium hydrogen carbonate solution, and saturated brine, dried over anhydrous magnesium sulfate, and concentrated under reduced pressure. The obtained oily substance was purified by silica gel column chromatography (hexane:ethyl acetate=3:1 to 1:2) to obtain methyl 3-{4-[(cyclopropylmethyl)(tetrahydro-2H-pyran-4-ylcarbonyl)amino]phenoxy}-5-{[(2S)-1-methoxypropan-2-yl]oxy}benzoate (5.526 g) as a pink oily substance.

### Preparation Example 73

A mixture of tetrahydro-2H-pyran-4-carboxylic acid (328 mg), oxalyl chloride (0.22 mL), DMF (0.019 mL), and dichloromethane (2 mL) was stirred at room temperature for 2 hours. To the reaction mixture were added methyl 3-({5-[(cyclopropylmethyl)amino]pyrazin-2-yl}oxy)-5-isopropoxybenzoate (180 mg) and 4-dimethylaminopyridine (123 mg), followed by stirring at 50°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=40:60) to obtain methyl 3-({5-[(cyclopropylmethyl)(tetrahydro-2H-pyran-4-ylcarbonyl)amino]pyrazin-2-yl}oxy)-5-isopropoxybenzoate (165 mg) as a light yellow oily substance.

### Preparation Example 74

A mixture of 3-({5-[(cyclopropylmethyl)amino]pyrazin-2-yl}oxy)-5-{[(2S)-1-methoxypropan-2-yl]oxy}benzoic acid (1.9 g), 1-methyl-1H-pyrazole-3-amine (988 mg), EDCI (1.46 g), HOBt (1.03 g), and DMF (10 mL) was stirred at room temperature overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:chloroform=70:30) to obtain 3-({5-[(cyclopropylmethyl)amino]pyrazin-2-yl}oxy)-5-{[(2S)-1-methoxypropan-2-yl]oxy}-N-(1-methyl-1H-pyrazol-3-yl)benzamide (1.4 g) as a light yellow amorphous substance.

### Preparation Example 75

A mixture of methyl 3-({5-[(cyclopropylmethyl)(tetrahydro-2H-pyran-4-ylcarbonyl)amino]pyrazin-2-yl}oxy)-5-{[(2S)-1-methoxypropan-2-yl]oxy}benzoate (180 mg), a 1 M aqueous sodium hydroxide solution (2 mL), ethanol (2 mL), 2-propanol (2 mL), and tetrahydrofuran (2 mL) was stirred at room temperature for 20 minutes. To the reaction mixture was added 1 M hydrochloric acid to adjust the pH to 4, followed by extraction with chloroform. The organic layer was washed with saturated brine and dried over anhydrous sodium sulfate, and the solvent was evaporated to obtain a white amorphous substance. A mixture of this amorphous substance, tert-butyl 3-amino-1H-pyrazole-1-carboxylate (66 mg), N-[(dimethylamino)(3H-[1,2,3]triazolo[4,5-b]pyridin-3-yloxy)methylene]-N-methylmethaneamminium hexafluorophosphate (137 mg), DMF (0.5 mL), and 4-dimethylaminopyridine (44 mg) was stirred at 50°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=50:50) to obtain tert-butyl 3-{[3-({5-[(cyclopropylmethyl)(tetrahydro-2H-pyran-4-ylcarbonyl)amino]pyrazin-2-yl}oxy)-5-{[(2S)-1-methoxypropan-2-yl]oxy}benzoyl]amino}-1H-pyrazole-1-carboxylate (200 mg) as a white amorphous substance.

### Preparation Example 76

To a mixture of tert-butyl 5-[3-isopropoxy-5-(methoxycarbonyl)phenoxy]pyridine-2-carboxylate (1.05 g), and chloroform (4 mL) was added trifluoroacetic acid (4 mL), followed by stirring at 50°C for 4 hours. After leaving to stand for cooling to room temperature, the solvent was evaporated under reduced pressure and azeotroped with toluene to obtain a light yellow oily substance. To the obtained oily substance were added tert-butanol (15 mL), triethylamine (0.58 mL), and diphenyl phosphoryl azide (0.73 mL), followed by stirring at 90°C for 5 hours. After leaving to stand for cooling to room temperature, to the reaction mixture was added water, followed by extraction with ethyl acetate. The organic layer was washed with saturated brine and dried over anhydrous magnesium sulfate, and the solvent was then evaporated under reduced pressure. The residue was purified by silica gel column chromatography (hexane/ethyl acetate=80/20) to obtain methyl 3-({6-[(tert-butoxycarbonyl)amino]pyridin-3-yl}oxy)-5-isopropoxybenzoate (350 mg) as a colorless solid.

### Preparation Example 77

To a solution of methyl 3-({6-[(tert-butoxycarbonyl)amino]pyridin-3-yl}oxy)-5-isopropoxybenzoate (312 mg) in dimethylformamide (5 mL) was added 60% sodium hydride (210 mg), followed by stirring at room temperature for 15 minutes. To the reaction mixture was added (bromomethyl)cyclopropane (0.3 mL), followed by stirring at the same temperature for 2 hours. To the reaction mixture was added a 10% aqueous citric acid solution, followed by extraction with ethyl acetate. The organic layer was washed with water and saturated brine, dried over anhydrous magnesium sulfate, and then concentrated under reduced pressure. The residue was purified by silica gel column chromatography (hexane/ethyl acetate=6/4-3/7) to obtain 3-({6-[(tert-butoxycarbonyl)(cyclopropylmethyl)amino]pyridin-3-yl}oxy)-5-isopropoxybenzoic acid (269 mg) as a colorless amorphous substance.

### Preparation Example 78

A mixture of methyl 3-({5-[(tert-butoxycarbonyl)(cyclopropylmethyl)amino]pyrazin-2-yl}oxy)-5-hydroxybenzoate (200 mg), sodium chlorodifluoroacetate (184 mg), cesium carbonate (235 mg), and DMF (2 mL) was stirred at 100°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=10:90) to obtain methyl 3-({5-[(tert-butoxycarbonyl)(cyclopropylmethyl)amino]pyrazin-2-yl}oxy)-5-(difluoromethoxy)benzoate (100 mg) as a light yellow oil.

### Preparation Example 79

3-{[1-({[tert-Butyl (dimethyl)silyl]oxy}methyl)cyclobutyl]oxy}-5-hydroxy-N-(1-methyl-1H-pyrazol-3-yl)benzamide (490 mg), ethyl 5-fluoropyridine-2-carboxylate (231 mg), potassium carbonate (377 mg), and DMF (10 mL) were mixed, followed by stirring at an oil bath temperature of 80°C for 1 hour and then stirring at an oil bath temperature of 120°C for 3 hours. The reaction mixture was cooled to room temperature, diluted with ethyl acetate, washed with water and a saturated aqueous sodium chloride solution, and dried over anhydrous magnesium sulfate, and the solvent was evaporated under reduced pressure.
The obtained residue and THF (10 mL) were mixed, and tetrabutylammonium fluoride (1 M THF solution; 1.14 mL) was added thereto, followed by stirring at room temperature for 1 hour, diluting with ethyl acetate, washing with water and then washing with a saturated aqueous sodium chloride solution, and drying over anhydrous magnesium sulfate. Then solvent was evaporated under reduced pressure. The obtained residue was purified by silica gel column chromatography (hexane:ethyl acetate=1:1 to 0:1) to obtain ethyl 5-(3-{[1-(hydroxymethyl)cyclobutyl]oxy}-5-[(1-methyl-1H-pyrazol-3-yl)carbamoyl]phenoxy)pyridine-2-carboxylate (419 mg) as a white amorphous substance.

The Preparation Example Compounds 80 to 193 were prepared in the same manner as the methods of Preparation Examples 1 to 79. For the Preparation Example Compounds, the structures are shown in Tables 4 to 50 below, and the preparation methods and the physicochemical data are shown in Tables 96 to 101.

### Example 1

To a solution of ethyl 5-[5-(3-{[(2S)-1-methoxypropan-2-yl]oxy}-5-[(1-methyl-1H-pyrazol-3-yl)carbamoyl]phenoxy)pyridin-2-yl]-1,2,4-oxadiazole-3-carboxylate (320 mg) in THF (4 mL) were added a 28% aqueous ammonia solution (1 mL) and methanol (0.6 mL), followed by stirring for 0.5 hours. The solid collected by filtration after concentration and then dilution with water was washed with ethyl acetate to obtain 5-[5-(3-{[(2S)-1-methoxypropan-2-yl]oxy}-5-[(1-methyl-1H-pyrazol-3-yl)carbamoyl]phenoxy)pyridin-2-yl]-1,2,4-oxadiazole-3-carboxamide (260 mg) as a colorless solid.

### Example 2

Ethyl 5-(5-{3-isopropoxy-5-[(1-methyl-1H-pyrazol-3-yl)carbamoyl]phenoxy}pyridin-2-yl)-1,2,4-oxadiazole-3-carboxylate (100 mg), ethanol (3 mL), and a 2 M methylamine/THF solution (1.01 mL) were mixed, stirred at room temperature for 1 hour, and left to stand overnight. The reaction mixture was concentrated under reduced pressure and purified by silica gel column chromatography (hexane:ethyl acetate=9:1 to 0:1). The obtained residue was dissolved in ethyl acetate, added dropwise to hexane, and concentrated under reduced pressure. The obtained solid was washed with diisopropyl ether and collected by filtration to obtain 5-(5-{3-isopropoxy-5-[(1-methyl-1H-pyrazol-3-yl)carbamoyl]phenoxy}pyridin-2-yl)-N-methyl-1,2,4-oxadiazole-3-carboxamide (66 mg) as a white solid.

### Example 3

To a solution of ethyl 5-(5-{3-isopropoxy-5-[(1-methyl-1H-pyrazol-3-yl)carbamoyl]phenoxy}pyridin-2-yl)-1,3,4-oxadiazole-2-carboxylate (1.035 g) in THF (10 mL) was added a 2 M methylamine/THF solution (10.5 mL) at room temperature, followed by stirring at room temperature for 2 hours. After concentration, the obtained crude product was purified by silica gel column chromatography (hexane:ethyl acetate=1:1 to 1:10) to obtain a light yellow oily substance. A solution of the obtained yellow oily substance in ethyl acetate (30 mL) was heated to reflux, and hexane (25 mL) was added thereto, followed by leaving to stand for cooling to room temperature and then stirring at room temperature overnight. The precipitated solid was collected by filtration while washing with a solvent (hexane:ethyl acetate=5:6) to obtain 5-(5-{3-isopropoxy-5-[(1-methyl-1H-pyrazol-3-yl)carbamoyl]phenoxy}pyridin-2-yl)-N-methyl-1,3,4-oxadiazole-2-carboxamide (774 mg) as a white solid.

### Example 4

Ethyl 3-[5-(3-{[(2S)-1-methoxypropan-2-yl]oxy}-5-[(1-methyl-1H-pyrazol-3-yl)carbamoyl]phenoxy)pyridin-2-yl]-1,2,4-oxadiazole-5-carboxylate (270 mg), ethanol (6 mL), and cyclopropanamine (0.4 mL) were mixed, followed by stirring at room temperature for one hour and a half. The reaction mixture was concentrated under reduced pressure and the obtained residue was purified by silica gel column chromatography (70 to 100% ethyl acetate/hexane) to obtain N-cyclopropyl-3-[5-(3-{[(2S)-1-methoxypropan-2-yl]oxy}-5-[(1-methyl-1H-pyrazol-3-yl)caxbamoyl]phenoxy)pyridin-2-yl]-1,2,4-oxadiazole-5-carboxamide (265 mg) as a colorless solid.

### Example 5

Ethyl 3-(4-{3-[(1-methyl-1H-pyrazol-3-yl)carbamoyl]phenoxy}phenyl)-1,2,4-oxadiazole-5-carboxylate (200 mg) and NMP (4 mL) were mixed, and azetidine hydrochloride (216 mg) and triethylamine (0.325 mL) were added thereto, followed by stirring at an oil bath temperature of 80°C for 1 hour. Further, azetidine hydrochloride (216 mg) and 1,8-diazabicyclo[5.4.0]undec-7-ene (DBU) (0.690 mL) were added thereto, followed by stirring at an oil bath temperature of 80°C for 1 hour. The reaction mixture was cooled to room temperature, and water and 1 M hydrochloric acid were added thereto, followed by extraction with ethyl acetate. The organic layer was washed with 1 M hydrochloric acid, water, and a saturated aqueous sodium chloride solution, and dried over anhydrous magnesium sulfate, and the solvent was evaporated under reduced pressure. The obtained residue was purified by silica gel column chromatography (hexane:ethyl acetate=1:1 to 0:1). The obtained solid was washed with ethyl acetate and collected by filtration to obtain 3-(4-[5-(azetidin-1-ylcarbonyl)-1,2,4-oxadiazol-3-yl]phenoxy)-N-(1-methyl-1H-pyrazol-3-yl)benzamide (60 mg) as a white solid.

### Example 6

Ethyl 3-(3-chloro-4-(3-[(1-methyl-1H-pyrazol-3-yl)carbamoyl]phenoxy)phenyl)-1,2,4-oxadiazole-5-carboxylate (307 mg), ethanol (6 mL), and THF (4 mL) were mixed, and sodium borohydride (75 mg) was added thereto under ice-cooling, followed by stirring for 15 minutes. The reaction mixture was warmed to room temperature and stirred for 15 minutes. Sodium borohydride (75 mg) was added thereto, followed by stirring for 15 minutes. To the reaction mixture were added water and 1 M hydrochloric acid, followed by stirring for 15 minutes. After extraction with ethyl acetate, the organic layer was washed with water and a saturated aqueous sodium chloride solution, and dried over anhydrous magnesium sulfate. Then, the solvent was evaporated under reduced pressure. The obtained residue was purified by silica gel column chromatography (hexane:ethyl acetate=1:1 to 0:1). The obtained oily substance was dissolved in ethyl acetate and added dropwise to hexane. The solvent was evaporated under reduced pressure. The obtained solid was washed with hexane and collected by filtration to obtain 3-{2-chloro-4-[5-(hydroxymethyl)-1,2,4-oxadiazol-3-yl]phenoxy}-N-(1-methyl-1H-pyrazol-3-yl)benzamide (61 mg) as a white solid.

### Example 7

To a solution of 3-[5-(3-{[(2S)-1-methoxypropan-2-yl]oxy}-5-[(1-methyl-1H-pyrazol-3-yl)carbamoyl]phenoxy)pyridin-2-yl]-N-methyl-1,2,4-oxadiazole-5-carboxamide (230 mg) in acetonitrile (10 mL) was added iodotrimethylsilane (0.32 mL) at room temperature. The reaction mixture was stirred at room temperature overnight, and then a saturated aqueous sodium hydrogen carbonate solution was added thereto under ice-cooling, followed by extraction with ethyl acetate. The organic layer was washed with a saturated aqueous sodium sulfite solution and saturated brine, and then dried over anhydrous magnesium sulfate. After concentration, the obtained crude product was purified by silica gel column chromatography (hexane→ethyl acetate/hexane=1/1→chloroform→chloroform:methanol=10:1) to obtain 3-[5-(3-{[(2S)-1-hydroxypropan-2-yl]oxy}-5-[(1-methyl-1H-pyrazol-3-yl)carbamoyl]phenoxy)pyridin-2-yl]-N-methyl-1,2,4-oxadiazole-5-carboxamide (140 mg) as a colorless amorphous substance.

### Example 8

Ethyl 3-(2-chloro-4-{3-[(1-methyl-1H-pyrazol-3-yl)carbamoyl]phenoxy}phenyl)-1,2,4-oxadiazole-5-carboxylate (218 mg) and NMP (4 mL) were mixed, and a 2 M dimethylamine/THF solution (1.17 mL) was added thereto, followed by stirring at an oil bath temperature of 60°C for 2 hours. The reaction mixture was cooled to room temperature, and water was added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (hexane:ethyl acetate=9:1 to 0:1), and the obtained oily substance was dissolved in ethyl acetate and added dropwise to hexane. The solvent was evaporated under reduced pressure, and the obtained solid was washed with diisopropyl ether and collected by filtration to obtain 3-(2-chloro-4-{3-[(1-methyl-1H-pyrazol-3-yl)carbamoyl]phenoxy}phenyl)-N,N-dimethyl-1,2,4-oxadiazole-5-carboxamide (39 mg) as a white solid.

### Example 9

To a solution of ethyl 5-(5-{3-isopropoxy-5-[(1-methyl-1H-pyrazol-3-yl)carbamoyl]phenoxy}pyridin-2-yl)-1,2,4-oxadiazole-3-carboxylate (297 mg) in THF (4 mL) was added a 28% aqueous ammonia solution (1 mL). Methanol (0.6 mL) was added thereto, followed by stirring for 0.5 hours, concentrating, thereafter, diluting with water, and the solid was collected by filtration. The obtained solid was dissolved in chloroform and methanol, and a 4 M hydrogen chloride/ethyl acetate solution (1 mL) was added thereto. After concentration, the resulting solid was washed with diethyl ether and methanol to obtain 5-(5-{3-isopropoxy-5-[(1-methyl-1H-pyrazol-3-yl)carbamoyl]phenoxy}pyridin-2-yl)-1,2,4-oxadiazole-3-carboxamide hydrochloride (220 mg) as a colorless solid.

### Example 10

Under a nitrogen atmosphere, to THF (3 mL) was added 2 M dimethylamine/THF (0.765 mL), and then diisobutylaluminum hydride (1.01 M solution in toluene, 1.40 mL) was added thereto under ice-cooling, followed by stirring at room temperature for 1 hour. Ethyl 5-(5-{3-isopropoxy-5-[(1-methyl-1H-pyrazol-3-yl)carbamoyl]phenoxy}pyridin-2-yl)-1,2,4-oxadiazole-3-carboxylate (150 mg) was added thereto, followed by stirring at room temperature for 2 hours. To the reaction mixture was added an aqueous potassium hydrogen sulfate solution, followed by extraction with ethyl acetate. The organic layer was washed with a saturated aqueous sodium chloride solution and dried over anhydrous magnesium sulfate, and the solvent was evaporated under reduced pressure. The obtained residue was purified by silica gel column chromatography (hexane:ethyl acetate=2:1 to 0:1). The obtained residue was dissolved in ethyl acetate, added dropwise to hexane, and concentrated under reduced pressure. The obtained residue was washed with hexane and collected by filtration to obtain 5-(5-{3-isopropoxy-5-[(1-methyl-1H-pyrazol-3-yl)carbamoyl]phenoxy}pyridin-2-yl)-N,N-dimethyl-1,2,4-oxadiazole-3-carboxamide (47 mg) as a white amorphous substance.

### Example 11

To a mixture of 3-{[6-(N'-hydroxycarbamimidoyl)pyridin-3-yl]oxy}-5-isopropoxy-N-(1-methyl-1H-pyrazol-3-yl)benzamide (81 mg) and dioxane (2 mL) were sequentially added 1,1'-carbonothioylbis(1H-imidazole) (39 mg) and DBU (44 µL) at room temperature, followed by stirring at room temperature for 15 hours. To the reaction mixture was added 1 M hydrochloric acid (1.2 mL), followed by addition of ethyl acetate (15 mL) and water (15 mL) to carry out liquid separation. The organic layer was washed with 0.1 M hydrochloric acid (15 mL) and saturated brine (15 mL) in this order, dried over anhydrous magnesium sulfate, and then filtered, and the filtrate was concentrated under reduced pressure.
The residue (104 mg) was dissolved in THF (2 mL), and triethylamine (48 µL) and then iodomethane (16 µL) were added thereto under ice-cooling, followed by stirring for 30 minutes under ice-cooling and at room temperature for 15 hours. To the reaction mixture were added diethyl ether (15 mL) and water (15 mL) to carry out liquid separation, and the organic layer was washed with water (15 mL) and saturated brine (15 mL) in this order, and dried over anhydrous magnesium sulfate. The filtrate was concentrated under reduced pressure. The residue was purified by silica gel column chromatography to obtain 3-isopropoxy-N-(1-methyl-1H-pyrazol-3-yl)-5-({6-[5-(methylsulfanyl)-1,2,4-oxadiazol-3-yl]pyridin-3-yl}oxy)benzamide (90 mg) as a white amorphous substance.

### Example 12

3-({6-[3-(Hydroxymethyl)-1,2,4-oxadiazol-5-yl]pyridin-3-yl}oxy)-5-isopropoxy-N-(1-methyl-1H-pyrazol-3-yl)benzamide (240 mg), dichloromethane (5 mL), and triethylamine (0.180 mL) were mixed, and methanesulfonyl chloride (0.050 mL) was added thereto under ice-cooling, followed by stirring for 15 minutes. Further, methanesulfonyl chloride (0.025 mL) was added thereto, followed by stirring for 5 minutes. Further, methanesulfonyl chloride (0.025 mL) was added thereto, followed by stirring for 5 minutes. The reaction mixture was diluted with ethyl acetate, washed with a saturated aqueous sodium hydrogen carbonate solution, water, and a saturated aqueous sodium chloride solution, and dried over anhydrous magnesium sulfate, and the solvent was evaporated under reduced pressure.
The obtained residue and DMF (5 mL) were mixed, and sodium cyanide (53 mg) was added thereto, followed by stirring at an oil bath temperature of 50°C for 1 hour. The reaction mixture was cooled to room temperature, diluted with ethyl acetate, washed with a saturated aqueous sodium hydrogen carbonate solution, water, and a saturated aqueous sodium chloride solution, and dried over anhydrous magnesium sulfate, and the solvent was evaporated under reduced pressure. The obtained residue was purified by silica gel column chromatography (hexane:ethyl acetate=7:3 to 2:8). The obtained solid was washed with hexane and collected by filtration to obtain 3-({6-[3-(cyanomethyl)-1,2,4-oxadiazol-5-yl]pyridin-3-yl}oxy)-5-isopropoxy-N-(1-methyl-1H-pyrazol-3-yl)benzamide (103 mg) as a white solid.

### Example 13

3-({6-[3-(Cyanomethyl)-1,2,4-oxadiazol-5-yl]pyridin-3-yl}oxy)-5-isopropoxy-N-(1-methyl-1H-pyrazol-3-yl)benzamide (53 mg) and THF (3 mL) were mixed, and a 1 M aqueous sodium hydroxide solution (0.255 mL) was added thereto, followed by stirring at room temperature for 1 hour and stirring at an oil bath temperature of 60°C for 1 hour. Further, a 1 M aqueous sodium hydroxide solution (0.125 mL) was added thereto, followed by stirring for 1 hour. Further, a 1 M aqueous sodium hydroxide solution (0.125 mL) was added thereto, followed by stirring for 1 hour. The reaction mixture was cooled to room temperature, and water was added thereto, followed by extraction with ethyl acetate. The organic layer was washed with water, a saturated aqueous ammonium chloride solution, and a saturated aqueous sodium chloride solution, and dried over anhydrous magnesium sulfate, and the solvent was evaporated under reduced pressure. The obtained residue was purified by silica gel column chromatography (ethyl acetate:methanol=1:0 to 19:1). The obtained residue was dissolved in ethyl acetate and added dropwise to hexane. After concentration under reduced pressure, the resulting solid was washed with hexane and collected by filtration to obtain 3-({6-[3-(2-amino-2-oxoethyl)-1,2,4-oxadiazol-5-yl]pyridin-3-yl}oxy)-5-isopropoxy-N-(1-methyl-1H-pyrazol-3-yl)benzamide (21 mg) as a beige solid.

### Example 14

A mixture of 3-{[6-(N'-hydroxycarbamimidoyl)pyridin-3-yl]oxy}-5-isopropoxy-N-(1-methyl-1H-pyrazol-3-yl)benzamide (80 mg), triethylamine (60 µL), and dichloromethane (3.2 mL) was ice-cooled, and cyclopropanecarboxylic acid chloride (21 µL) was added thereto, followed by stirring at the same temperature for 15 minutes and at room temperature for 45 minutes. Then, ethyl acetate (15 mL) and water (15 mL) were added to the reaction mixture to carry out liquid separation, and the organic layer was washed with 0.1 M hydrochloric acid (15 mL), a saturated aqueous sodium hydrogen carbonate solution (15 mL), and saturated brine (15 mL) in this order, dried over anhydrous magnesium sulfate, and then filtered, and the filtrate was concentrated under reduced pressure. To the residue were added dioxane (2 mL) and pyridinium p-toluenesulfonite (2.4 mg), followed by heating and stirring at 100°C for 48 hours, and toluene (4 mL) was added thereto, followed by heating and stirring for an additional 24 hours. The reaction mixture was cooled to room temperature, and ethyl acetate (15 mL) and water (15 mL) were added thereto to carry out liquid separation. The organic layer was washed with 0.1 M hydrochloric acid (15 mL), a saturated aqueous sodium hydrogen carbonate solution (15 mL), and saturated brine (15 mL) in this order, dried over anhydrous magnesium sulfate, and then filtered, and the filtrate was concentrated under reduced pressure. The residue was purified by thin film silica gel chromatography to obtain 3-{[6-(5-cyclopropyl-1,2,4-oxadiazol-3-yl)pyridin-3-yl]oxy}-5-isopropoxy-N-(1-methyl-1H-pyrazol-3-yl)benzamide (34 mg) as a colorless oil.

### Example 15

To a solution of 3-isopropoxy-N-(1-methyl-1H-pyrazol-3-yl)-5-({6-[5-(methylsulfanyl)-1,2,4-oxadiazol-3-yl]pyridin-3-yl}oxy)benzamide (115 mg) in NMP (1 mL) was added a 2 M methylamine/THF solution (1 mL), followed by stirring at room temperature for 24 hours. To the reaction mixture were added diethyl ether (10 mL), ethyl acetate (10 mL), and water (15 mL) to carry out liquid separation. The organic layer was washed with water (15 mL) and saturated brine (15 mL) in this order, dried over anhydrous magnesium sulfate, and then filtered, and the filtrate was concentrated under reduced pressure. The residue was purified by silica gel column chromatography (n-hexane-ethyl acetate=1:1 to 1:2 to 1:3 to 0:1), and the residue was washed with diisopropyl ether to obtain 3-({6-[5-(dimethylamino)-1,2,4-oxadiazol-3-yl]pyridin-3-yl}oxy)-5-isopropoxy-N-(1-methyl-1H-pyrazol-3-yl)benzamide (80 mg) as a pale yellow amorphous substance.

### Example 16

5-[5-(3-{[1-({[tert-Butyl (dimethyl)silyl]oxy}methyl)cyclobutyl]oxy}-5-[(1-methyl-1H-pyrazol-3-yl)carbamoyl]phenoxy)pyrazin-2-yl]-N-methyl-1,2,4-oxadiazole-3-carboxamide (159 mg), and THF (5 mL) were mixed, and 1 M hydrochloric acid (1.26 mL) was added thereto, followed by stirring for 30 minutes. Further, 1 M hydrochloric acid (1.26 mL) was added thereto, followed by stirring at an oil bath temperature of 55°C for 1 hour. The reaction mixture was diluted with ethyl acetate, washed with water, a saturated aqueous sodium hydrogen carbonate solution, and a saturated aqueous sodium chloride solution, dried over anhydrous magnesium sulfate, and concentrated under reduced pressure. The obtained residue was purified by silica gel column chromatography (hexane:ethyl acetate=7:3 to 0:1). The obtained residue was washed with ethyl acetate and collected by filtration to obtain 5-[5-(3-{[1-(hydroxymethyl)cyclobutyl]oxy}-5-[(1-methyl-1H-pyrazol-3-yl)carbamoyl]phenoxy)pyrazin-2-yl]-N-methyl-1,2,4-oxadiazole-3-carboxamide (47 mg) as a white solid.

### Example 17

5-(3-{[1-(Hydroxymethyl)cyclobutyl]oxy}-5-[(1-methyl-1H-pyrazol-3-yl)carbamoyl]phenoxy)pyridine-2-carboxylic acid (264 mg) and NMP (5.3 mL) were mixed, and EDCI (174 mg), 3H-[1,2,3]triazolo[4,5-b]pyridin-3-ol (HOAT) (123 mg), ethyl 2-oxyiminooxamate (120 mg), and triethylamine (0.255 mL) were added thereto, followed by stirring at room temperature for 1 hour, stirring at an oil bath temperature of 80°C for 2 hours and then stirring at 100°C for 3 hours. The reaction mixture was cooled to room temperature, diluted with ethyl acetate, washed with water, a 1 M aqueous sodium hydroxide solution, and a saturated aqueous sodium chloride solution, and dried over anhydrous magnesium sulfate, and the solvent was evaporated under reduced pressure. The obtained residue was purified by silica gel column chromatography (hexane:ethyl acetate=7:3 to 2:8).
The obtained residue and THF (6 mL) were mixed, and a 2 M methylamine/THF solution (1.51 mL) was added thereto, followed by stirring for 1 hour. Further, ethanol (1 mL) and a 2 M methylamine/THF solution (1.51 mL) were added thereto, followed by stirring for 30 minutes. The reaction mixture was concentrated under reduced pressure, diluted with ethyl acetate, washed with water, a saturated aqueous sodium chloride solution, and dried over anhydrous magnesium sulfate, and the solvent was evaporated under reduced pressure. The obtained residue was purified by silica gel column chromatography (ethyl acetate:methanol=1:0 to 19:1). The obtained residue was washed with hexane and collected by filtration to obtain 5-[5-(3-{[1-(hydroxymethyl)cyclobutyl]oxy}-5-[(1-methyl-1H-pyrazol-3-yl)carbamoyl]phenoxy)pyridin-2-yl]-N-methyl-1,2,4-oxadiazole-3-carboxamide (62 mg) as a white amorphous substance.

### Example 18

3-{[5-(Hydrazinocarbonyl)pyrazin-2-yl]oxy}-5-isopropoxy-N-(1-methyl-1H-pyrazol-3-yl)benzamide (393 mg), THF (10 mL), and triethylamine (0.4 mL) were mixed, and ethylchloro(oxo)acetate (0.16 mL) was added thereto under ice-cooling, followed by stirring for 15 minutes. NMP (2 mL) was added thereto, followed by stirring at room temperature for 1 hour. The reaction mixture was diluted with ethyl acetate, and washed with water, and a saturated aqueous sodium chloride solution. The organic layer was concentrated under reduced pressure.
The obtained residue, dichloromethane (8 mL), and pyridine (0.310 mL) were mixed, and under ice-cooling, trifluoromethanesulfonic anhydride (0.320 mL) was added thereto, followed by stirring for 30 minutes. The reaction mixture was diluted with ethyl acetate, washed with water, a saturated aqueous sodium hydrogen carbonate solution, a saturated aqueous ammonium chloride solution, and a saturated aqueous sodium chloride solution, and dried over anhydrous magnesium sulfate, and the solvent was evaporated under reduced pressure. The obtained residue was purified by silica gel column chromatography (hexane:ethyl acetate=7:3 to 3:7).
The obtained residue and ethanol (5 mL) were mixed, and a 2 M methylamine/THF solution (0.480 mL) was added thereto under ice-cooling, followed by stirring for 30 minutes. The reaction mixture was diluted with ethyl acetate, washed with water, and a saturated aqueous sodium chloride solution, and dried over anhydrous magnesium sulfate, and the solvent was evaporated under reduced pressure. The obtained residue was purified by silica gel column chromatography (hexane:ethyl acetate=1:1 to 0:1). The obtained solid was washed with hexane and collected by filtration to obtain 5-(5-{3-isopropoxy-5-[(1-methyl-1H-pyrazol-3-yl)carbamoyl]phenoxy}pyrazin-2-yl)-N-methyl-1,3,4-oxadiazole-2-carboxamide (20 mg) as a white amorphous substance.

### Example 19

To a solution of 3-hydroxy-5-{[(2S)-1-methoxypropan-2-yl]oxy}-N-(1-methyl-1H-pyrazol-3-yl)benzamide (400 mg) in NMP (10 mL) were added potassium carbonate (362 mg) and 5-(5-fluoropyridin-2-yl)-N-methyl-1,3,4-oxadiazole-2-carboxamide (320 mg) at room temperature under a nitrogen air flow, followed by stirring at an oil bath temperature of 110°C for 3 hours. 5-(5-Fluoropyridin-2-yl)-N-methyl-1,3,4-oxadiazole-2-carboxamide (29 mg) was added thereto, followed by stirring at 110°C for 1 hour. After leaving to stand for cooling to room temperature, water was added thereto, followed by extraction with ethyl acetate. The organic layer was washed with water and saturated brine, and dried over anhydrous magnesium sulfate, and then the drying agent was removed. The solvent was evaporated under reduced pressure and the obtained crude product was purified by silica gel column chromatography (hexane:ethyl acetate=1:1 to 1:10) to obtain 5-[5-(3-{[(2S)-1-methoxypropan-2-yl]oxy}-5-[(1-methyl-1H-pyrazol-3-yl)carbamoyl]phenoxy)pyridin-2-yl]-N-methyl-1,3,4-oxadiazole-2-carboxamide (632 mg) as a white amorphous substance.

### Example 20

To a solution of 3-{4-[acetyl(cyclopropylmethyl)amino]phenoxy}-5-isopropoxybenzoic acid (80 mg) in DMF (3 mL) were added 1,3-thiazole-2-amine (45 mg), EDCI (50 mg), and HOBt (30 mg), followed by stirring at room temperature for 4 hours. Water (20 mL) and ethyl acetate (20 mL) were added thereto, and the organic layer was washed with 1 M hydrochloric acid (20 mL), a saturated aqueous sodium hydrogen carbonate solution (20 mL), and saturated brine (20 mL), and dried over anhydrous magnesium sulfate. Then, the solvent was evaporated under reduced pressure to obtain 3-{4-[acetyl(cyclopropylmethyl)amino]phenoxy}-5-isopropoxy-N-1,3-thiazol-2-ylbenzamide (85 mg) as a colorless amorphous substance.

### Example 21

A mixture of 3-{4-[(cyclopropylmethyl)amino]phenoxy}-5-isopropoxy-N-1,3-thiazol-2-ylbenzamide (75 mg), propanoyl chloride (0.027 mL), and pyridine (1 mL) was stirred at room temperature for 2 hours. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=50:50) to obtain 3-{4-[(cyclopropylmethyl)(propionyl)amino]phenoxy}-5-isopropoxy-N-1,3-thiazol-2-ylbenzamide (70 mg) as a white amorphous substance.

### Example 22

A mixture of 3-({5-[(cyclopropylmethyl)amino]pyrazin-2-yl}oxy)-5-isopropoxy-N-(1-methyl-1H-pyrazol-3-yl)benzamide (75 mg), isoxazole-5-carboxylic acid chloride (233 mg), 4-dimethylaminopyridine (217 mg), and dichloromethane (0.5 mL) was stirred at room temperature for 1 hour. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=70:30) to obtain N-(cyclopropylmethyl)-N-(5-{3-isopropoxy-5-[(1-methyl-1H-pyrazol-3-yl)carbamoyl]phenoxy}pyrazin-2-yl)-1,2-oxazole-5-carboxamide (70 mg) as a white amorphous substance.

### Example 23

A mixture oftetrahydro-2H-pyran-4-carboxylic acid (738 mg), oxalyl chloride (0.49 mL), DMF (0.044 mL), and dichloromethane (5 mL) was stirred at room temperature for 30 minutes. To the reaction mixture were added 3-({5-[(cyclopropylmethyl)amino]pyrazin-2-yl}oxy)-5-isopropoxy-N-(1-methyl-1H-pyrazol-3-yl)benzamide (798 mg), and 4-dimethylaminopyridine (400 mg), followed by stirring at room temperature overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=70:30) to obtain N-(cyclopropylmethyl)-N-(5-{3-isopropoxy-5-[(1-methyl-1H-pyrazol-3-yl)carbamoyl]phenoxy}pyrazin-2-yl)tetrahydro-2H-pyran-4-carboxamide (800 mg) as a light yellow amorphous substance.

### Example 24

A mixture of tetrahydro-2H-pyran-4-carboxylic acid (591 mg), oxalyl chloride (0.39 mL), DMF (0.035 mL), and dichloromethane (5 mL) was stirred at room temperature for 20 minutes. To the reaction mixture were added 3-({5-[(cyclopropylmethyl)amino]pyrazin-2-yl}oxy)-N-(1-methyl-1H-pyrazol-3-yl)-5-({(2S)-1-[(triisopropylsilyl)oxy]propan-2-yl}oxy)benzamide (270 mg) and 4-dimethylaminopyridine (555 mg), followed by stirring at room temperature for 1 hour. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=50:50) to obtain a light yellow oil. A mixture of this oil and N,N,N-tributylbutane-1-ammonium fluoride (1 M solution in THF, 2 mL) was stirred at room temperature for 10 minutes, and the solvent was evaporated. The obtained residue was purified by silica gel column chromatography (methanol:chloroform=3:97) to obtain N-(cyclopropylmethyl)-N-[5-(3-{[(2S)-1-hydroxypropan-2-yl]oxy}-5-[(1-methyl-1H-pyrazol-3-yl)carbamoyl]phenoxy)pyrazin-2-yl]tetrahydro-2H-pyran-4-carboxamide (86 mg) as a white amorphous substance.

### Example 25

To a solution of tert-butyl 3-[(3-{4-[acetyl(cyclopropylmethyl)amino]phenoxy}-5-[(1S)-2-methoxy-1-methylethoxy]benzoyl)amino]-1H-pyrazole-1-carboxylate (181 mg) in dichloromethane (10 mL) was added trifluoroacetic acid (0.48 mL) at room temperature, followed by stirring at room temperature overnight, concentrating under reduced pressure, and carrying out liquid separation with ethyl acetate and a saturated aqueous sodium hydrogen carbonate solution. The organic layer was washed with saturated brine, dried over anhydrous magnesium sulfate, and concentrated. The obtained crude product was purified by silica gel column chromatography (hexane:ethyl acetate=1:1, chloroform:methanol=1:0 to 10:1) to obtain 3-{4-[acetyl(cyclopropylmethyl)amino]phenoxy}-5-5-[(1S)-2-methoxy-1-methylethoxy]-N-1H-pyrazol-3-ylbenzamide (133 mg) as a white amorphous substance.

### Example 26

To a solution of 3-{4-[acetyl(cyclopropylmethyl)amino]phenoxy}-5-({1-[(benzyloxy)methyl]cyclobutyl}methoxy)-N-1,3-thiazol-2-ylbenzamide (176 mg) in trifluoroacetic acid (5 mL) was added 1,2,3,4,5-pentamethylbenzene (427 mg) at room temperature, followed by stirring at room temperature overnight. After concentration, a saturated aqueous sodium hydrogen carbonate solution was added thereto, followed by extraction with ethyl acetate, and the organic layer was washed with saturated brine. The organic layer was dried over anhydrous magnesium sulfate and concentrated. The obtained crude product was purified by silica gel column chromatography (hexane:ethyl acetate=2:1 to 1:2) to obtain an oily substance. To a solution of this oily substance in methanol (5 mL) was added a 1 M aqueous sodium hydroxide solution (1 mL) at room temperature, followed by stirring at room temperature for 2 hours, and 1 M hydrochloric acid was added thereto at room temperature, followed by extraction with ethyl acetate. The organic layer was washed with saturated brine. The organic layer was dried over anhydrous magnesium sulfate and concentrated to obtain a colorless oily substance. A solvent (diisopropyl ether:hexane=1:1) was added thereto and the precipitate was collected by filtration to obtain 3-{4-[acetyl(cyclopropylmethyl)amino]phenoxy}-5-{[1-(hydroxymethyl)cyclobutyl]methoxy}-N-1,3-thiazol-2-ylbenzamide (101 mg) as a white solid.

### Example 27

To a solution of 3-{4-[acetyl(cyclopropylmethyl)amino]phenoxy}-5-isopropoxybenzoic acid (220 mg) and methyl 6-aminonicotinate (105 mg) in pyridine (4.4 mL) was added phosphorous oxychloride (0.06 mL) at -5°C, followed by stirring at the same temperature for 0.5 hours. The reaction mixture was stirred at room temperature overnight, and then diluted with ethyl acetate. The organic layer was washed with a 10% aqueous citric acid solution and saturated brine, and dried over anhydrous magnesium sulfate. After concentration, to the residue was added methanol (0.6 mL), followed by stirring for 0.5 hours. After concentration, the residue was purified by silica gel column chromatography (0 to 5% methanol/chloroform) to obtain methyl 6-[(3-{4-[acetyl(cyclopropylmethyl)amino]phenoxy}-5-isopropoxybenzoyl)amino]nicotinate (203 mg) as a colorless amorphous substance.

### Example 28

To a solution of 3-{4-[acetyl(cyclopropylmethyl)amino]phenoxyl-5-[(1S)-2-methoxy-1-methylethoxy]-N-1H-pyrazol-3-ylbenzamide (100 mg) in acetonitrile (5 mL) was added iodo(trimethyl)silane (0.15 mL) under ice-cooling, followed by stirring at room temperature overnight. Then, iodo(trimethyl)silane (0.2 mL) was added thereto at room temperature, followed by stirring at room temperature for 3 hours, and then a saturated aqueous sodium hydrogen carbonate solution was added thereto under ice-cooling, followed by extraction with ethyl acetate. The organic layer was washed with a saturated aqueous sodium thiosulfate solution and saturated brine, dried over anhydrous magnesium sulfate, and concentrated. To a solution of the obtained oily substance in methanol solution (4 mL) was added a 1 M aqueous sodium hydroxide solution (1 mL) at room temperature, followed by stirring at room temperature for 1 hour and neutralizing by the addition of 1 M hydrochloric acid at room temperature. Then, saturated brine was added thereto, followed by extraction with a solvent (chloroform:isopropyl alcohol=4:1). The organic layer was dried over anhydrous magnesium sulfate and concentrated. The obtained crude product was purified by silica gel column chromatography (hexane:ethyl acetate=1:1, chloroform:methanol=1:0 to 10:1). The obtained white amorphous substance was purified by thin film chromatography (chloroform:methanol=10:1) to obtain 3-{4-[acetyl(cyclopropylmethyl)amino]phenoxy}-5-[(1S)-2-hydroxy-1-methylethoxy]-N-1H-pyrazol-3-ylbenzamide (56 mg) as a white amorphous substance.

### Example 29

A mixture of 3-({5-[(cyclopropylmethyl)amino]pyrazin-2-yl}oxy)-5-{[(2S)-1-methoxypropan-2-yl]oxy}-N-(1-methyl-1H-pyrazol-3-yl)benzamide (136 mg), chloroacetyl chloride (0.024 mL), 4-dimethylaminopyridine (37 mg), and dichloromethane (2 mL) was stirred at room temperature for 2 hours. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=70:30) to obtain a white amorphous substance. N,N-Dimethylamine (2 M solution in THF, 2 mL) was added thereto, followed by stirring at room temperature for 2 hours, and the solvent was evaporated. The obtained residue was purified by silica gel column chromatography (methanol:chloroform=4:96) to obtain 3-({5-[(cyclopropylmethyl)(N,N-dimethylglycyl)amino]pyrazin-2-yl}oxy)-5-{[(2S)-1-methoxypropan-2-yl]oxy}-N-(1-methyl-1H-pyrazol-3-yl)benzamide (135 mg) as a white amorphous substance.

### Example 30

A mixture of 3-{4-[acetyl(cyclopropylmethyl)amino]phenoxy}-N-[4-(2,2-dimethyl-1,3-dioxolan-4-yl)-1,3-thiazol-2-yl]-5-isopropoxybenzamide (95 mg), THF (1 mL), and 1 M hydrochloric acid (1 mL) was stirred at 50°C for 1 hour. The reaction mixture was concentrated, then dissolved in chloroform, and washed with saturated brine. The organic layer was dried over anhydrous magnesium sulfate and then concentrated to obtain 3-{4-[acetyl(cyclopropylmethyl)amino]phenoxy}-N-[4-(1,2-dihydroxyethyl)-1,3-thiazol-2-yl]-5-isopropoxybenzamide (75 mg) as a colorless amorphous substance.

### Example 31

A mixture of 2-[(cyclopropylmethyl){4-[3-isopropoxy-5-(1,3-thiazol-2-ylcarbamoyl)phenoxy]phenyl}amino]-2-oxoethylacetate (50 mg), potassium carbonate (13 mg), and methanol (2 mL) was stirred at room temperature for 2 hours. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate) to obtain 3-{4-[(cyclopropylmethyl)(glycoloyl)amino]phenoxy}-5-isopropoxy-N-1,3-thiazol-2-ylbenzamide (40 mg) as a white amorphous substance.

### Example 32

A mixture of 3-{4-[(cyclopropylmethyl)amino]phenoxy}-5-isopropoxy-N-1,3-thiazol-2-ylbenzamide (80 mg), dimethylcarbamoyl chloride (0.076 mL), and pyridine (1 mL) was stirred at 80°C for 3 hours. To the reaction mixture was added 1 M hydrochloric acid, followed by extraction with ethyl acetate. The organic layer was washed with saturated brine and dried over anhydrous sodium sulfate, and the solvent was evaporated. The obtained residue was purified by silica gel column chromatography (ethyl acetate) to obtain a white amorphous substance. A mixture of the obtained amorphous substance, a 1 M aqueous sodium hydroxide solution (1 mL), and methanol (1 mL) was stirred at room temperature for 3 hours. To the reaction mixture was added 1 M hydrochloric acid, followed by extraction with ethyl acetate. The organic layer was washed with saturated brine and dried over anhydrous sodium sulfate, and the solvent was evaporated. The obtained residue was purified by silica gel column chromatography (ethyl acetate) to obtain 3 - {4-[(cyclopropylmethyl)(dimethylcarbamoyl)amino]phenoxy -5 -isopropoxy-N-1,3-thiazol-2-ylbenzamide (30 mg) as a white amorphous substance.

### Example 33

To a solution of 6-[(3-{4-[acetyl(cyclopropylmethyl)amino]phenoxy}-5-isopropoxybenzoyl)amino]nicotinic acid (200 mg) in THF (5 mL) was added N,N' - carbonyldiimidazole (100 mg), followed by stirring at room temperature for 1 hour, and then sodium borohydride (22 mg) was dissolved in about 0.2 mL of water, which was then added to the reaction mixture at -5°C or lower. After stirring at 5°C or lower for 0.5 hours, water and saturated brine were added to the reaction mixture, followed by extraction with ethyl acetate. The organic layer was washed with saturated brine, and then the organic layer was dried over anhydrous magnesium sulfate. After concentration, the residue was purified by silica gel column chromatography (0 to 5% methanol/chloroform) to obtain 3-{4-[acetyl(cyclopropylmethyl)amino]phenoxy}-N-[5-(hydroxymethyl)pyridin-2-yl]-5-isopropoxybenzamide (120 mg).

### Example 34

To a solution of methyl 6-[(3-{4-[acetyl(cyclopropylmethyl)amino]phenoxy}-5-isopropoxybenzoyl)amino]nicotinate (520 mg) in dioxane (5 mL) was added a 1 M aqueous sodium hydroxide solution (1.2 mL), followed by stirring overnight. The reaction mixture was neutralized with 1 M hydrochloric acid and extracted with chloroform. The organic layer was dried over anhydrous magnesium sulfate and then concentrated, and the residue was purified by silica gel column chromatography (0 to 10% methanol/chloroform) to obtain 6-[(3-{4-[acetyl(cyclopropylmethyl)amino]phenoxy}-5-isopropoxybenzoyl)amino]nicotinic acid (430 mg).

### Example 35

(3-(4-[Acetyl(cyclopropylmethyl)amino]phenoxy}-5-[(1-methyl-1H-pyrazol-3-yl)carbamoyl]phenoxy)acetic acid (70 mg), DMF (3 mL), and N,N'-carbonyldiimidazole (48 mg) were mixed, followed by stirring at an oil bath temperature of 50°C for 1 hour. Methanesulfonamide (56 mg) and DBU (0.088 mL) were added, followed by stirring for 2 hours. The reaction mixture was cooled to room temperature, and water and 1 M hydrochloric acid were added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (chloroform:methanol=10:0 to 9:1). The obtained oily substance was dissolved in ethyl acetate and added dropwise to hexane. The solvent was evaporated under reduced pressure. The obtained solid was washed with diisopropyl ether and collected by filtration to obtain 3-{4-[acetyl(cyclopropylmethyl)amino]phenoxy}-N-(1-methyl-1H-pyrazol-3-yl)-5-{2-[(methylsulfonyl)amino]-2-oxo ethoxy}benzamide (40 mg) as a white solid.

### Example 36

Methyl 3-{4-[Acetyl(cyclopropylmethyl)amino]phenoxy}-5-hydroxybenzoate (300 mg), potassium carbonate (467 mg), 2-chloro-N,N-dimethylethanamine hydrochloride (244 mg), and DMF (3 mL) were mixed, followed by stirring at an oil bath temperature of 60°C for 2 hours. The reaction mixture was cooled to room temperature, and water was added thereto, followed by extraction with ethyl acetate. The organic layer was washed with water and a saturated aqueous sodium chloride solution, and dried over anhydrous magnesium sulfate, and the solvent was evaporated under reduced pressure. The obtained residue was purified by silica gel column chromatography (chloroform:methanol=100:0 to 95:5).
The obtained residue and methanol (6 mL) were mixed, and a 1 M aqueous sodium hydroxide solution (1.01 mL) was added thereto, followed by stirring at room temperature for 1 hour. Further, a 1 M aqueous sodium hydroxide solution (1.01 mL) was added thereto, followed by stirring at an oil bath temperature of 60°C for 1 hour. The reaction mixture was returned to room temperature and concentrated under reduced pressure. Water and 1 M hydrochloric acid (2.02 mL) were added thereto, followed by extraction with a mixed solution of chloroform/isopropanol (4:1). The organic layer was washed with water and a saturated aqueous sodium chloride solution, and dried over anhydrous magnesium sulfate, and the solvent was evaporated under reduced pressure.
The obtained residue, 1-methyl-1H-pyrazole-3-amine (164 mg), EDCI (243 mg), HOBt (171 mg), and DMF (6 mL) were mixed, followed by stirring at an oil bath temperature of 60°C for 30 minutes. To the reaction mixture were added water and a 1 M aqueous sodium hydroxide solution, followed by extraction with ethyl acetate. The organic layer was washed with water, a 1 M aqueous sodium hydroxide solution, and a saturated aqueous sodium chloride solution, and dried over anhydrous magnesium sulfate, and the solvent was evaporated under reduced pressure. The obtained residue was purified by silica gel column chromatography (chloroform:methanol=10:0 to 95:5). The obtained oily substance was dissolved in ethyl acetate, and added dropwise to hexane. The solvent was evaporated under reduced pressure to obtain 3-{4-[acetyl(cyclopropylmethyl)amino]phenoxy}-5-[2-(dimethylamino)ethoxy]-N-(1-methyl-1H-pyrazol-3-yl)benzamide (64 mg) as a white amorphous substance.

### Example 37

To a solution of 3-{4-[(cyclopropylmethyl)(tetrahydro-2H-pyran-4-ylcarbonyl)amino]phenoxy}-5-{[(2S)-1-methoxypropan-2-yl]oxy}benzoic acid (497 mg) in dichloromethane (10 mL) were added 1 M oxalyl chloride/dichloromethane (1.13 mL) and DMF (3 droplets) under ice-cooling, followed by stirring at room temperature for 30 minutes. 1-Methyl-1H-pyrazole-3-amine (120 mg) and pyridine (0.17 mL) were added thereto under ice-cooling, followed by stirring at room temperature for 2 hours. Water was added thereto, followed by extraction with ethyl acetate, and the organic layer was washed with 1 M hydrochloric acid, a saturated aqueous sodium hydrogen carbonate solution, and saturated brine, and dried over anhydrous magnesium sulfate. Then, the solvent was evaporated under reduced pressure, and the obtained crude product was purified by silica gel column chromatography (hexane:ethyl acetate=2:1 to 1:10) to obtain N-(cyclopropylmethyl)-N-[4-(3-{[(2S)-1-methoxypropan-2-yl]oxy}-5-[(1-methyl-1H-pyrazol-3-yl)carbamoyl]phenoxy)phenyl]tetrahydro-2H-pyran-4-carboxamide (334 mg) as a white amorphous substance.

### Example 38

To a solution of N-(5-{3-(benzyloxy)-5-[(1-methyl-1H-pyrazol-3-yl)carbamoyl]phenoxy}pyrazin-2-yl)-N-(cyclopropylmethyl)tetrahydro-2H-pyran-4-carboxamide (107 mg) in ethyl acetate (10 mL) was added 10% palladium/carbon (30 mg), followed by stirring for 8 hours under a hydrogen atmosphere of 0.3 MPa. The reaction mixture was filtered using Celite and the filtrate was concentrated. To a solution of the obtained oily substance in ethyl acetate (10 mL) was added 10% palladium/carbon (50 mg), followed by stirring for 8 hours under a hydrogen atmosphere of 0.3 MPa. Further, the reaction mixture was filtered using Celite and the filtrate was concentrated. To a solution of the obtained oily substance in ethyl acetate (10 mL) was added 10% palladium/carbon (50 mg), followed by stirring for 21 hours under a hydrogen atmosphere of 0.3 MPa. Then the reaction mixture was filtrated using Celite and the filtrate was concentrated to obtain N-(cyclopropylmethyl)-N-(5-{3-hydroxy-5-[(1-methyl-1H-pyrazol-3-yl)carbamoyl]phenoxy}pyrazin-2-yl)tetrahydro-2H-pyran-4-carboxamide (70 mg) as a white amorphous substance.

### Example 39

A mixture of methyl 3-({5-[(cyclopropylmethyl)(tetrahydro-2H-pyran-4-ylcarbonyl)amino]pyrazin-2-yl}oxy)-5-isopropoxybenzoate (162 mg), a 1 M aqueous sodium hydroxide solution (2 mL), ethanol (2 mL), and THF (2 mL) was stirred at room temperature for 20 minutes. To the reaction mixture was added hydrochloric acid, followed by extraction with ethyl acetate. The organic layer was washed with saturated brine and dried over anhydrous sodium sulfate, and the solvent was evaporated to obtain a white amorphous substance. A mixture of this amorphous substance, tert-butyl 3-amino-1H-pyrazole-1-carboxylate (121 mg), N-[(dimethylamino)(3H-[1,2,3]triazolo[4,5-b]pyridin-3-yloxy)methylene]-N-methylmethaneaminiumhexafluorophosphate (167 mg), 4-dimethylaminopyridine (54 mg), and DMF (0.4 mL) was stirred at 50°C overnight. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate:hexane=50:50) to obtain a colorless oily substance.
To the obtained oily substance were added trifluoroacetic acid (2 mL) and chloroform (2 mL), followed by stirring at room temperature for 2 hours. The solvent was evaporated and the obtained residue was purified by silica gel column chromatography (ethyl acetate:hexane=85:15) to obtain N-(cyclopropylmethyl)-N-{5-[3-isopropoxy-5-(1H-pyrazol-3-ylcarbamoyl)phenoxy]pyrazin-2-yl}tetrahydro-2H-pyran-4-carboxamide (77 mg) as a light yellow amorphous substance.

### Example 40

A mixture of 3-({5-[(cyclopropylmethyl)amino]pyrazin-2-yl}oxy)-N-(1-methyl-1H-pyrazol-3-yl)-5-({(2S)-1-[(triisopropylsilyl)oxy]propan-2-yl}oxy)benzamide (200 mg), chloroacetyl chloride (0.27 mL), 4-dimethylaminopyridine (411 mg), and dichloromethane (1.3 mL) was stirred at room temperature for 1 hour. To the reaction mixture was added water, followed by extraction with ethyl acetate. The organic layer was washed with saturated brine and dried over anhydrous sodium sulfate, and the solvent was evaporated. The obtained residue was purified by silica gel column chromatography (ethyl acetate) to obtain a white amorphous substance.
To the obtained amorphous substance was added N,N-dimethylamine (2 M solution in THF, 3 mL), followed by stirring at room temperature for 2 hours. The solvent was evaporated, and to the obtained residue was added N,N,N-tributylbutane-1-ammonium fluoride (1 M solution in THF, 2 mL), followed by stirring at room temperature for 5 minutes. The solvent was evaporated and the obtained residue was purified by silica gel column chromatography (methanol/chloroform=8/92) to obtain a light yellow oily substance. To this oily substance was added water, followed by extraction with chloroform. The organic layer was extracted with hydrochloric acid, and the aqueous layer was neutralized with an aqueous sodium hydroxide solution and extracted from chloroform. The organic layer was dried over anhydrous sodium sulfate and the solvent was evaporated. The obtained residue was purified by silica gel column chromatography (methanol/chloroform=8/92) to obtain 3-({5-[(cyclopropylmethyl)(N,N-dimethylglycyl)amino]pyrazin-2-yl}oxy)-5-{[(2S)-1-hydroxypropan-2-yl]oxy}-N-(1-methyl-1H-pyrazol-3-yl)benzamide as a white amorphous substance (69 mg).

The Example Compounds 41 to 177 were prepared in the same manner as the methods of Examples 1 to 40. For the Example Compounds, the structures are shown in Tables 51 to 95 below, and the preparation methods and the physicochemical data are shown in Tables 102 to 110.

Furthermore, the following abbreviations are used in Tables to be described later. PEx: Preparation Example number, Ex: Example number, Data: Physicochemical data (EI: m/z value in EI-MS, FAB+: m/z value in FAB-MS (positive ion), FAB-: m/z value in FAB-MS (negative ion), ESI+: m/z value in ESI-MS (positive ion), ESI-: m/z value in ESI-MS (negative ion), CI+: m/z value in CI-MS (positive ion), m/z value in CI-MS (positive ion), APCI+: m/z value in APCI-MS (positive ion), APCI/ESI+: simultaneous measurement of APCI+ and ESI+, NMR1: δ (ppm) in 1H NMR in DMSO-d₆, NMR2: δ (ppm) in 1H NMR in CDCl₃, Structure: Structural formula (HCl in the structural formula represents hydrochloride), Syn: Production method (the numeral shows that in the same manner as in the Example compound having the number as its Example number, the compound was prepared using the corresponding starting material), PSyn: Production method (the numeral shows that in the same manner as in the Preparation Example compound having the number as its Preparation Example number, the compound was prepared using the corresponding starting material), tBu: tert-butyl, Boc: tert-butoxycarbonyl.
Moreover, 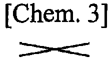 means that the double bond is a mixture of E forms and Z forms.

**[Table 4]**

| PEx | Structure |
|---|---|
| 1 | 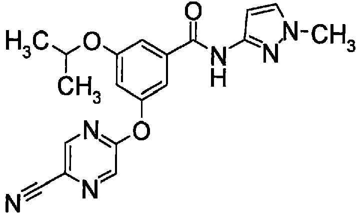 |
| 2 | 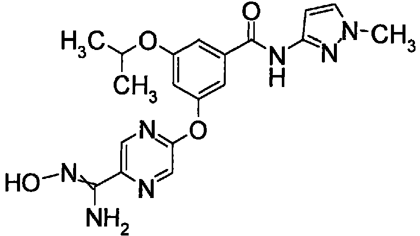 |
| 3 | 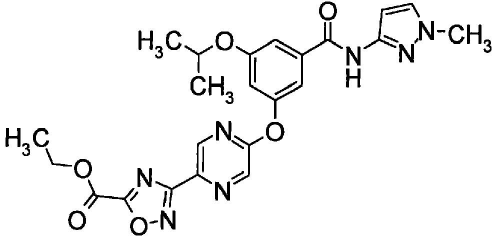 |
| 4 | 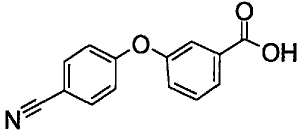 |
| 5 | 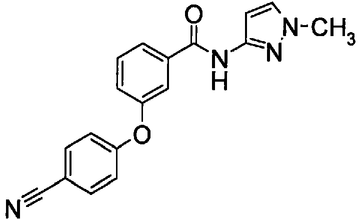 |

**[Table 5]**

| PEx | Structure |
|---|---|
| 6 | 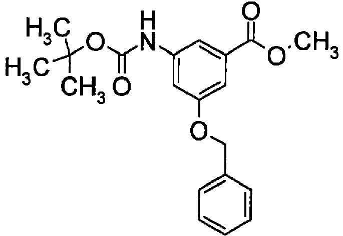 |
| 7 | 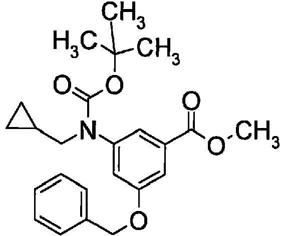 |
| 8 | 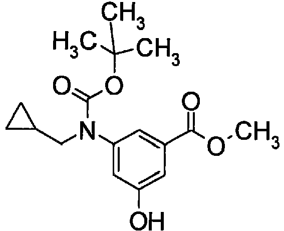 |
| 9 | 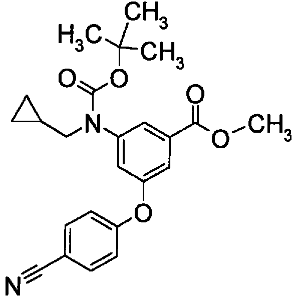 |

**[Table 6]**

| PEx | Structure |
|---|---|
| 10 | 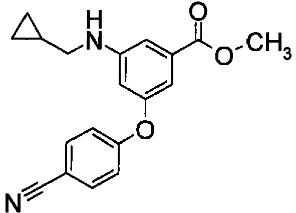 |
| 11 | 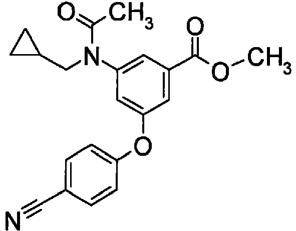 |
| 12 | 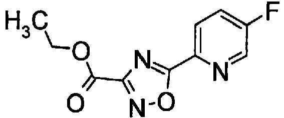 |
| 13 | 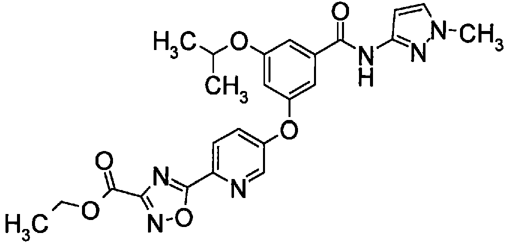 |
| 14 | 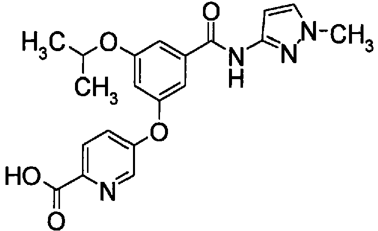 |

**[Table 7]**

| PEx | Structure |
|---|---|
| 15 | 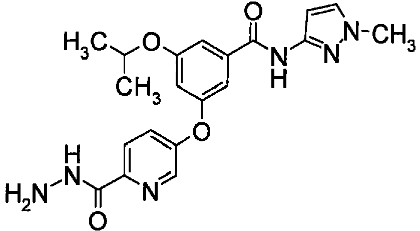 |
| 16 | 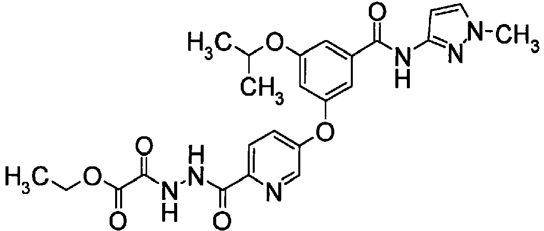 |
| 17 | 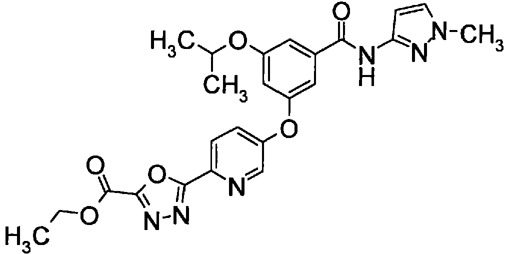 |
| 18 | 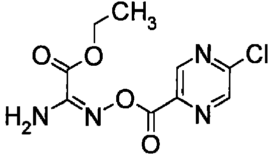 |
| 19 | 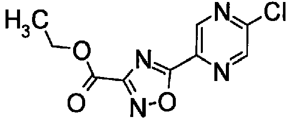 |

**[Table 8]**

| PEx | Structure |
|---|---|
| 20 | 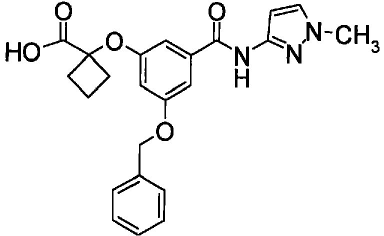 |
| 21 | 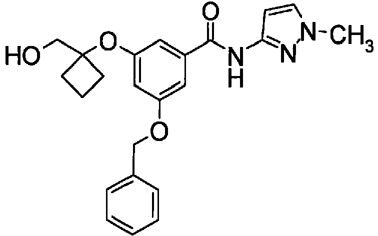 |
| 22 | 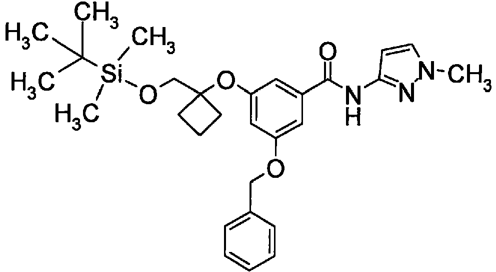 |
| 23 | 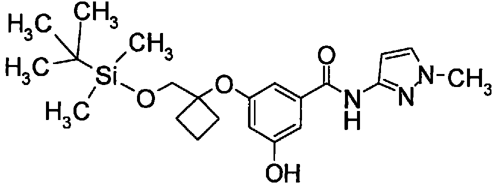 |

**[Table 9]**

| PEx | Structure |
|---|---|
| 24 | 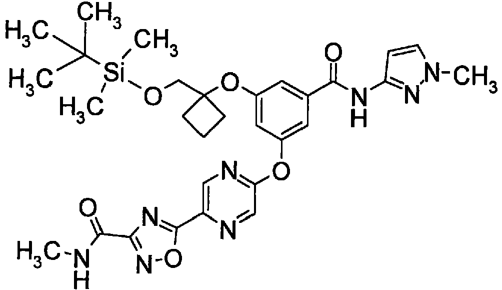 |
| 25 | 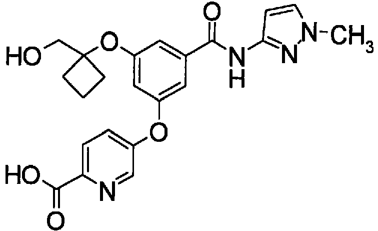 |
| 26 | 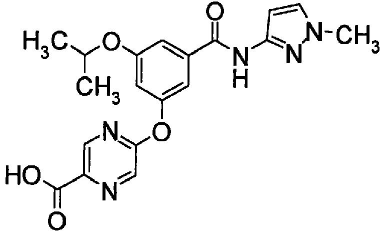 |
| 27 | 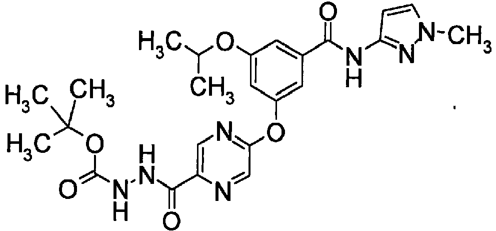 |

**[Table 10]**

| PEx | Structure |
|---|---|
| 28 | 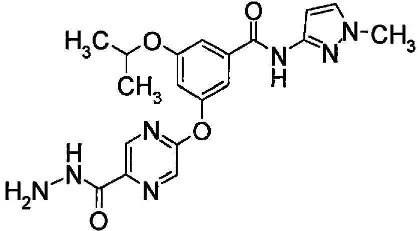 |
| 29 | 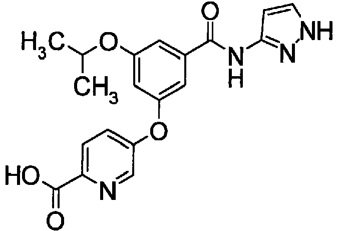 |
| 30 | 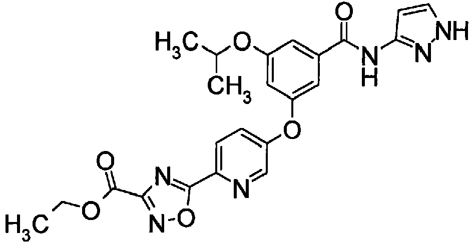 |
| 31 | 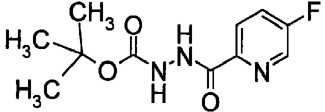 |
| 32 | 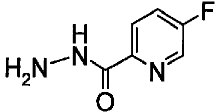 |
| 33 | 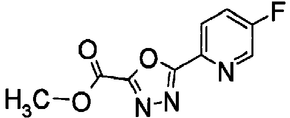 |

**[Table 11]**

| PEx | Structure |
|---|---|
| 34 | 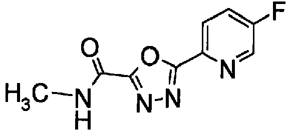 |
| 35 | 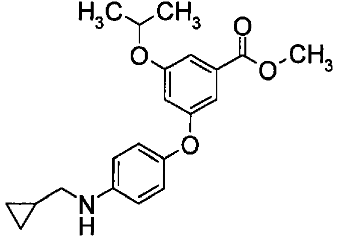 |
| 36 | 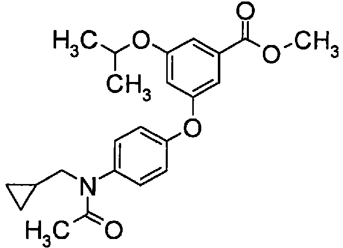 |
| 37 | 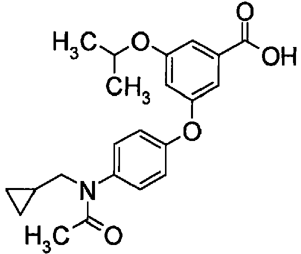 |
| 38 | 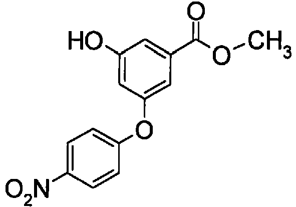 |

**[Table 12]**

| PEx | Structure |
|---|---|
| 39 | 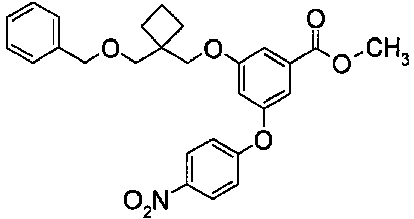 |
| 40 | 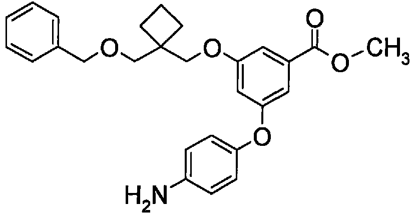 |
| 41 | 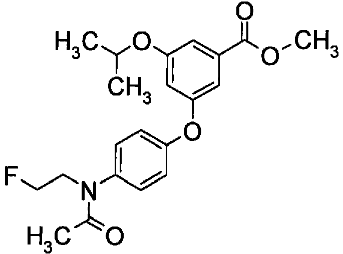 |
| 42 | 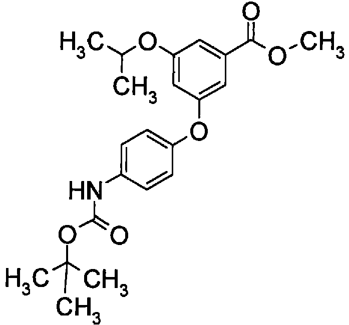 |

**[Table 13]**

| PEx | Structure |
|---|---|
| 43 | 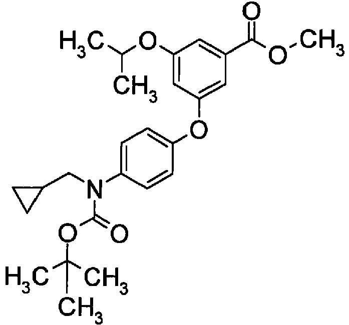 |
| 44 | 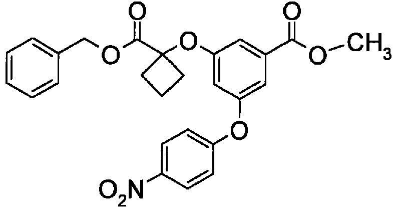 |
| 45 | 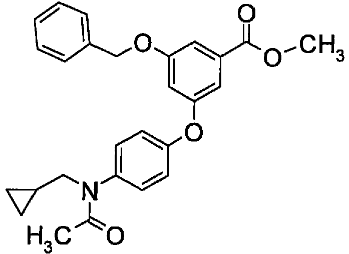 |
| 46 | 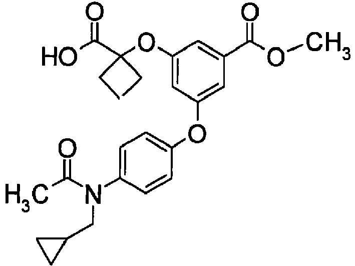 |

**[Table 14]**

| PEx | Structure |
|---|---|
| 47 | 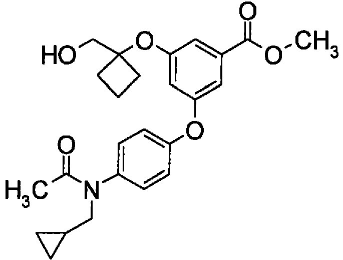 |
| 48 | 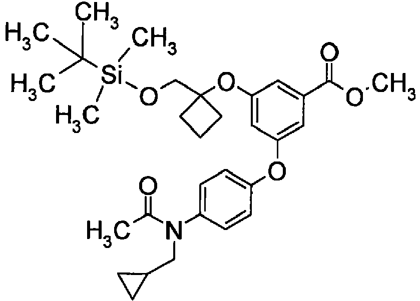 |
| 49 | 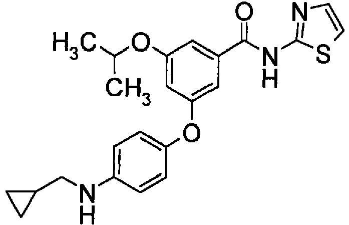 |
| 50 | 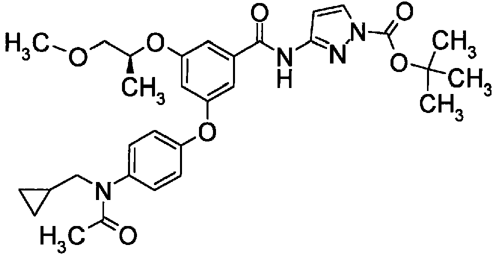 |

**[Table 15]**

| PEx | Structure |
|---|---|
| 51 | 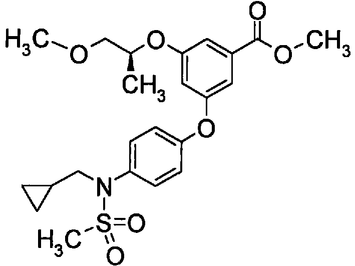 |
| 52 | 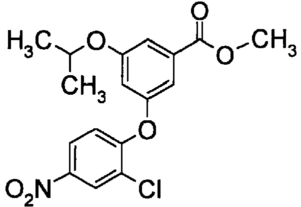 |
| 53 | 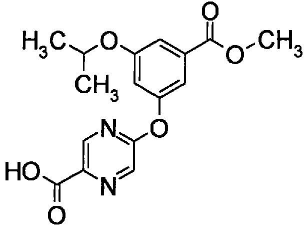 |
| 54 | 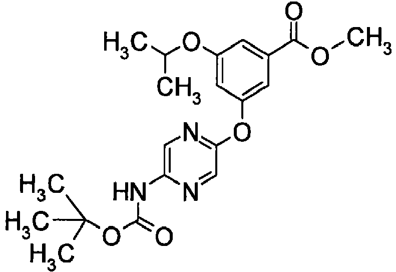 |

**[Table 16]**

| PEx | Structure |
|---|---|
| 55 | 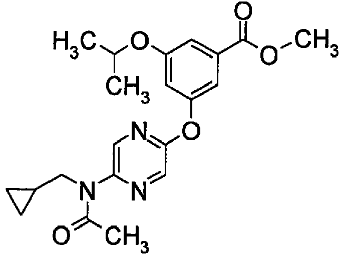 |
| 56 | 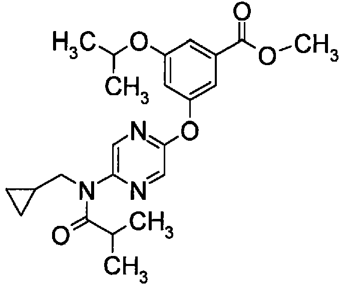 |
| 57 | 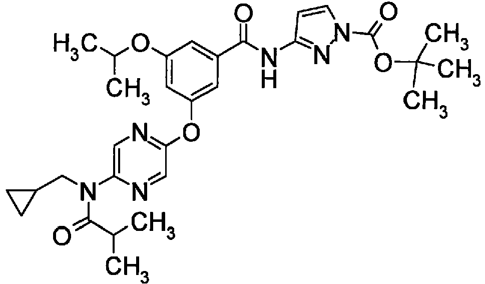 |
| 58 | 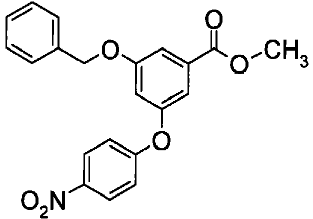 |

**[Table 17]**

| PEx | Structure |
|---|---|
| 59 | 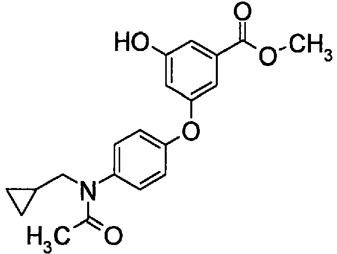 |
| 60 | 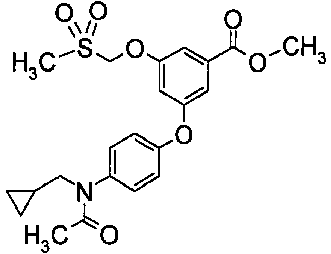 |
| 61 | 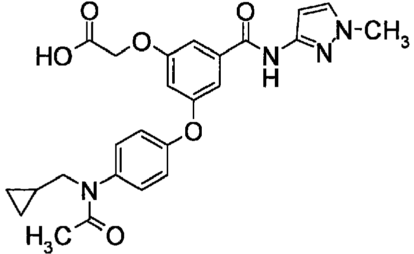 |
| 62 | 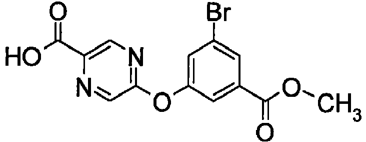 |
| 63 | 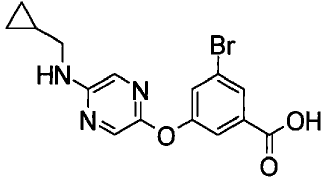 |

**[Table 18]**

| PEx | Structure |
|---|---|
| 64 | 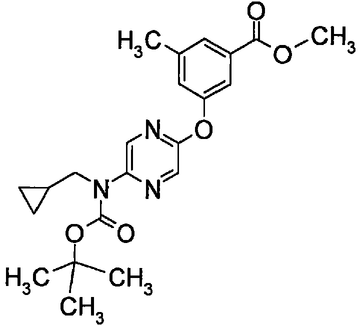 |
| 65 | 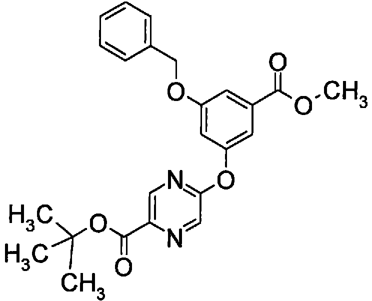 |
| 66 | 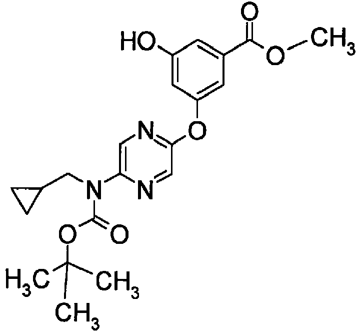 |

**[Table 19]**

| PEx | Structure |
|---|---|
| 67 | 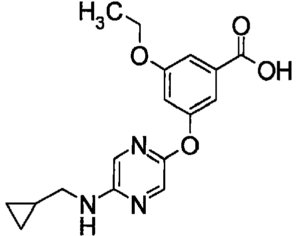 |
| 68 | 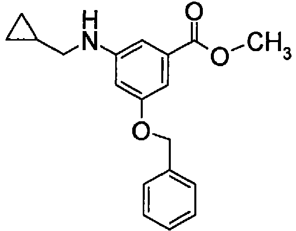 |
| 69 | 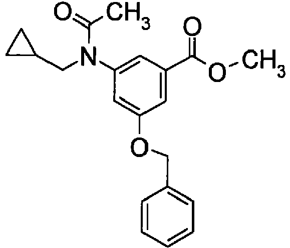 |
| 70 | 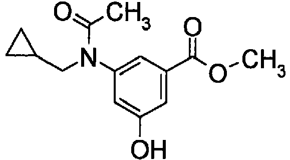 |

**[Table 20]**

| PEx | Structure |
|---|---|
| 71 | 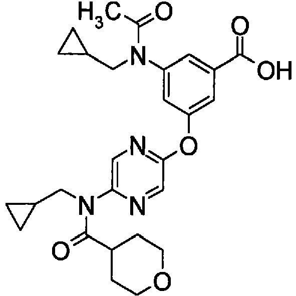 |
| 72 | 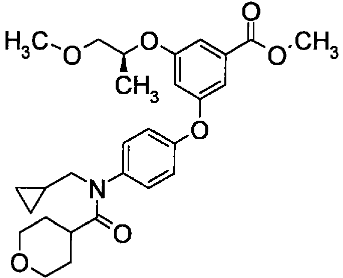 |
| 73 | 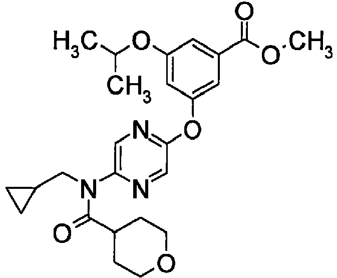 |
| 74 | 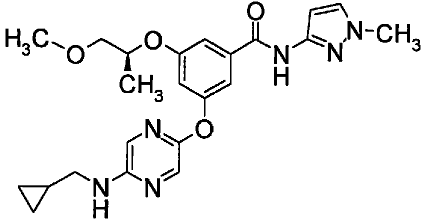 |

**[Table 21]**

| PEx | Structure |
|---|---|
| 75 | 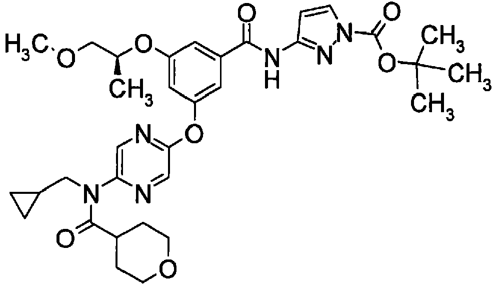 |
| 76 | 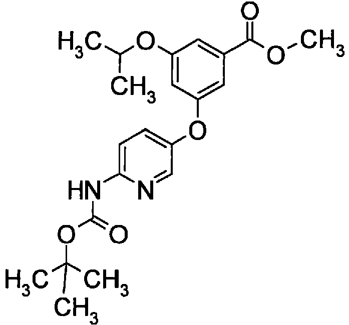 |
| 77 | 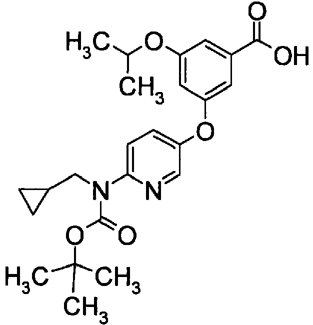 |

**[Table 22]**

| PEx | Structure |
|---|---|
| 78 | 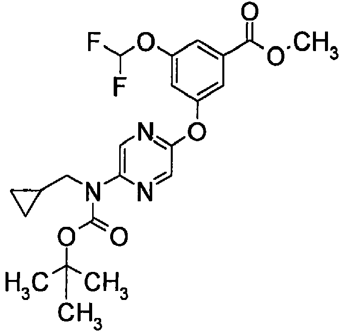 |
| 79 | 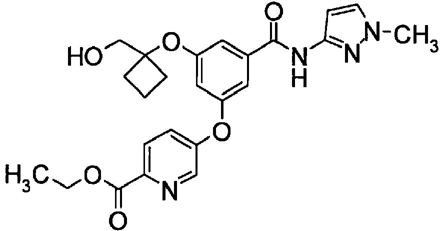 |
| 80 | 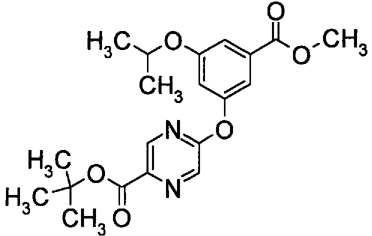 |
| 81 | 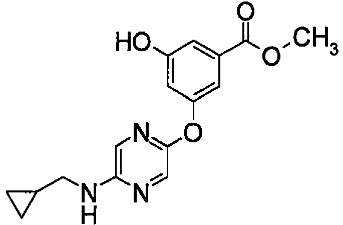 |

**[Table 23]**

| PEx | Structure |
|---|---|
| 82 | 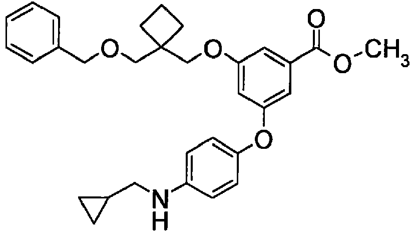 |
| 83 | 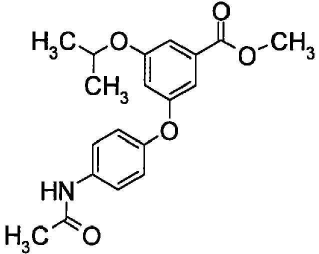 |
| 84 | 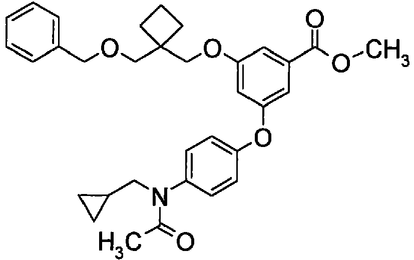 |
| 85 | 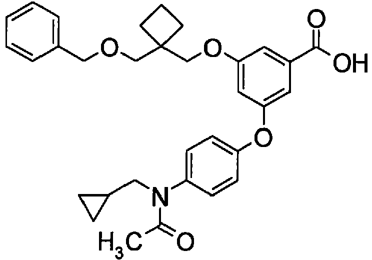 |

**[Table 24]**

| PEx | Structure |
|---|---|
| 86 | 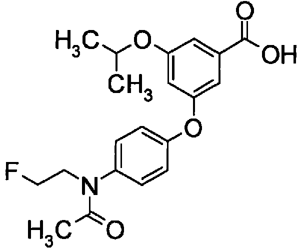 |
| 87 | 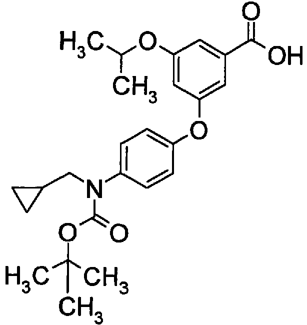 |
| 88 | 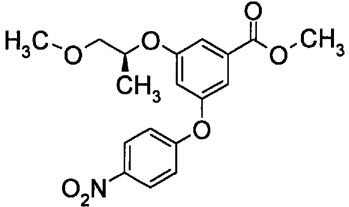 |
| 89 | 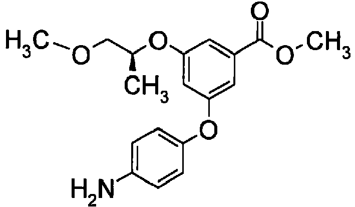 |

**[Table 25]**

| PEx | Structure |
|---|---|
| 90 | 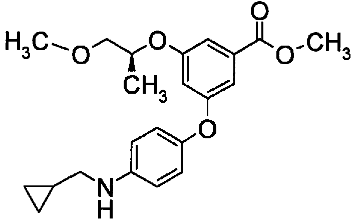 |
| 91 | 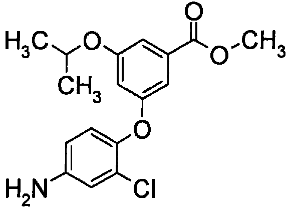 |
| 92 | 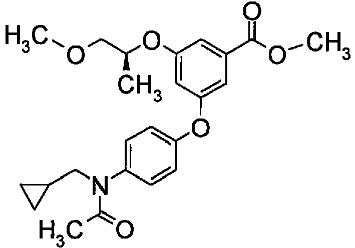 |
| 93 | 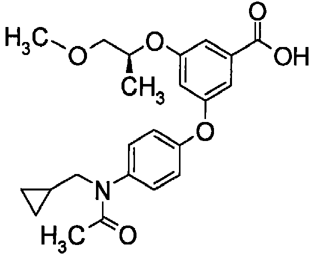 |

**[Table 26]**

| PEx | Structure |
|---|---|
| 94 | 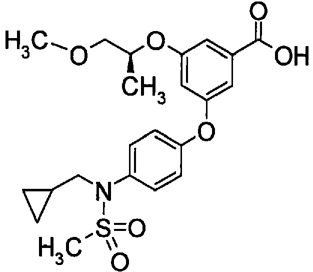 |
| 95 | 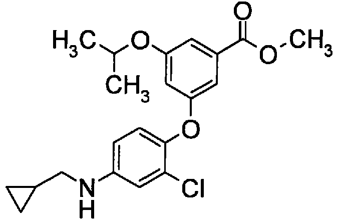 |
| 96 | 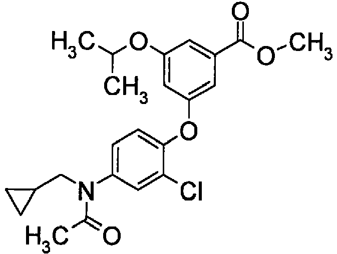 |
| 97 | 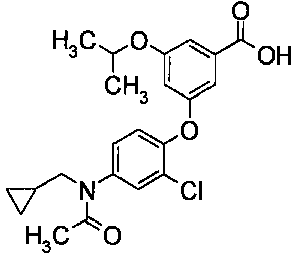 |

**[Table 27]**

| PEx | Structure |
|---|---|
| 98 | 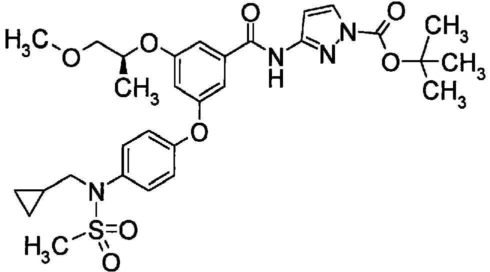 |
| 99 | 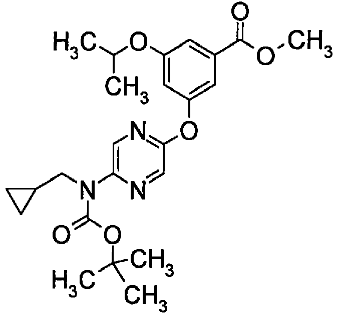 |
| 100 | 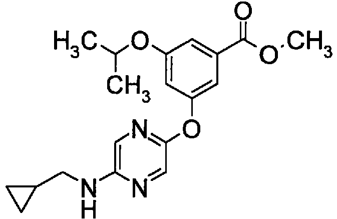 |
| 101 | 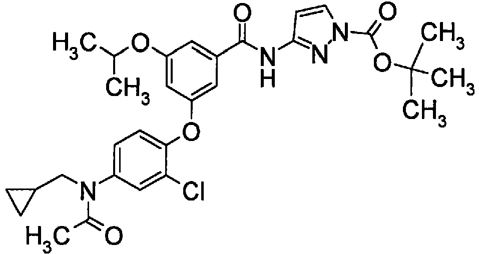 |

**[Table 28]**

| PEx | Structure |
|---|---|
| 102 | 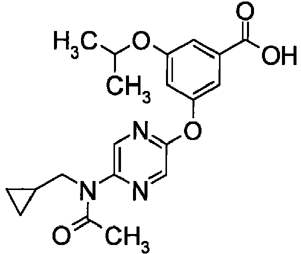 |
| 103 | 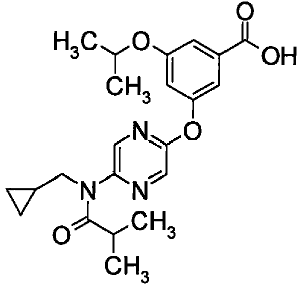 |
| 104 | 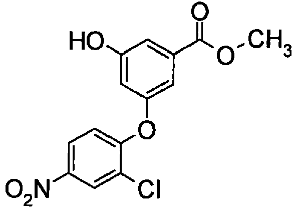 |
| 105 | 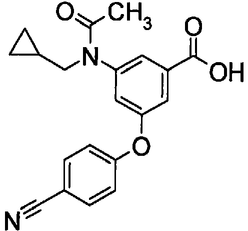 |

**[Table 29]**

| PEx | Structure |
|---|---|
| 106 | 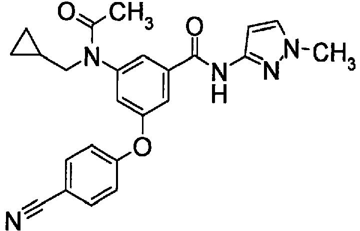 |
| 107 | 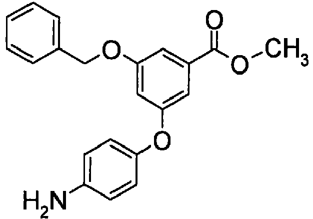 |
| 108 | 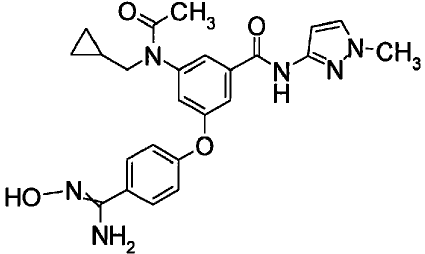 |
| 109 | 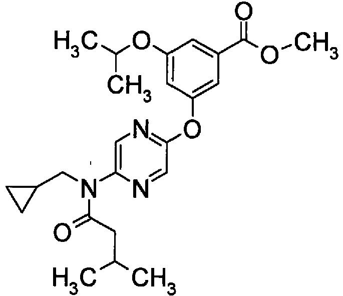 |

**[Table .30]**

| PEx | Structure |
|---|---|
| 110 | 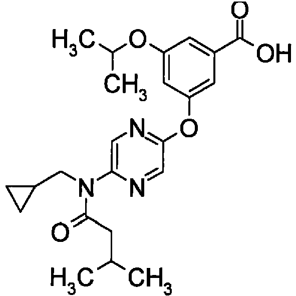 |
| 111 | 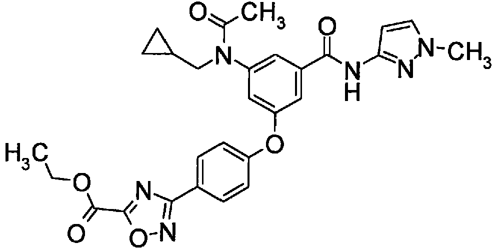 |
| 112 | 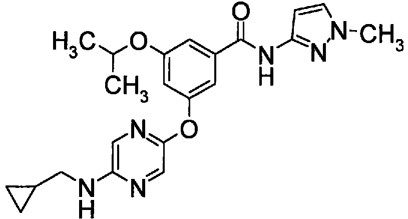 |
| 113 | 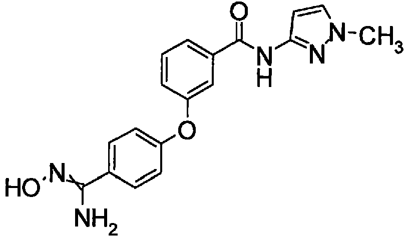 |

**[Table 31]**

| PEx | Structure |
|---|---|
| 114 | 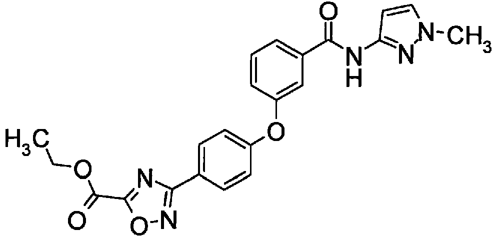 |
| 115 | 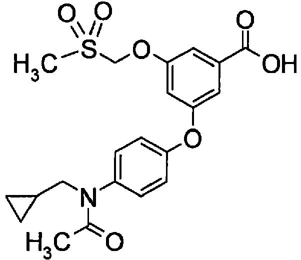 |
| 116 | 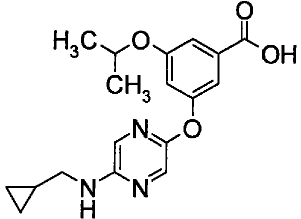 |
| 117 | 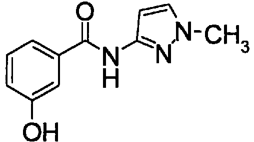 |
| 118 | 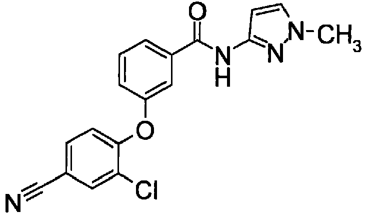 |

**[Table 32]**

| PEx | Structure |
|---|---|
| 119 | 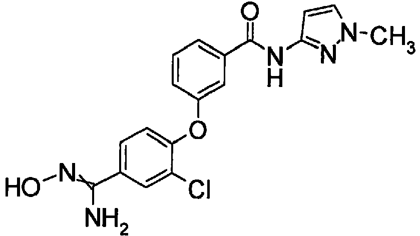 |
| 120 | 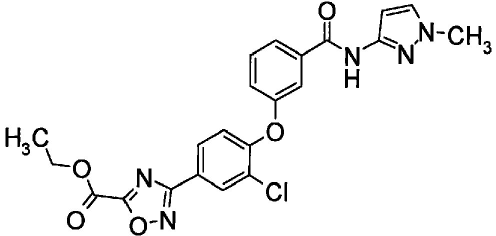 |
| 121 | 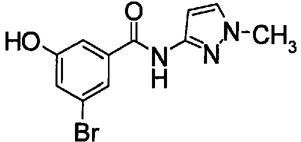 |
| 122 | 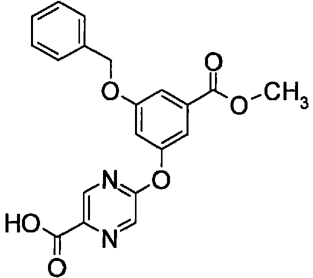 |

**[Table 33]**

| PEx | Structure |
|---|---|
| 123 | 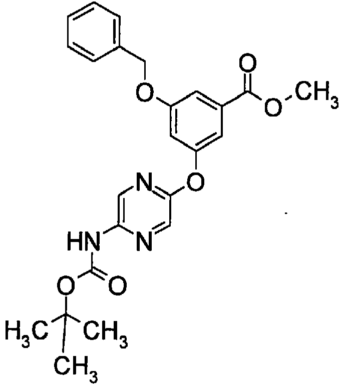 |
| 124 | 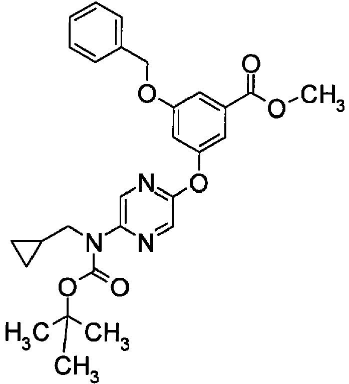 |
| 125 | 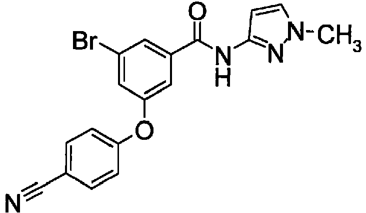 |

**[Table 34]**

| PEx | Structure |
|---|---|
| 126 | 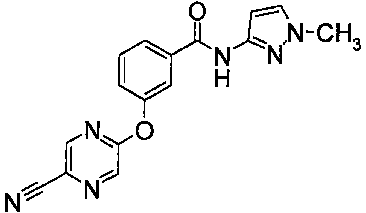 |
| 127 | 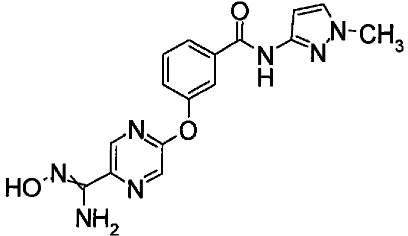 |
| 128 | 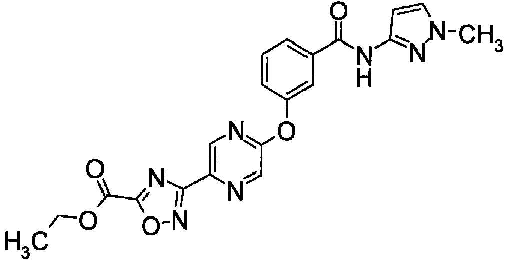 |
| 129 | 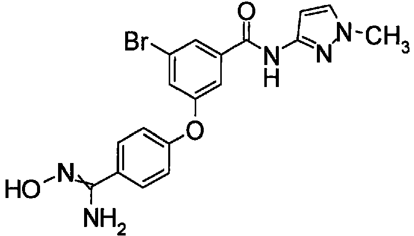 |

**[Table 35]**

| PEx | Structure |
|---|---|
| 130 | 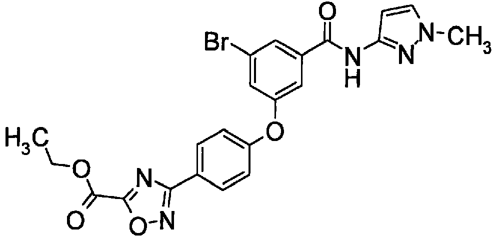 |
| 131 | 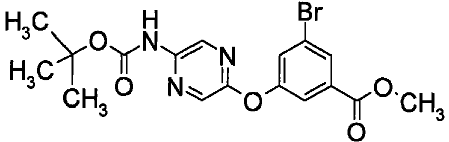 |
| 132 | 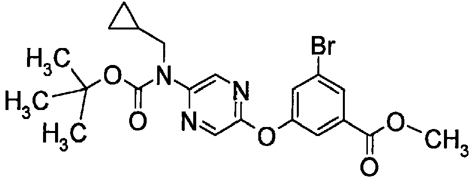 |
| 133 | 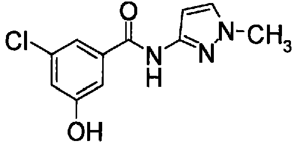 |
| 134 | 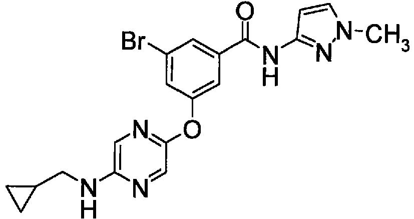 |

**[Table 36]**

| PEx | Structure |
|---|---|
| 135 | 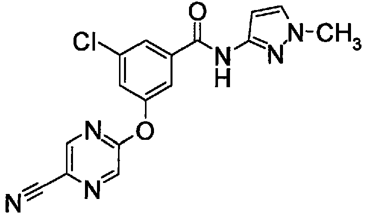 |
| 136 | 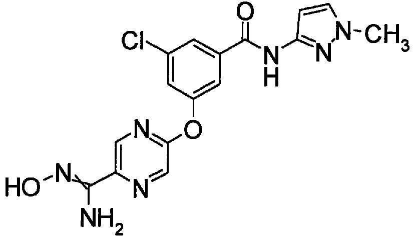 |
| 137 | 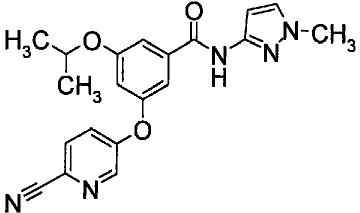 |
| 138 | 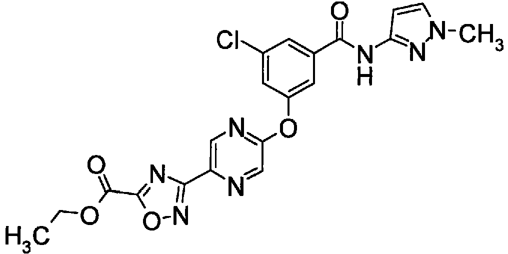 |

**[Table 37]**

| PEx | Structure |
|---|---|
| 139 | 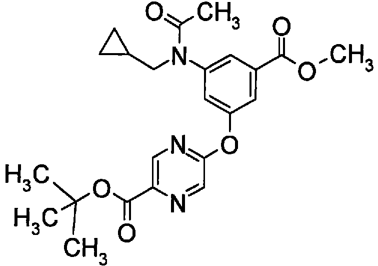 |
| 140 | 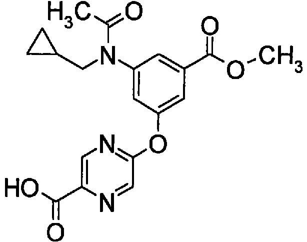 |
| 141 | 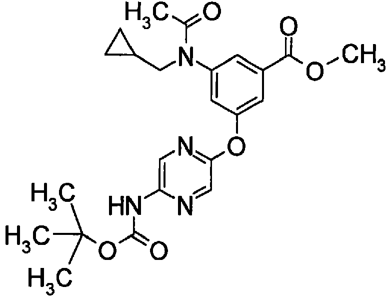 |
| 142 | 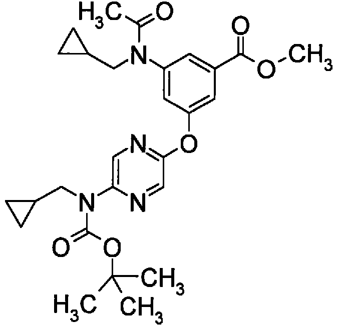 |

**[Table 38]**

| PEx | Structure |
|---|---|
| 143 | 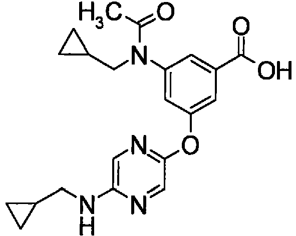 |
| 144 | 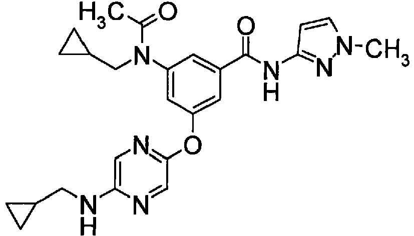 |
| 145 | 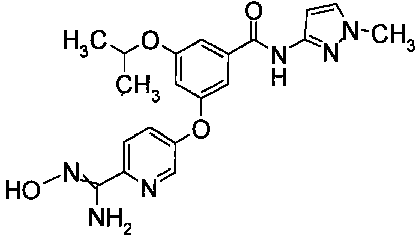 |
| 146 | 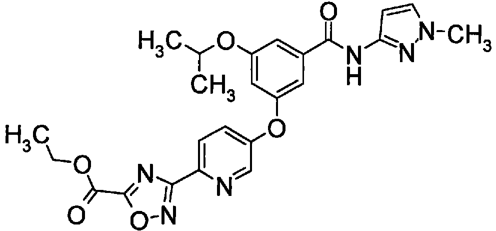 |

**[Table 39]**

| PEx | Structure |
|---|---|
| 147 | 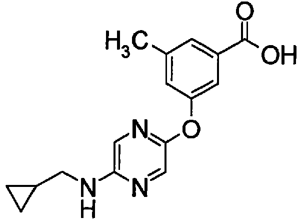 |
| 148 | 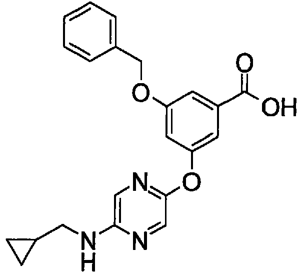 |
| 149 | 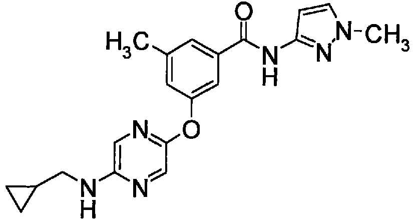 |
| 150 | 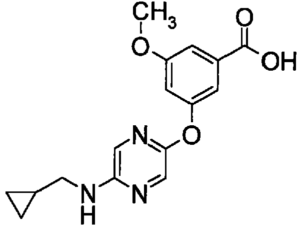 |

**[Table 40]**

| PEx | Structure |
|---|---|
| 151 | 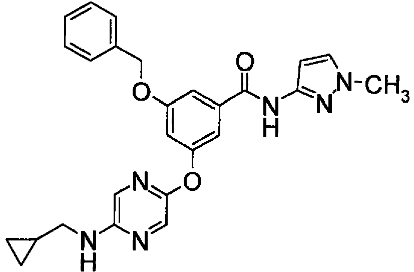 |
| 152 | 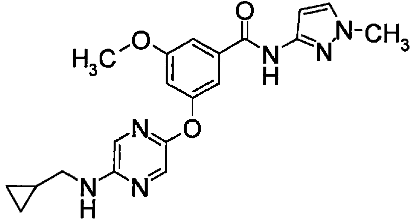 |
| 153 | 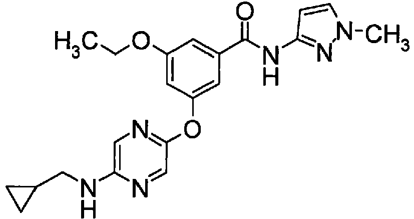 |
| 154 | 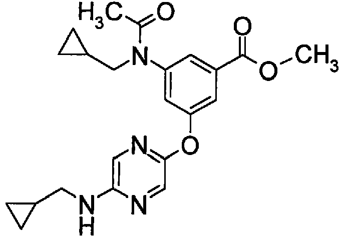 |

**[Table 41]**

| PEx | Structure |
|---|---|
| 155 | 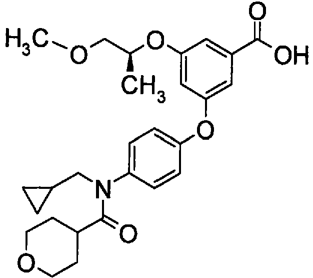 |
| 156 | 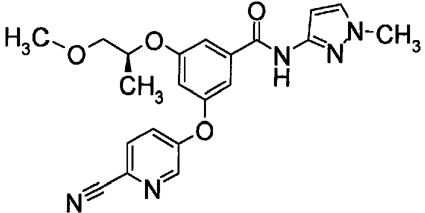 |
| 157 | 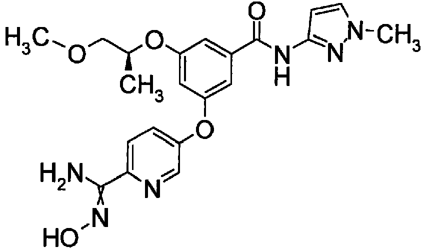 |
| 158 | 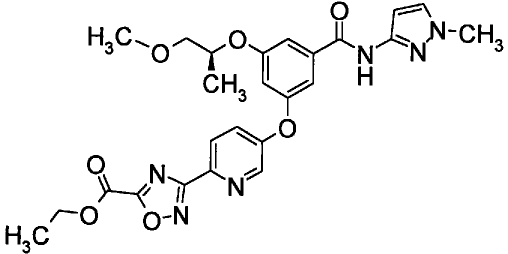 |

**[Table 42]**

| PEx | I Structure |
|---|---|
| 159 | 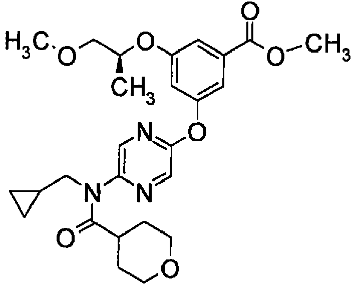 |
| 160 | 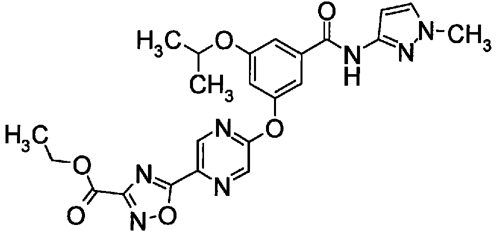 |
| 161 | 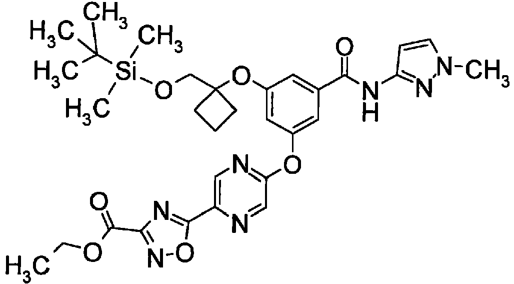 |
| 162 | 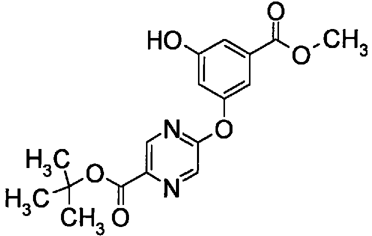 |

**[Table 43]**

| PEx | Structure |
|---|---|
| 163 | 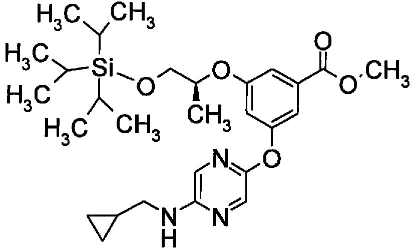 |
| 164 | 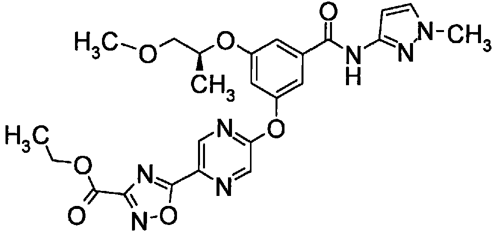 |
| 165 | 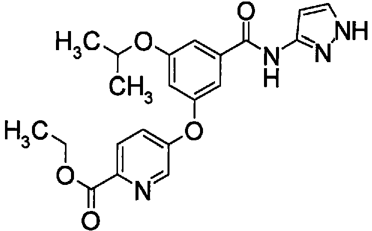 |
| 166 | 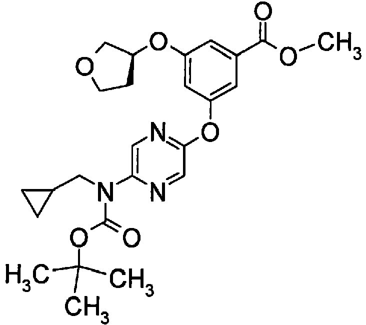 |

**[Table 44]**

| PEx | Structure |
|---|---|
| 167 | 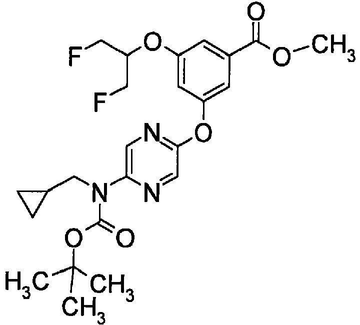 |
| 168 | 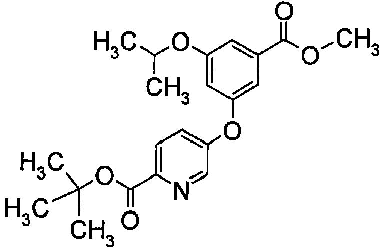 |
| 169 | 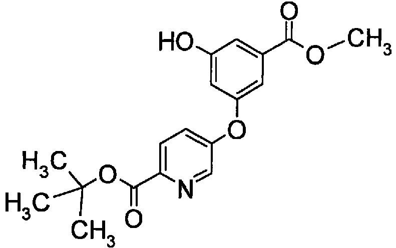 |
| 170 | 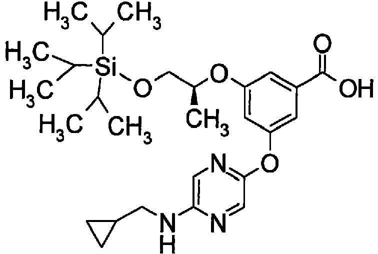 |

**[Table 45]**

| PEx | Structure |
|---|---|
| 171 | 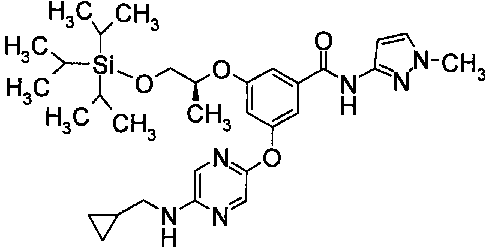 |
| 172 | 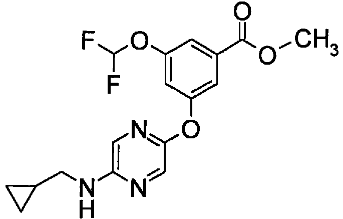 |
| 173 | 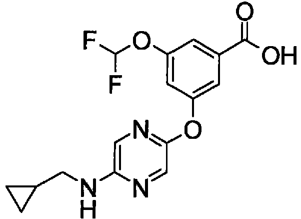 |
| 174 | 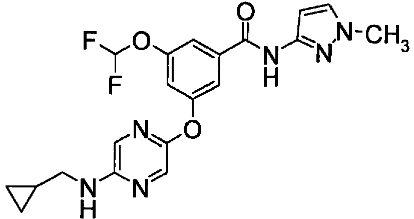 |

**[Table 46]**

| PEx | Structure |
|---|---|
| 175 | 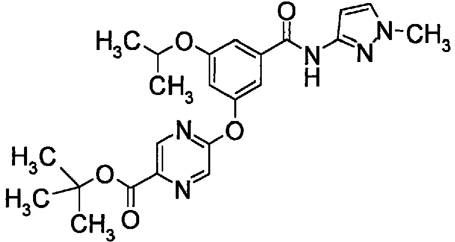 |
| 176 | 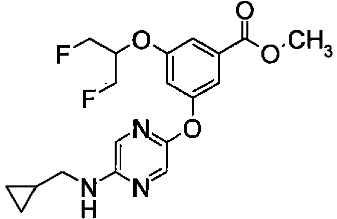 |
| 177 | 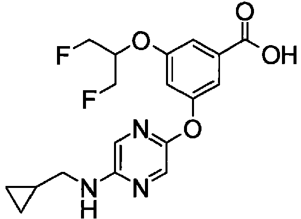 |
| 178 | 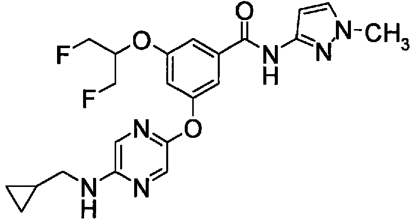 |

**[Table 47]**

| PEx | Structure |
|---|---|
| 179 | 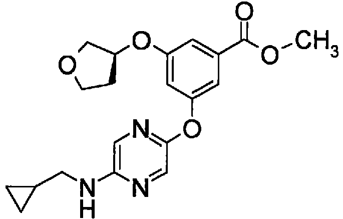 |
| 180 | 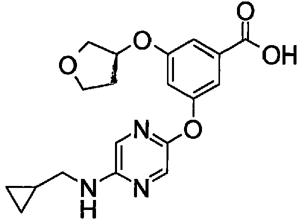 |
| 181 | 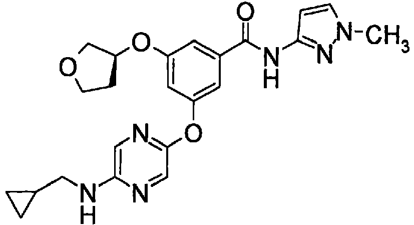 |
| 182 | 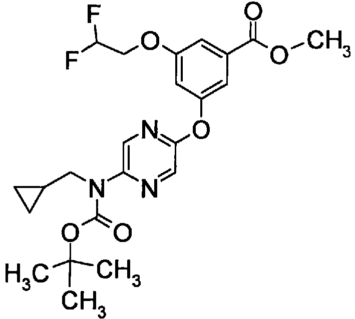 |

**[Table 48]**

| PEx | Structure |
|---|---|
| 183 | 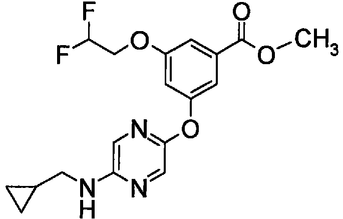 |
| 184 | 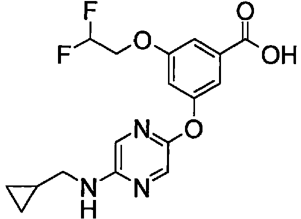 |
| 185 | 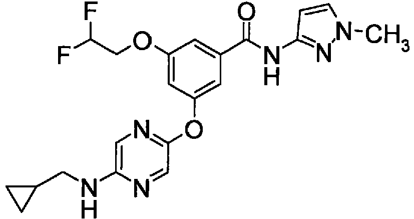 |
| 186 | 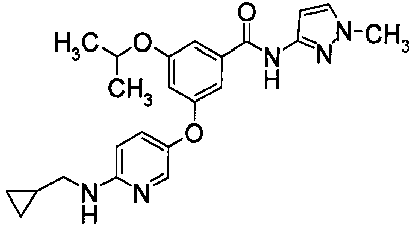 |

**[Table 49]**

| PEx | Structure |
|---|---|
| 187 | 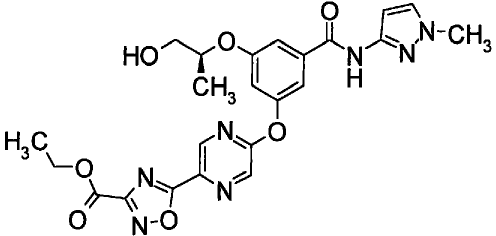 |
| 188 | 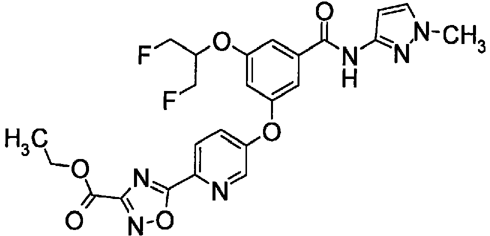 |
| 189 | 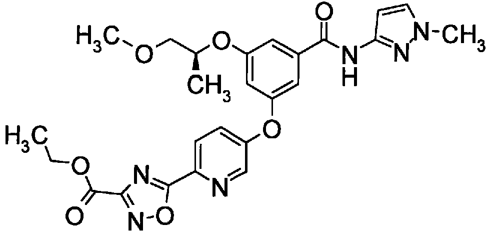 |
| 190 | 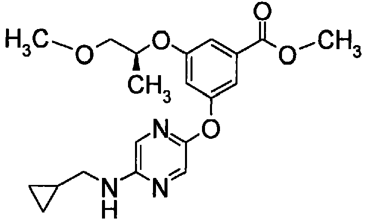 |

**[Table 50]**

| PEx | Structure |
|---|---|
| 191 | 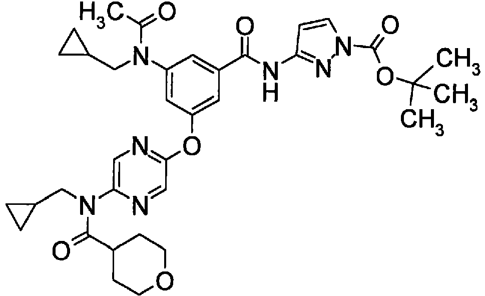 |
| 192 | 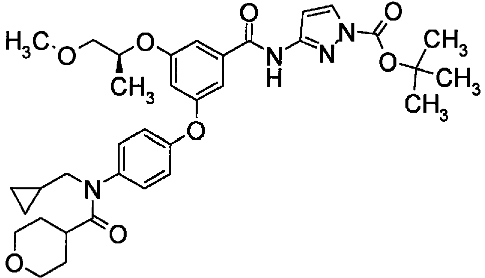 |
| 193 | 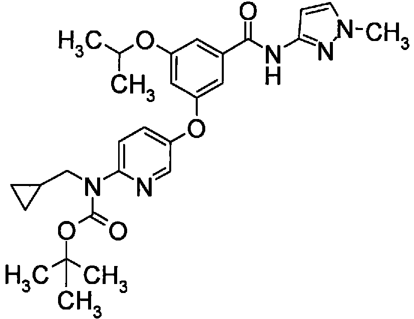 |

**[Table 51]**

| Ex | Structure |
|---|---|
| 1 | 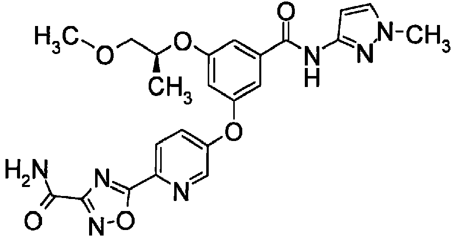 |
| 2 | 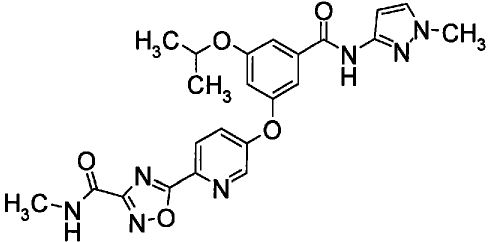 |
| 3 | 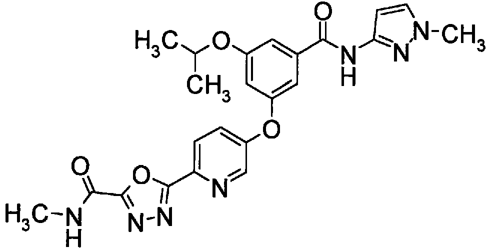 |
| 4 | 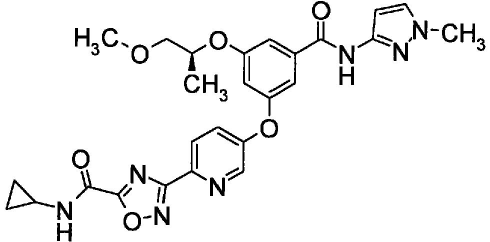 |

**[Table 52]**

| Ex | Structure |
|---|---|
| 5 | 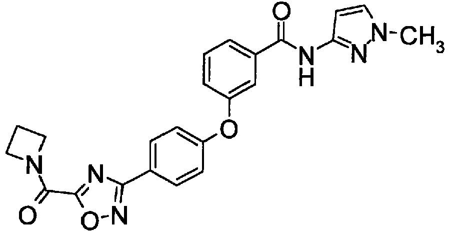 |
| 6 | 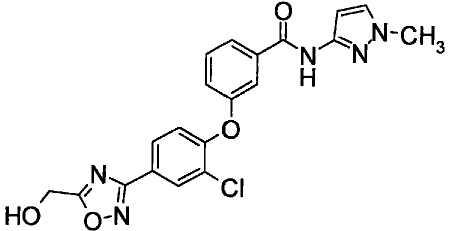 |
| 7 | 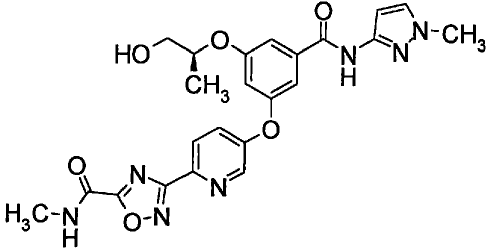 |
| 8 | 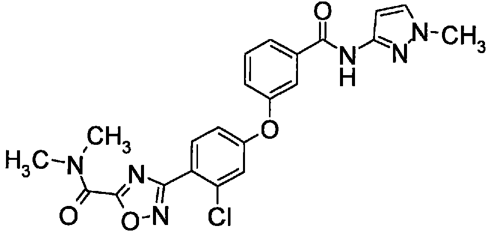 |

**[Table 53]**

| Ex | Structure |
|---|---|
| 9 | 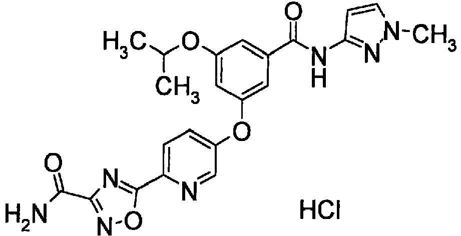 |
| 10 | 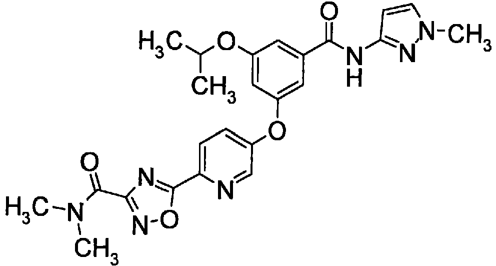 |
| 11 | 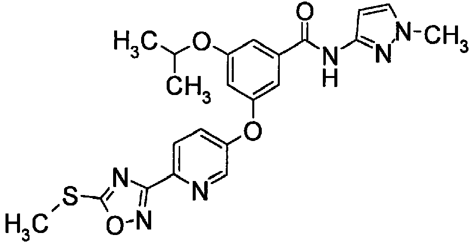 |
| 12 | 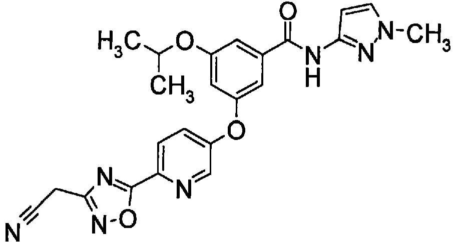 |

**[Table 54]**

| Ex | Structure |
|---|---|
| 13 | 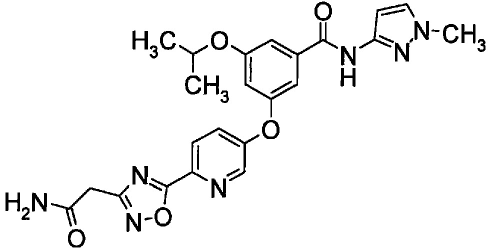 |
| 14 | 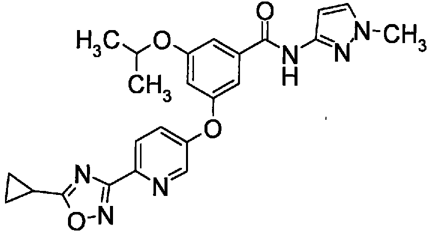 |
| 15 | 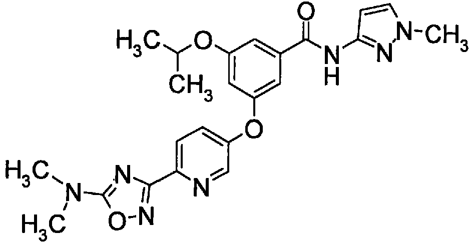 |
| 16 | 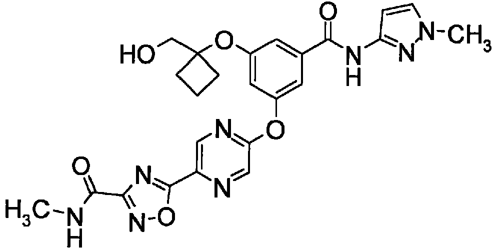 |

**[Table 55]**

| Ex | Structure |
|---|---|
| 17 | 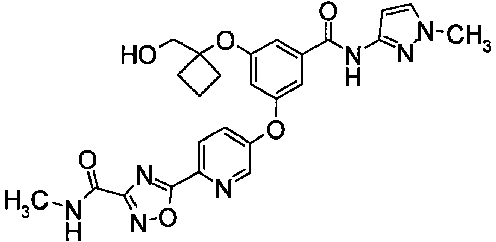 |
| 18 | 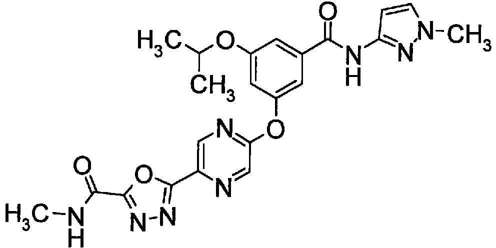 |
| 19 | 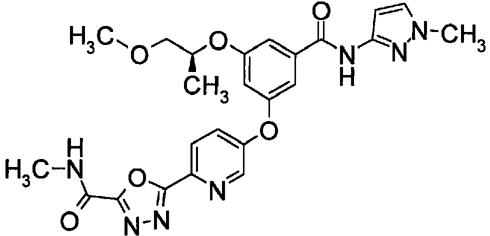 |
| 20 | 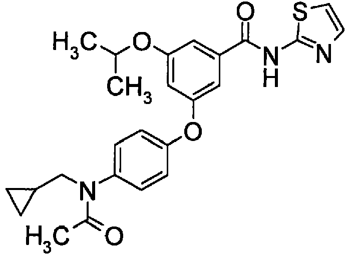 |

**[Table 56]**

| Ex | Structure |
|---|---|
| 21 | 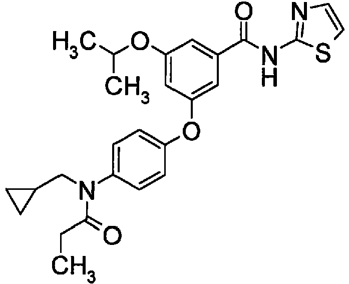 |
| 22 | 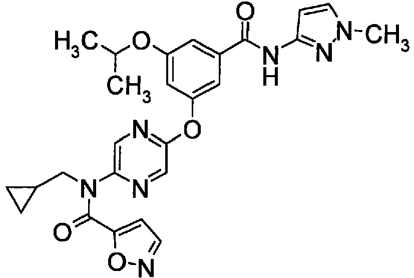 |
| 23 | 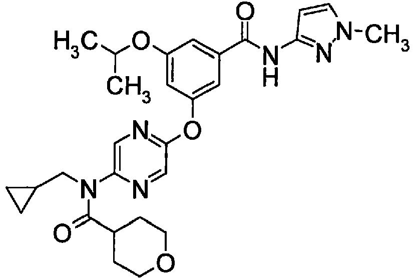 |

**[Table 57]**

| Ex | Structure |
|---|---|
| 24 | 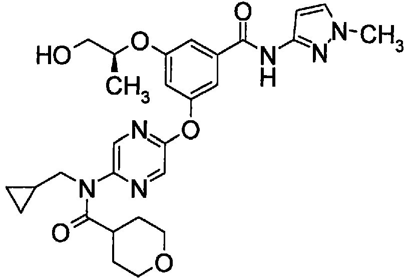 |
| 25 | 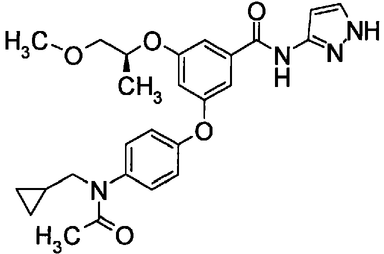 |
| 26 | 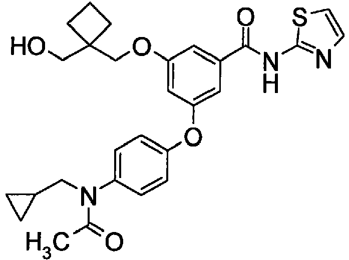 |
| 27 | 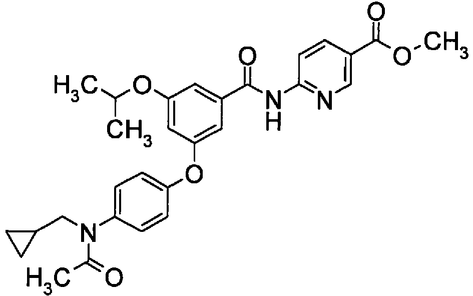 |

**[Table 58]**

| Ex | Structure |
|---|---|
| 28 | 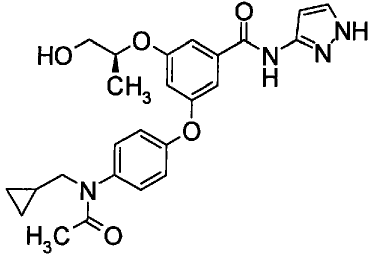 |
| 29 | 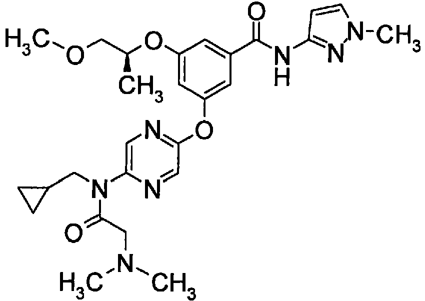 |
| 30 | 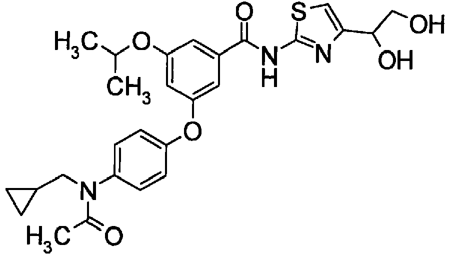 |
| 31 | 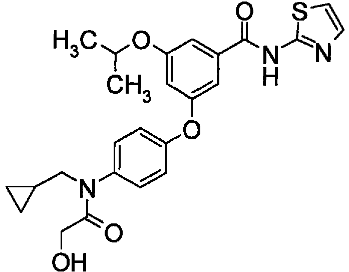 |

**[Table 59]**

| Ex | Structure |
|---|---|
| 32 | 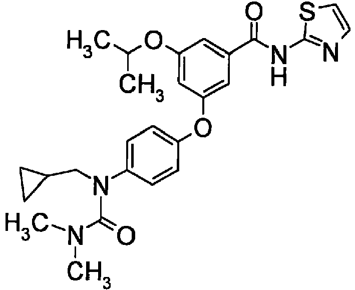 |
| 33 | 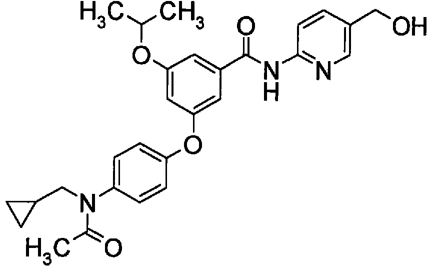 |
| 34 | 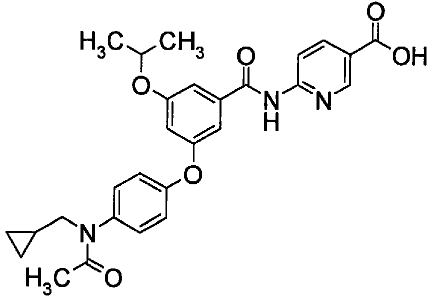 |
| 35 | 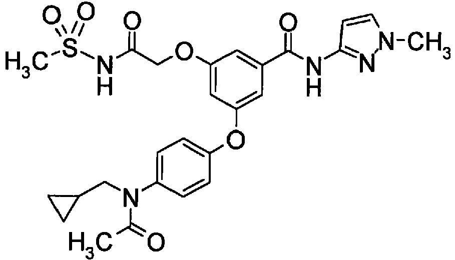 |

**[Table 60]**

| Ex | Structure |
|---|---|
| 36 | 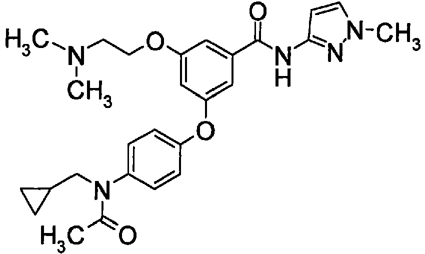 |
| 37 | 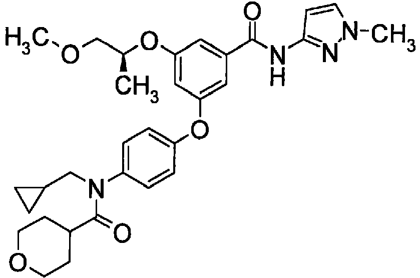 |
| 38 | 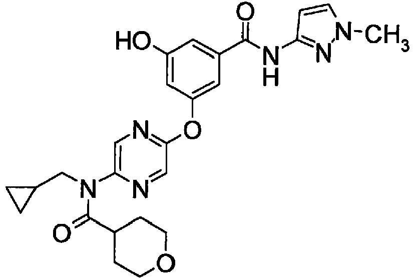 |
| 39 | 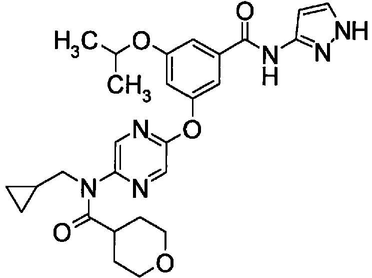 |

**[Table 61]**

| Ex | Structure |
|---|---|
| 40 | 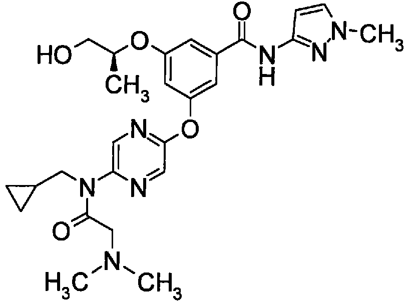 |
| 41 | 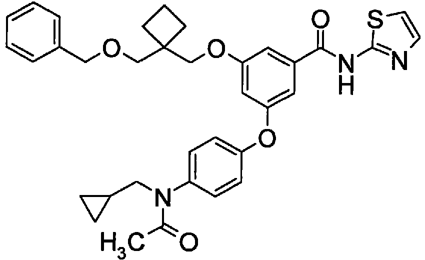 |
| 42 | 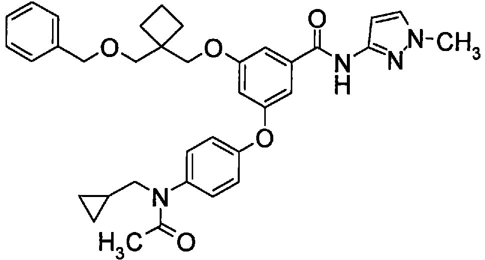 |
| 43 | 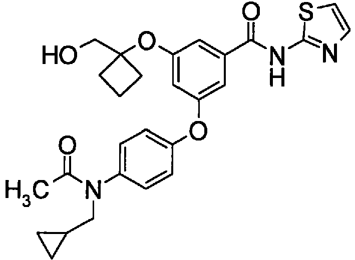 |

**[Table 62]**

| Ex | Structure |
|---|---|
| 44 | 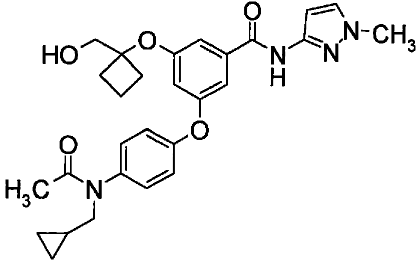 |
| 45 | 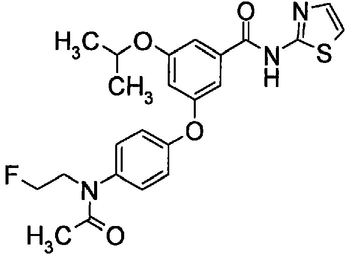 |
| 46 | 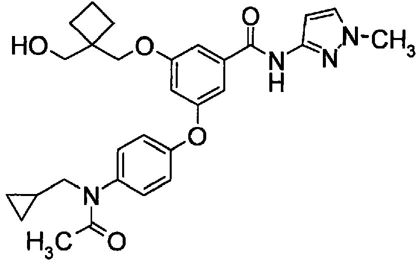 |
| 47 | 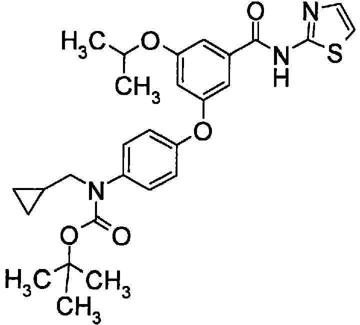 |

**[Table 63]**

| Ex | Structure |
|---|---|
| 48 | 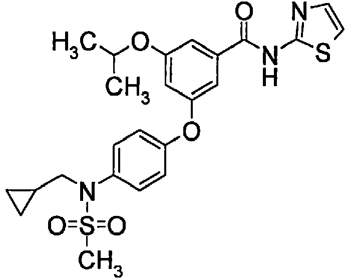 |
| 49 | 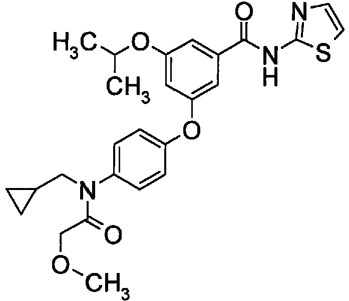 |
| 50 | 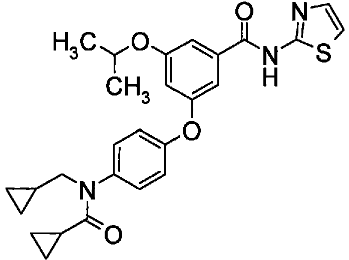 |
| 51 | 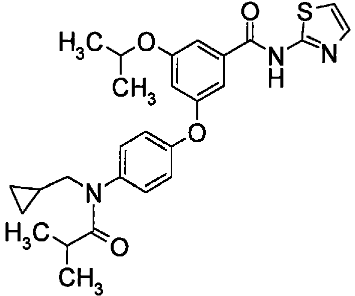 |

**[Table 64]**

| Ex | Structure |
|---|---|
| 52 | 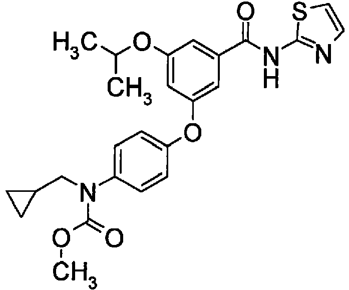 |
| 53 | 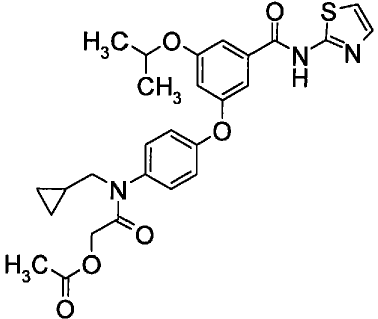 |
| 54 | 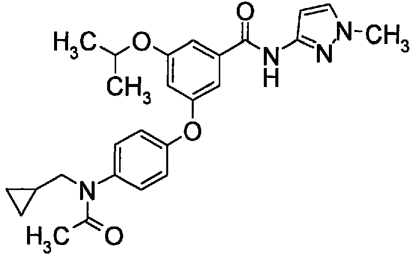 |
| 55 | 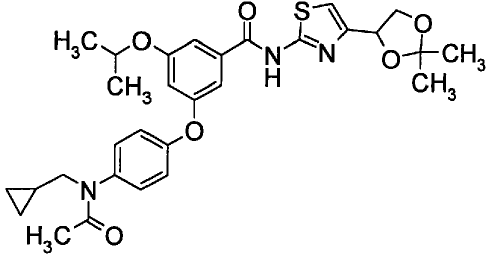 |

**[Table 65]**

| Ex | Structure |
|---|---|
| 56 | 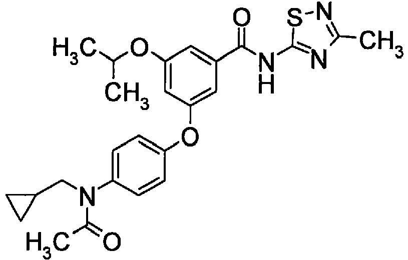 |
| 57 | 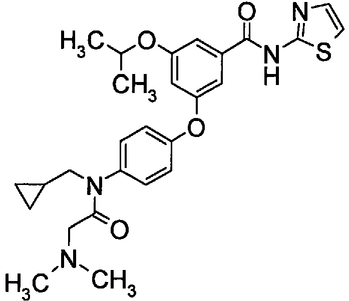 |
| 58 | 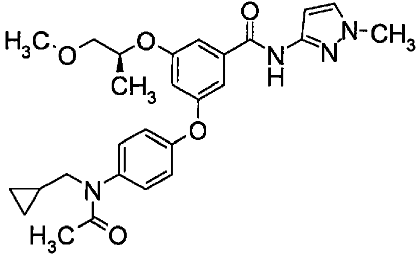 |
| 59 | 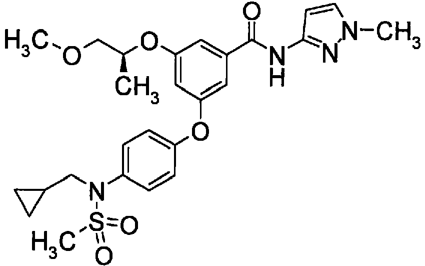 |

**[Table 66]**

| Ex | Structure I |
|---|---|
| 60 | 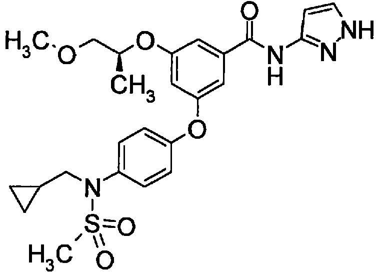 |
| 61 | 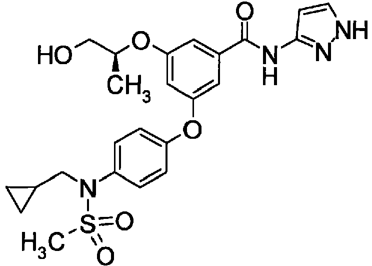 |
| 62 | 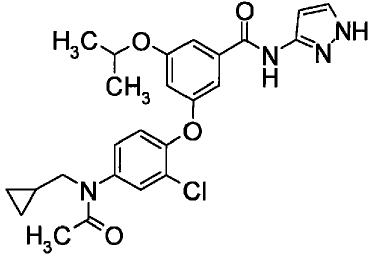 |
| 63 | 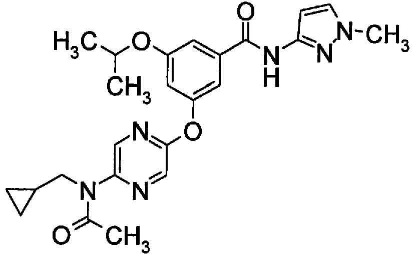 |

**[Table 67]**

| Ex | Structure |
|---|---|
| 64 | 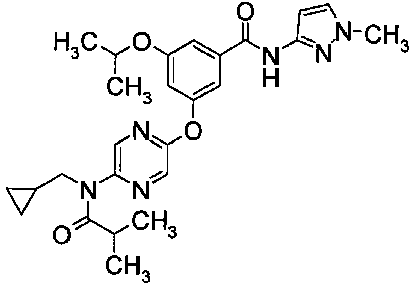 |
| 65 | 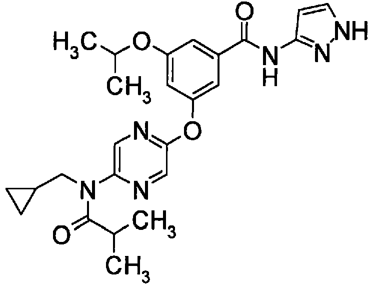 |
| 66 | 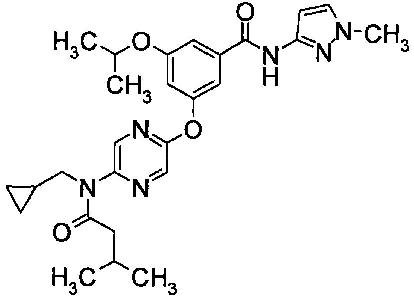 |
| 67 | 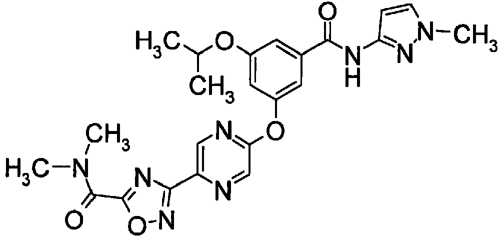 |

**[Table 68]**

| Ex | Structure |
|---|---|
| 68 | 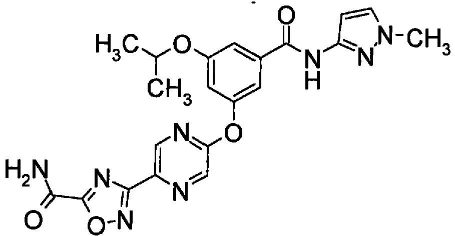 |
| 69 | 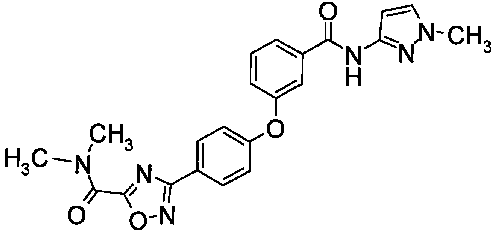 |
| 70 | 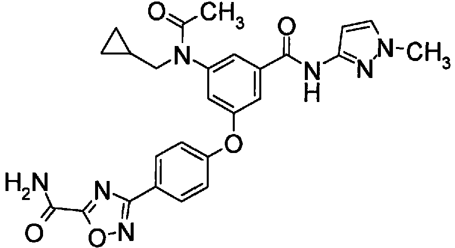 |
| 71 | 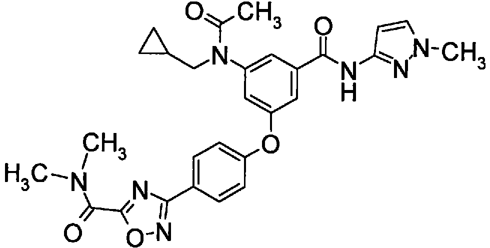 |

**[Table 69]**

| Ex | Structure |
|---|---|
| 72 | 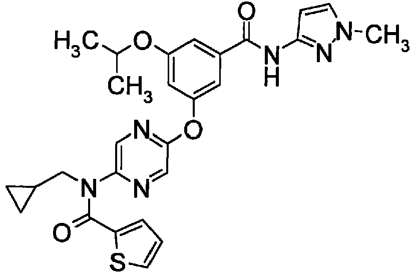 |
| 73 | 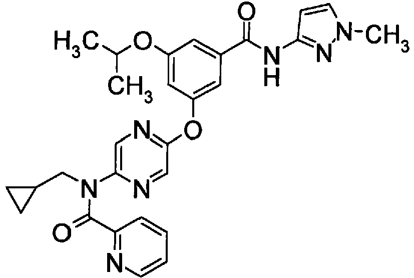 |
| 74 | 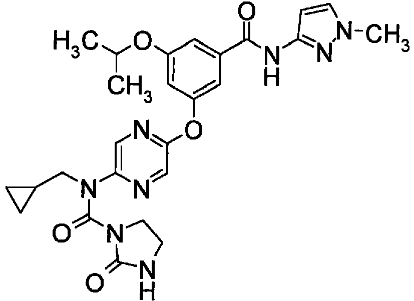 |
| 75 | 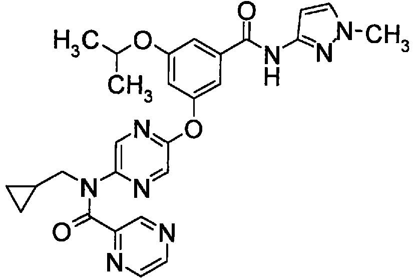 |

**[Table 70]**

| Ex | Structure |
|---|---|
| 76 | 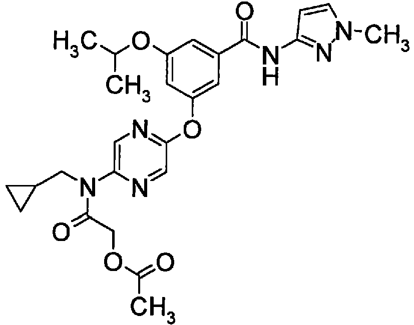 |
| 77 | 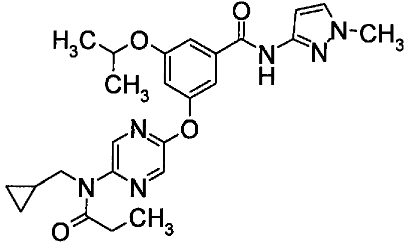 |
| 78 | 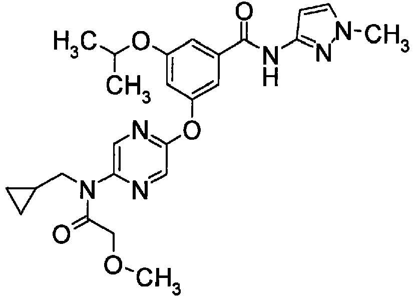 |
| 79 | 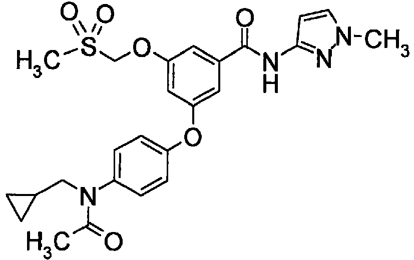 |

**[Table 71]**

| Ex | Structure |
|---|---|
| 80 | 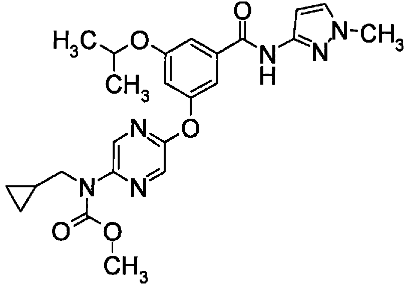 |
| 81 | 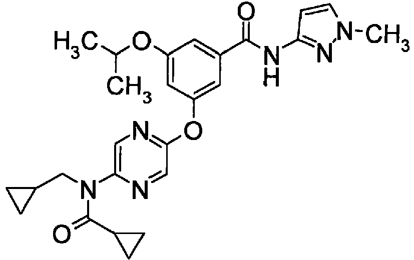 |
| 82 | 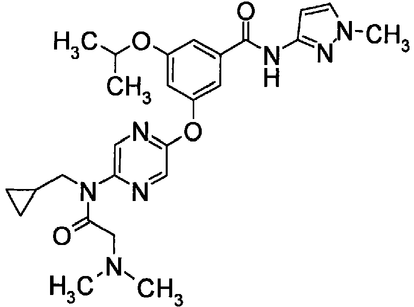 |
| 83 | 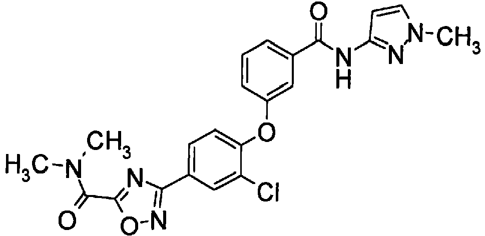 |

**[Table 72]**

| Ex | Structure |
|---|---|
| 84 | 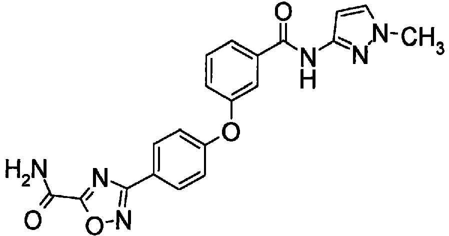 |
| 85 | 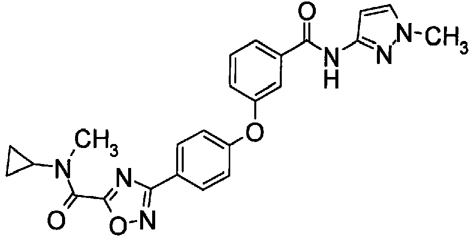 |
| 86 | 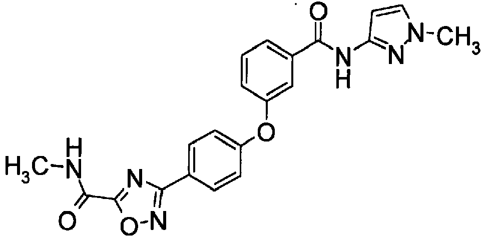 |
| 87 | 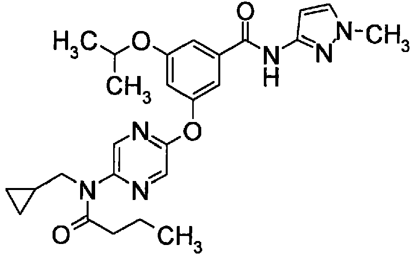 |

**[Table 73]**

| Ex | Structure |
|---|---|
| 88 | 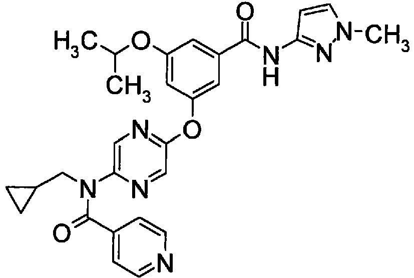 |
| 89 | 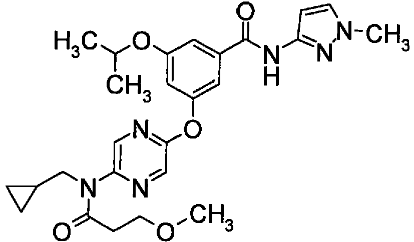 |
| 90 | 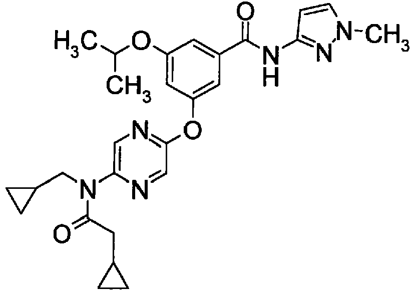 |
| 91 | 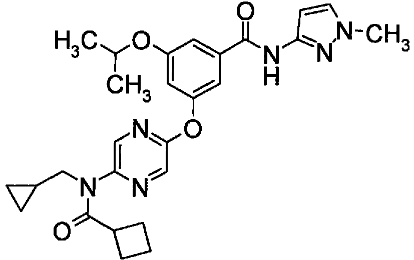 |

**[Table 74]**

| Ex | Structure |
|---|---|
| 92 | 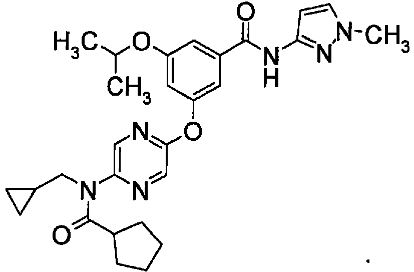 |
| 93 | 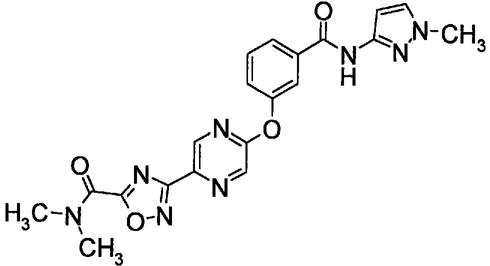 |
| 94 | 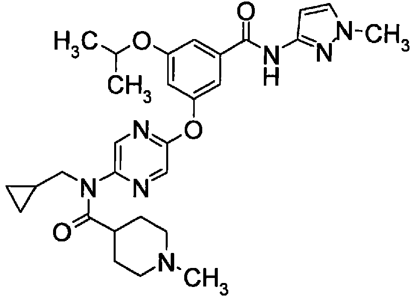 |
| 95 | 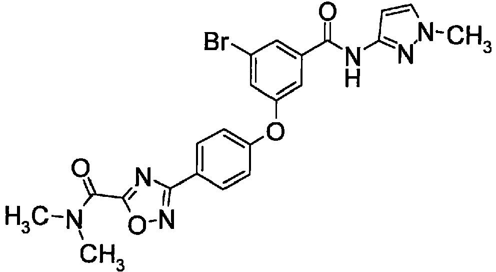 |

**[Table 75]**

| Ex | Structure |
|---|---|
| 96 | 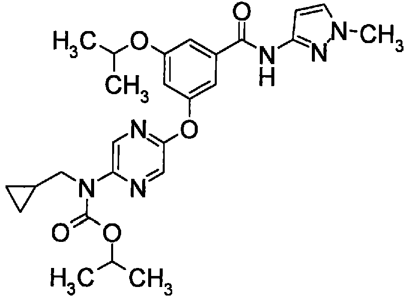 |
| 97 | 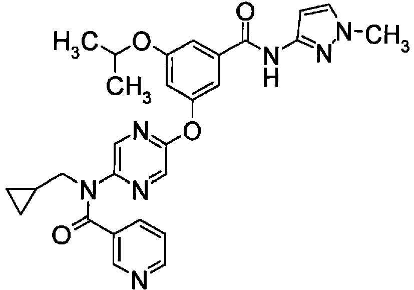 |
| 98 | 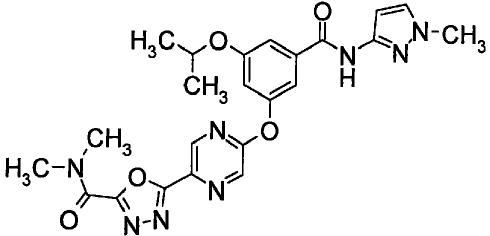 |
| 99 | 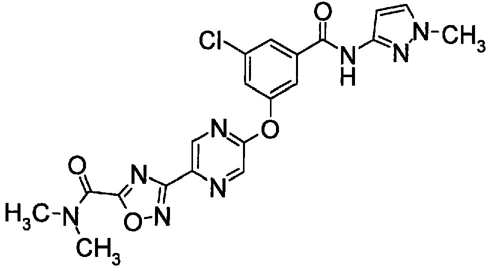 |

**[Table 76]**

| Ex | Structure |
|---|---|
| 100 | 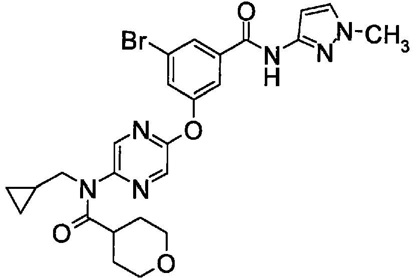 |
| 101 | 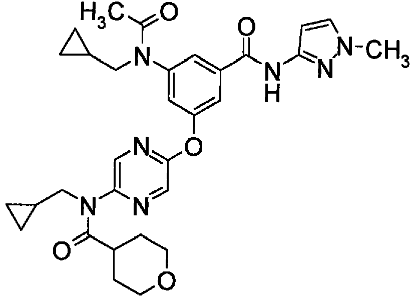 |
| 102 | 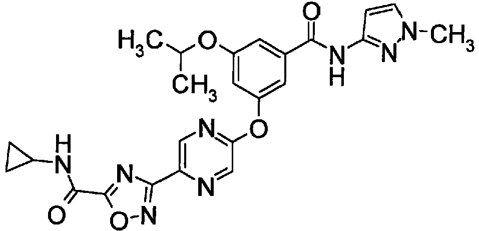 |
| 103 | 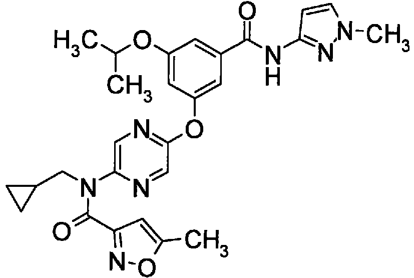 |

**[Table 77]**

| Ex | Structure |
|---|---|
| 104 | 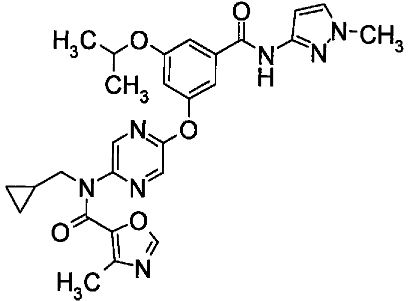 |
| 105 | 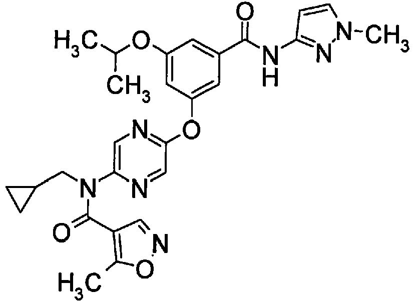 |
| 106 | 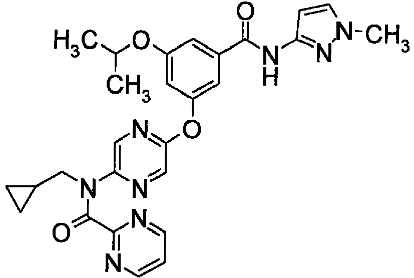 |

**[Table 78]**

| Ex | Structure |
|---|---|
| 107 | 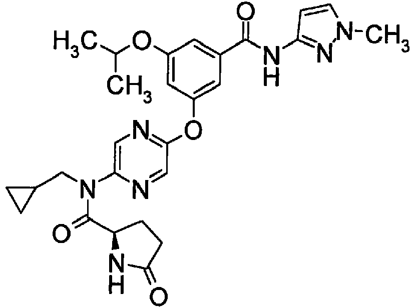 |
| 108 | 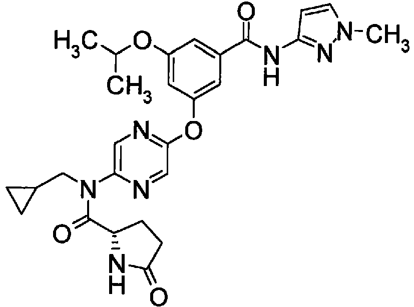 |
| 109 | 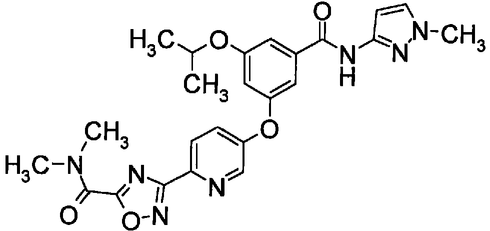 |
| 110 | 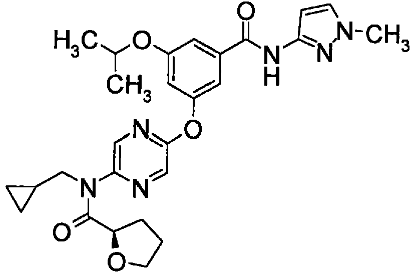 |

**[Table 79]**

| Ex | Structure |
|---|---|
| 111 | 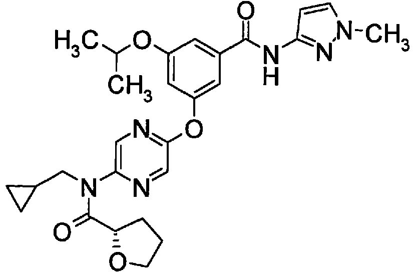 |
| 112 | 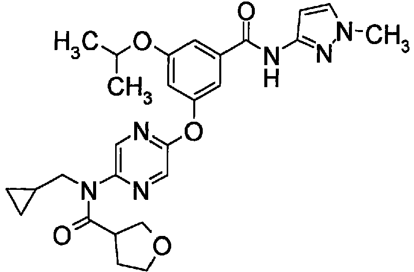 |
| 113 | 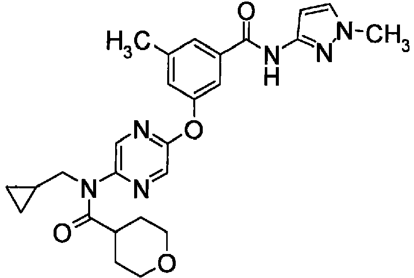 |
| 114 | 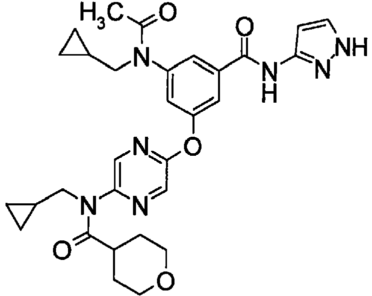 |

**[Table 801**

| Ex | Structure |
|---|---|
| 115 | 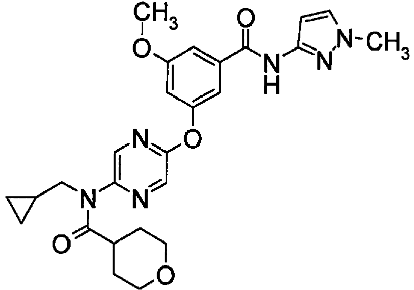 |
| 116 | 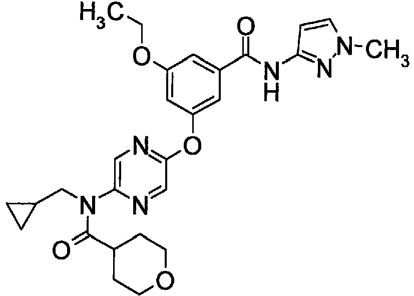 |
| 117 | 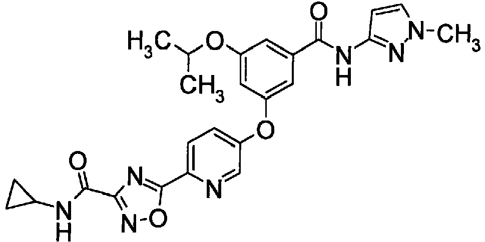 |
| 118 | 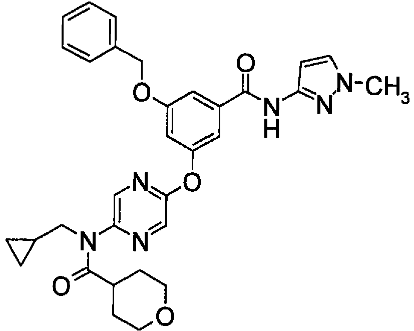 |

**[Table 81]**

| Ex | Structure |
|---|---|
| 119 | 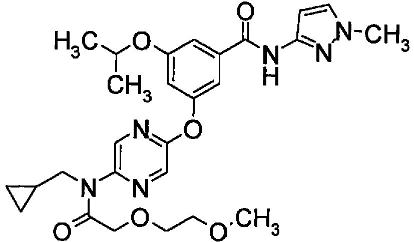 |
| 120 | 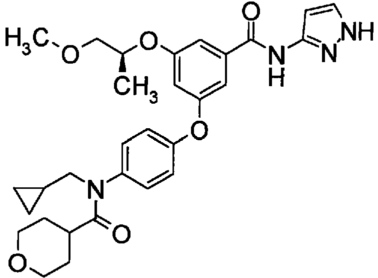 |
| 121 | 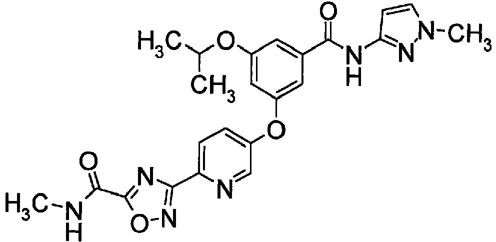 |
| 122 | 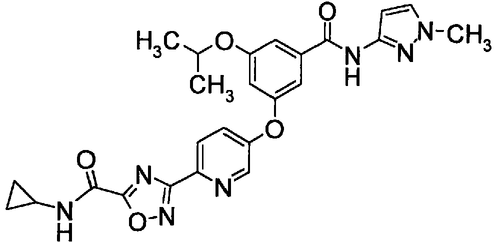 |

**[Table 82]**

| Ex | Structure |
|---|---|
| 123 | 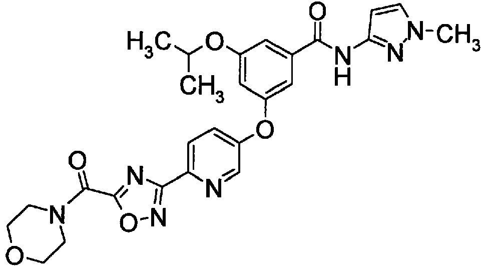 |
| 124 | 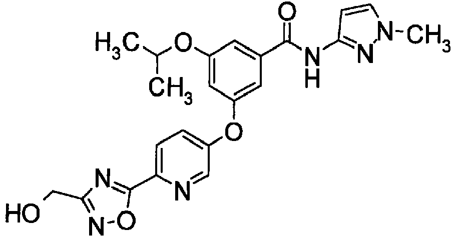 |
| 125 | 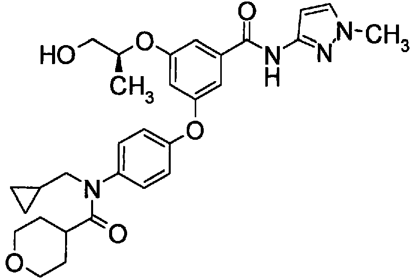 |
| 126 | 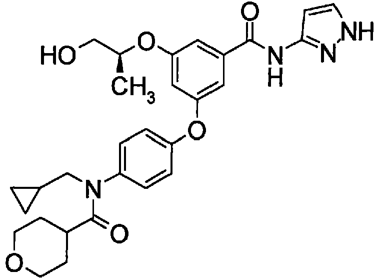 |

**[Table 83]**

| Ex | Structure |
|---|---|
| 127 | 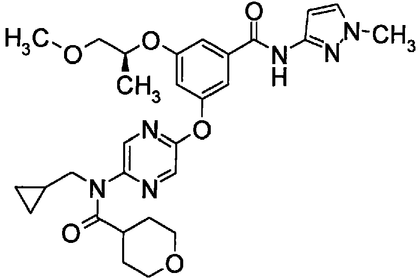 |
| 128 | 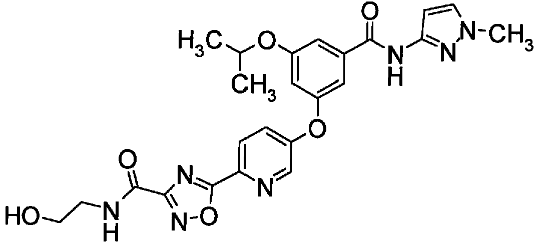 |
| 129 | 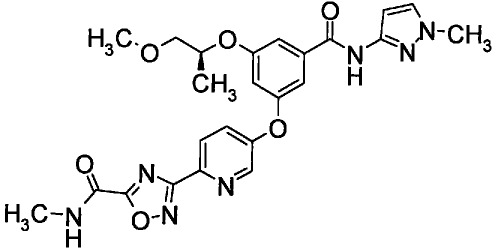 |
| 130 | 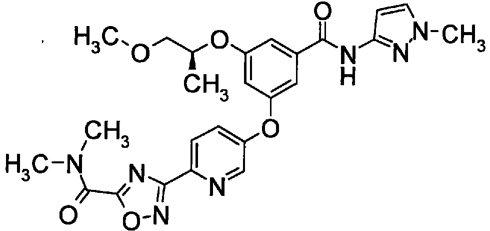 |

**[Table 84]**

| Ex | Structure |
|---|---|
| 131 | 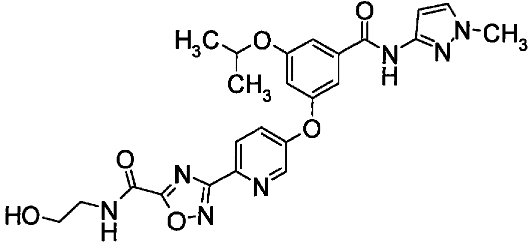 |
| 132 | 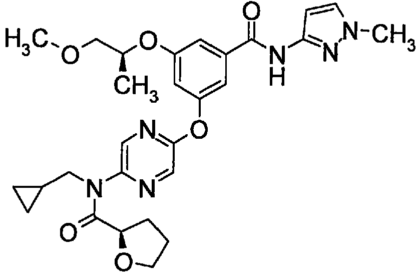 |
| 133 | 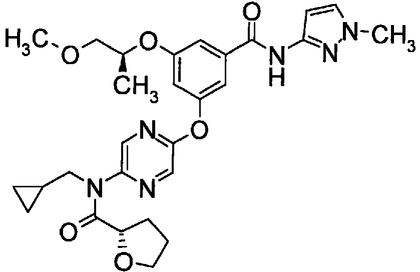 |
| 134 | 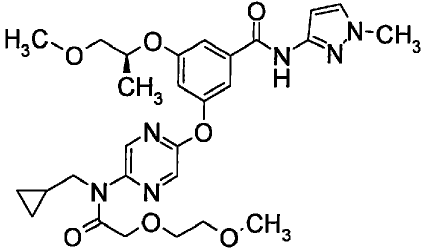 |

**[Table 85]**

| Ex | Structure |
|---|---|
| 135 | 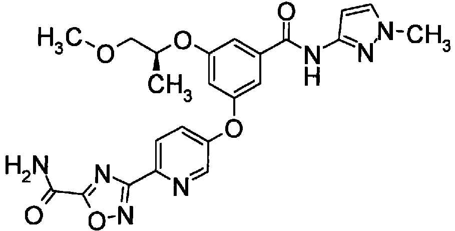 |
| 136 | 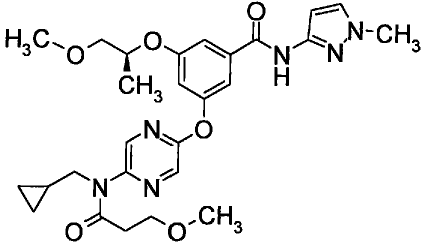 |
| 137 | 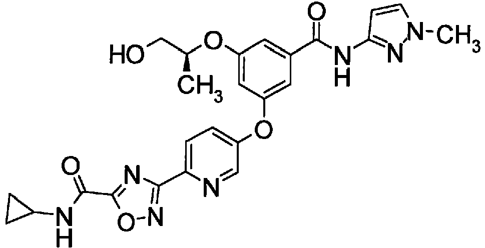 |
| 138 | 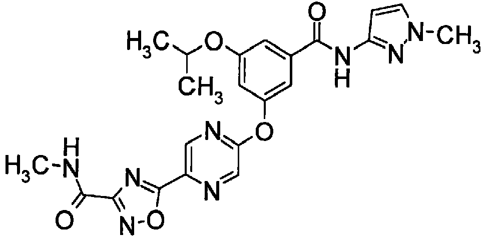 |

**[Table 86]**

| Ex | Structure |
|---|---|
| 139 | 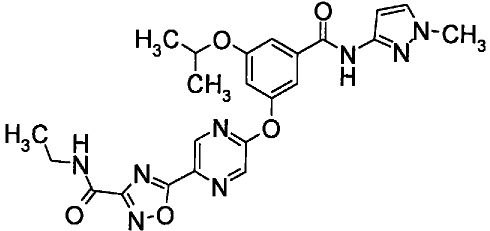 |
| 140 | 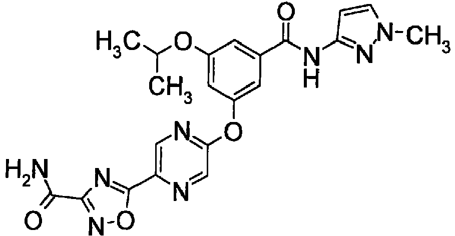 |
| 141 | 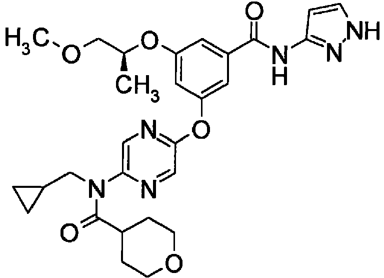 |
| 142 | 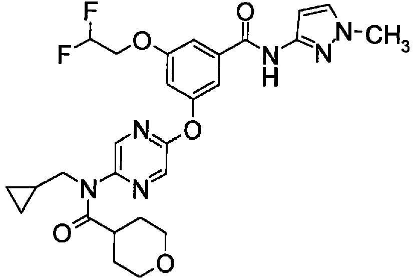 |

**[Table 87]**

| Ex | Structure |
|---|---|
| 143 | 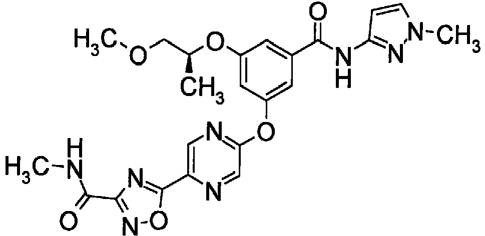 |
| 144 | 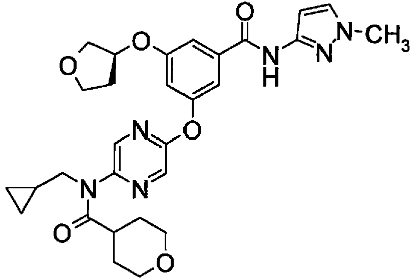 |
| 145 | 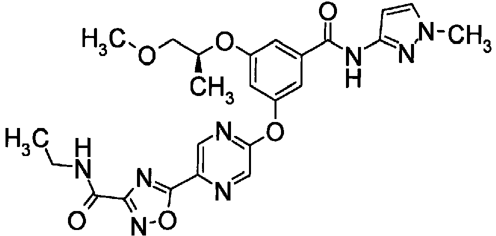 |
| 146 | 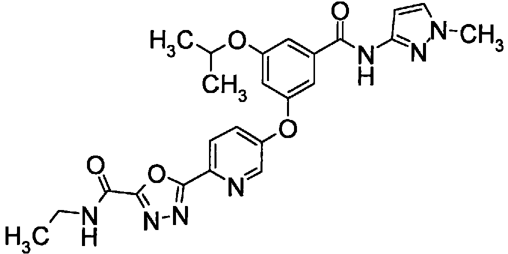 |

**[Table 88]**

| Ex | Structure |
|---|---|
| 147 | 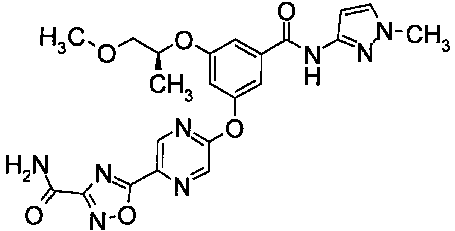 |
| 148 | 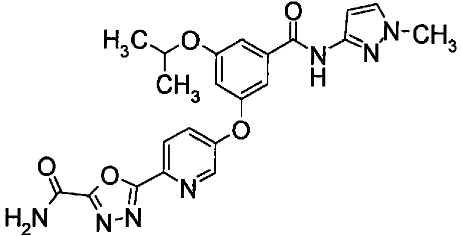 |
| 149 | 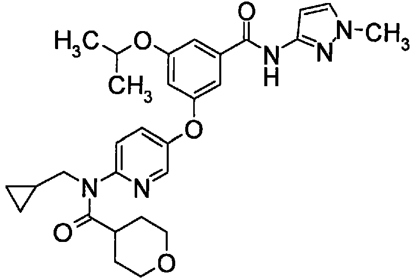 |
| 150 | 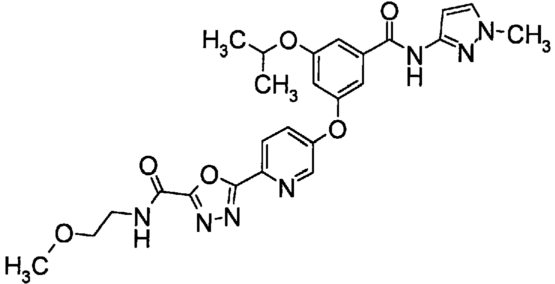 |

**[Table 89]**

| Ex | Structure |
|---|---|
| 151 | 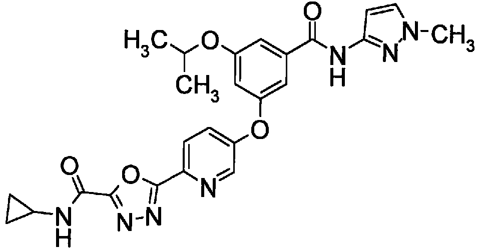 |
| 152 | 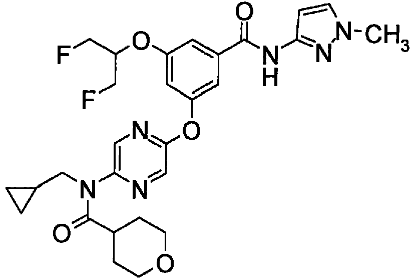 |
| 153 | 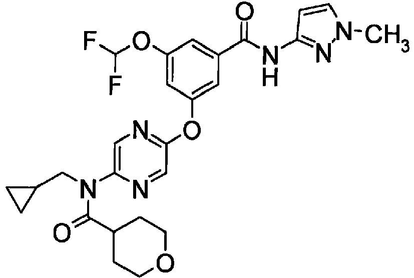 |
| 154 | 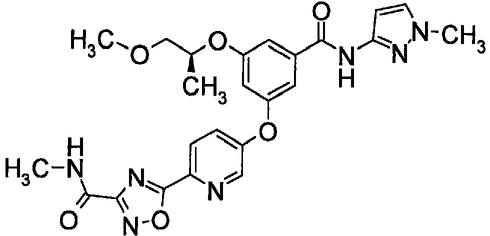 |

**[Table 90]**

| Ex | Structure |
|---|---|
| 155 | 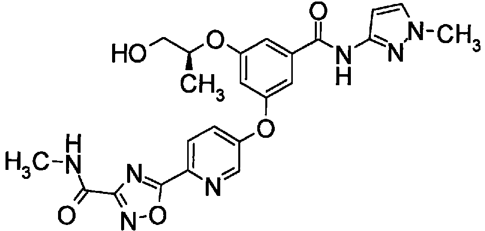 |
| 156 | 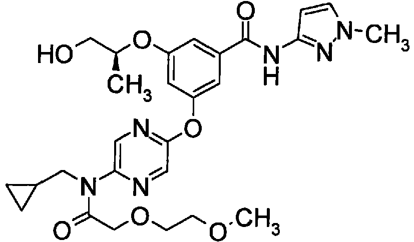 |
| 157 | 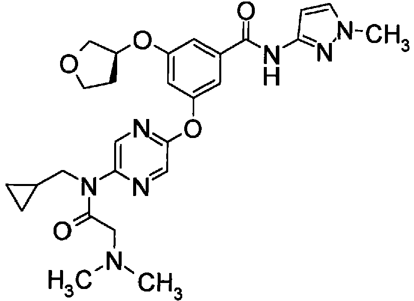 |
| 158 | 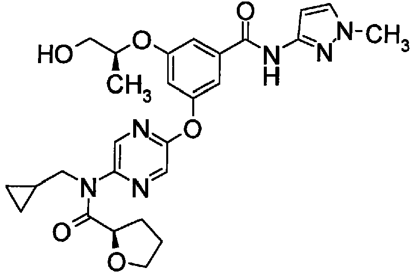 |

**[Table 91]**

| Ex | Structure |
|---|---|
| 159 | 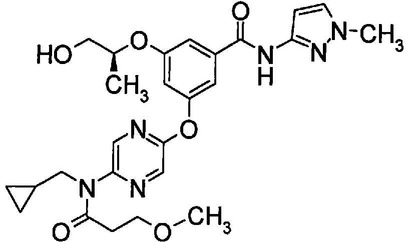 |
| 160 | 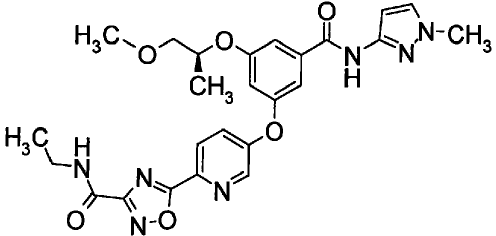 |
| 161 | 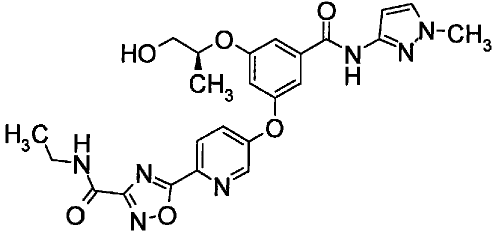 |
| 162 | 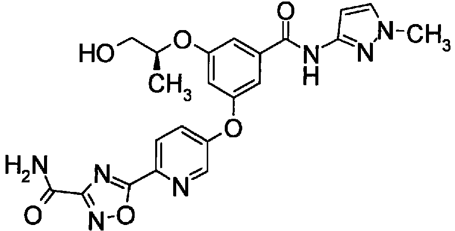 |

**[Table 92]**

| Ex | Structure |
|---|---|
| 163 | 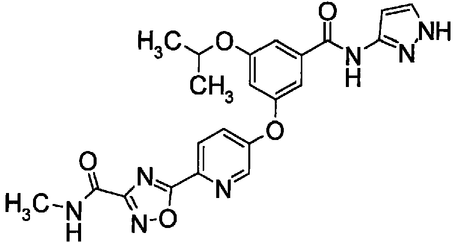 |
| 164 | 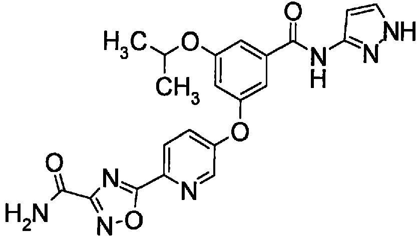 |
| 165 | 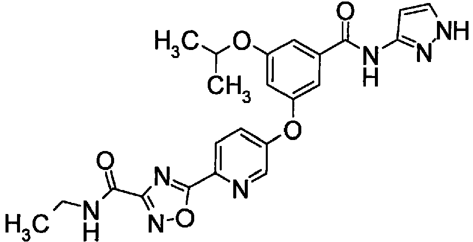 |
| 166 | 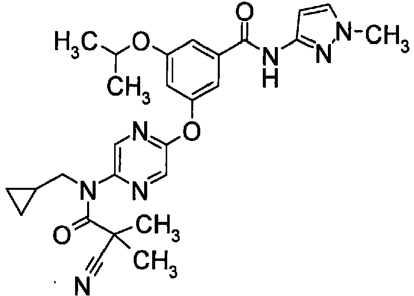 |

**[Table 93]**

| Ex | I Structure |
|---|---|
| 167 | 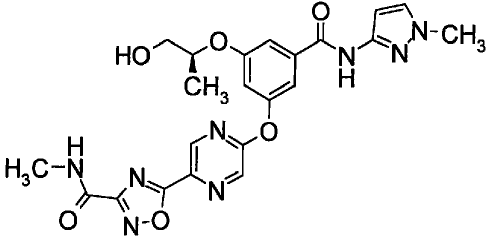 |
| 168 | 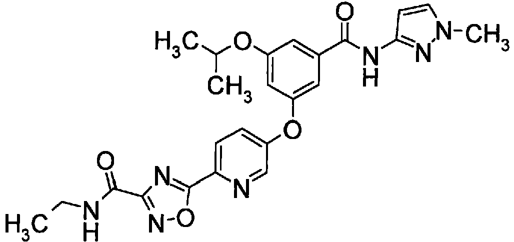 |
| 169 | 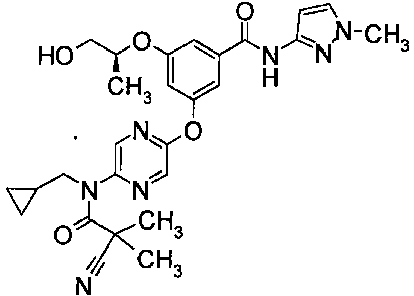 |
| 170 | 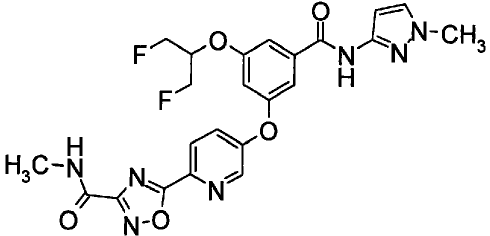 |

**[Table 94]**

| Ex | Structure |
|---|---|
| 171 | 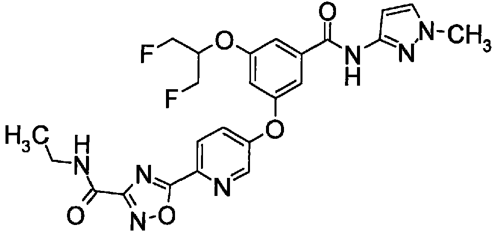 |
| 172 | 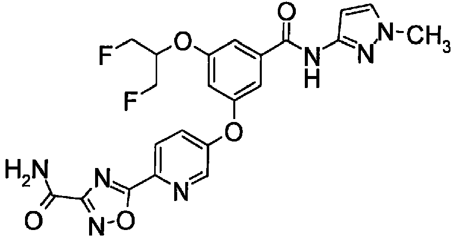 |
| 173 | 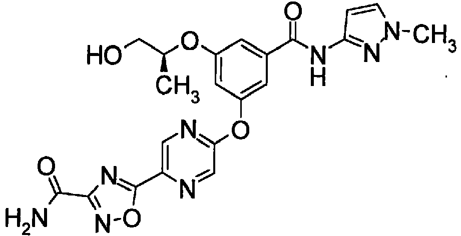 |
| 174 | 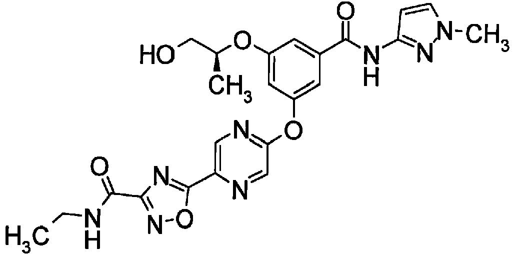 |

**[Table 95]**

| Ex | I Structure |
|---|---|
| 175 | 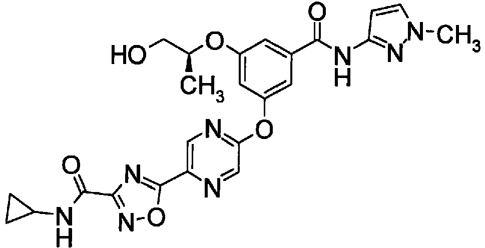 |
| 176 | 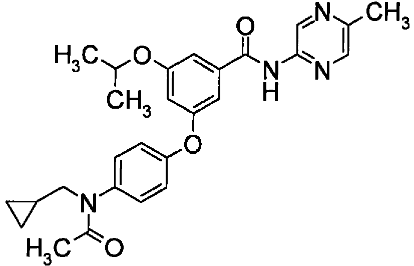 |
| 177 | 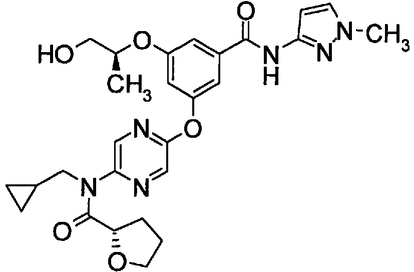 |

**[Table 96]**

| PEx | PSyn | Data |
|---|---|---|
| 1 | 1 | ESI+:379 |
| 2 | 2 | ESI+:412 |
| 3 | 3 | ESI+:494 |
| 4 | 4 | FAB-:238 |
| 5 | 5 | ESI+:319 |
| 6 | 6 | NMR2:1.53(9H,s),3.90(3H,s),5.10(2H,s),6.58(1H,brs),7. 20-7.56(8H,m) |
| 7 | 7 | ESI+:412 |
| 8 | 8 | EI:322 |
| 9 | 9 | FAB+:423 |
| 10 | 10 | EI:322 |
| 11 | 11 | ESI+:365 |
| 12 | 12 | ESI+:238 |
| 13 | 13 | ESI+:493 |
| 14 | 14 | ESI+:397 |
| 15 | 15 | ESI+:411 |
| 16 | 16 | ESI+:511 |
| 17 | 17 | ESI+:493 |
| 18 | 18 | NMR1:1.31(3H,t,J=7.1Hz),4.32(2H,q,J=7.1Hz),7.35(2H, bs),8.99(1H,s),9.42(1H,s) |
| 19 | 19 | ESI+:255,257 |
| 20 | 20 | ESI+:422 |
| 21 | 21 | ESI+:408 |
| 22 | 22 | ESI+:522 |
| 23 | 23 | ESI+:432 |
| 24 | 24 | ESI+:635 |
| 25 | 25 | ESI+:439 |
| 26 | 26 | ESI+:398 |
| 27 | 27 | ESI+:512 |
| 28 | 28 | ESI+:412 |
| 29 | 29 | ESI+:383 |
| 30 | 30 | ESI+:479 |
| 31 | 31 | ESI+:256 |
| 32 | 32 | ESI+:156 |
| 33 | 33 | EI:223 |
| 34 | 34 | ESI+:223 |
| 35 | 35 | NMR2:0.47(2H,m),0.58(2H,m),1.13(1H,m),1.32(6H,d),2. 97(2H,d),3.87(3H,s),4.56(1H,m),6.58-6.67(3H,m),6.88-6. 93(2H,m),7.12(1H,m),7.21(1H,m) |
| 36 | 36 | ESI+:398 |
| 37 | 37 | ESI+:384 |

**[Table 97]**

| PEx | PSyn | Data |
|---|---|---|
| 38 | 38 | NMR2:3.82(3H,s),6.83(1H,dd,J=2.4,2.4Hz),7.07(1H,dd,J =2.4,2.4Hz),7.20(2H,d,J=9.2Hz),7.27(1H,dd,J=2.4,2.4H z),8.27(2H,d),10.36(1H,br) |
| 39 | 39 | ESI+:478 |
| 40 | 40 | ESI+:448 |
| 41 | 41 | APCI/ESI+:390 |
| 42 | 42 | NMR2:1.32(6H,d,J=6.0Hz),1.52(9H,s),3.87(3H,s),4.56(1 H,m),6.47(1H,brs),6.68(1H,m),6.97(2H,m),7.16(1H,m),7. 26(1H,m),7.35(2H,m) |
| 43 | 43 | NMR2:0.11-0.17(2H,m),0.41-0.48(2H,m),0.85-0.89(1H, m),1.33(6H,d,J=6.0Hz),1.44(9H,s),3.48(2H,d,J=7.0Hz),3. 87(3H,s),4.55(1H,m),6.72-6.74(1H,m),6.96-6.99(2H,m), 7.18-7.22(1H,m),7.25-7.27(1H,m),7.29-7.31(2H,m) |
| 44 | 44 | ESI+:478 |
| 45 | 45 | ESI+:446 |
| 46 | 46 | ESI+:454 |
| 47 | 47 | ESI+:440 |
| 48 | 48 | ESI+:554 |
| 49 | 49 | ESI+:424 |
| 50 | 50 | ESI+:579 |
| 51 | 51 | ESI+:464 |
| 52 | 52 | ESI+:366,368 |
| 53 | 53 | ESI+:333 |
| 54 | 54 | ESI+:304(-Boc) |
| 55 | 55 | ESI+:400 |
| 56 | 56 | NMR2:0.06-0.12(2H,m),0.47(2H,m),0.88-0.96(1H,m),1.1 0(6H,d,J=6.6Hz),1.37(6H,d,J=6.0Hz),2.44(1H,m),3.67(2 H,d,J=7.0Hz),3.91(3H,s),4.58-4.67(1H,m),6.92-6.94(1H, m),7.42-7.44(1H,m),7.46-7.48(1H,m),8.08(1H,d,J=1.3H z),8.33(1H,d,J=1.3Hz) |
| 57 | 57 | NMR2:0.05-0.12(2H,m),0.40-0.48(2H,m),0.95-0.99(1H, m),1.11(6H,d,J=6.6Hz),1.39(6H,d,J=6.0Hz),1.65(9H,s),2. 44-2.47(1H,m),3.69(2H,d,J=7.1Hz),4.59-4.69(1H,m),6.9 0-6.96(1H,m),7.11(1H,d,J=2.9Hz),7.23-7.26(1H,m),7.27-7.31(1H,m),8.03(1H,d,J=2.9Hz),8.09(1H,br),8.38(1H,br), 8.62(1H,br) |
| 58 | 58 | FAB+:380 |
| 59 | 59 | ESI+:356 |
| 60 | 60 | ESI+:448 |
| 61 | 61 | ESI+:479 |
| 62 | 62 | ESI+:353,355 |
| 63 | 63 | ESI+:364,366 |

**[Table 98]**

| PEx | PSyn | Data |
|---|---|---|
| 64 | 64 | ESI+:414 |
| 65 | 65 | ESI+:437 |
| 66 | 66 | ESI+:416 |
| 67 | 67 | ESI+:330 |
| 68 | 68 | NMR2:0.23(2H,m),0.55(2H,m),1.04(1H,m),2.96(2H,d),3. 89(3H,s),5.06(2H,s),6.40(1H,m),6.92(1H,m),7.00(1H,m), 7.30-7.50(5H,m) |
| 69 | 69 | CI+:354 |
| 70 | 70 | EI:263 |
| 71 | 71 | ESI+:509 |
| 72 | 72 | ESI+:498 |
| 73 | 73 | ESI+:470 |
| 74 | 74 | ESI+:453 |
| 75 | 75 | ESI+:651 |
| 76 | 76 | ESI+:303(-Boc) |
| 77 | 77 | NMR2:0.21-0.24(2H,m),0.40-0.45(2H,m),1.13-1.19(1H, m),1.34(6H,d,J=6.0Hz),1.52(9H,s),3.81(2H,d,J=7.0Hz),4. 55-4.62(1H,m),6.75-6.77(1H,m),7.26-7.27(1H,m),7.31-7. 36(2H,m),7.60-7.62(1H,m),8.17-8.18(1H,m) |
| 78 | 78 | ESI+:366 |
| 79 | 79 | ESI+:467 |
| 80 | 52 | ESI+:333(-tBu) |
| 81 | 49 | NMR2:0.25-0.34(2H,m),0.54-0.62(2H,m),1.06-1.15(1H, m),3.17(2H,brd,J=6.8Hz),3.87(3H,s),4.69(1H,br),6.13(1 H,br),6.72-6.74(1H,m),7.06-7.08(1H,m),7.20-7.24(1H, m),7.55(1H,d,J=1.3Hz),7.91(1H,d,J=1.3Hz) |
| 82 | 35 | ESI+:502 |
| 83 | 36 | NMR2:1.32(6H,d,J=6.1Hz),2.19(3H,s),3.87(3H,s),4.57(1 H,m),6.69-6.71(1H,m),6.97-7.01(2H,m),7,16-7.18(1H, m),7.25-7.27(1H,m),7.46-7.50(2H,m) |
| 84 | 36 | ESI+:544 |
| 85 | 37 | ESI+:530 |
| 86 | 37 | ESI-:374 |
| 87 | 37 | ESI-:440 |
| 88 | 39 | ESI+:362 |
| 89 | 40 | ESI+:332 |
| 90 | 35 | ESI+:386 |
| 91 | 40 | ESI+:336,338 |
| 92 | 36 | ESI+:428 |
| 93 | 37 | ESI+:414 |
| 94 | 37 | ESI+:450 |
| 95 | 35 | ESI+:390 |

**[Table 99]**

| PEx | Psyn | Data |
|---|---|---|
| 96 | 36 | ESI+:432,434 |
| 97 | 37 | ESI+:418 |
| 98 | 50 | ESI+:615 |
| 99 | 43 | NMR2:0.23-0.27(2H,m),0.42-0.49(2H,m),1.10-1.19(1H, m),1.37(6H,d,J=6.0Hz),1.52(9H,s),3.78(2H,d,J=7.0Hz),3. 91(3H,s),4.61(1H,m),6.88-6.90(1H,m),7.36-7.38(1H,m), 7.42-7.44(1H,m),8.17(1H,d,J=1.3Hz),8.47(1H,d,J=1.3H z) |
| 100 | 49 | NMR2:0.26-0.30(2H,m),0.56-0.61(2H,m),1.08-1.16(1H, m),1.35(6H,d,J=6.0Hz),3.10-3.20(2H,m),3.89(3H,s),4.59 (1H,m),4.65(1H,br),6.77-6.79(1H,m),7.19-7.21(1H,m),7. 31-7.33(1H,m),7.57(1H,d,J=1.3Hz),7.95(1H,d,J=1.3Hz) |
| 101 | 50 | ESI+:583,585 |
| 102 | 37 | ESI-:384 |
| 103 | 37 | NMR2:0.98-0.13(2H,m),0.43-0.50(2H,m),0.94-1.00(1H, m),1,13(6H,d,J=6.6Hz),1,41(6H,d,J=6.0Hz),1.86-1.92(1 H,m),2.4(1H,br),3.71(2H,d,J=7.0Hz),4.62-4.69(1H,m), 7.00-7.02(1H,m),7.51-7.53(1H,m),7.53-7.55(1H,m),8.12 (1H,br),8.39(1H,br) |
| 104 | 9 | FAB+:324 |
| 105 | 4 | ESI+:351 |
| 106 | 5 | ESI+:430 |
| 107 | 40 | ESI+:350 |
| 108 | 2 | ESI+:463 |
| 109 | 56 | ESI+:442 |
| 110 | 37 | ESI+:428 |
| 111 | 3 | ESI+:545 |
| 112 | 5 | ESI+:423 |
| 113 | 2 | ESI+:352 |
| 114 | 3 | ESI+:434 |
| 115 | 37 | ESI+:434 |
| 116 | 37 | ESI+:344 |
| 117 | 5 | ESI+:218 |
| 118 | 9 | ESI+:353 |
| 119 | 2 | ESI+:386 |
| 120 | 3 | ESI+:468 |
| 121 | 5 | ESI+:296 |
| 122 | 53 | ESI+:381 |
| 123 | 54 | ESI+:452 |
| 124 | 63 | ESI+:506 |
| 125 | 9 | ESI+:397 |
| 126 | 9 | ESI+:321 |
| 127 | 2 | ESI+:354 |
| 128 | 3 | ESI+:436 |

**[Table 100]**

| PEx | PSyn | Data |
|---|---|---|
| 129 | 2 | ESI+:430 |
| 130 | 3 | ESI+:512 |
| 131 | 54 | ESI+:424,426 |
| 132 | 43 | ESI+:478,480 |
| 133 | 5 | ESI+:252 |
| 134 | 5 | ESI+:443,445 |
| 135 | 9 | ESI+:355 |
| 136 | 2 | ESI+:388 |
| 137 | 9 | ESI+:378 |
| 138 | 3 | ESI+:470 |
| 139 | 9 | ESI+:442 |
| 140 | 53 | ESI+:386 |
| 141 | 54 | ESI-:455 |
| 142 | 63 | ESI+:511 |
| 143 | 37 | ESI+:397 |
| 144 | 5 | ESI+:476 |
| 145 | 2 | ESI+:411 |
| 146 | 3 | ESI+:493 |
| 147 | 63 | ESI+:300 |
| 148 | 63 | ESI+:392 |
| 149 | 5 | ESI+:379 |
| 150 | 63 | ESI+:316 |
| 151 | 5 | ESI+:471 |
| 152 | 5 | ESI+:395 |
| 153 | 5 | ESI+:409 |
| 154 | 49 | ESI+:411 |
| 155 | 37 | ESI+:484 |
| 156 | 9 | ESI+:408 |
| 157 | 2 | ESI+:444 |
| 158 | 3 | NMR2:1.32(3H,d),1.97-2.07(2H,m),2.35-2.41(3H,t),2.85 (3H,s),3.41(3H,s),3.47-3.61(2H,m),3.81(3H,s),4.08-4.16 (2H,m),4.55-4.67(1H,m),6.78(1H,m),6.83(1H,m),7.13(1 H,m),7.25-7.32(1H,m),7.42(1H,dd),8.18(1H,d),8.38(1H,b rs),8.62(1H,d) |
| 159 | 73 | ESI+:500 |
| 160 | 9 | ESI+:494 |
| 161 | 9 | ESI+:650 |
| 162 | 9 | ESI-:345 |
| 163 | 39 | ESI+:530 |
| 164 | 9 | NMR2:1.35(3H,d),1.48(3H,t),3.42(3H,s),3.50-3.65(2H, m),3.82(3H,s),4.51-4.70(3H,m),6.80(1H,d),6.98(1H,m),7. 28(2H,m),7.49(1H,m),8.38(1H,brs),8.63(1H,s),9.05(1H, d) |
| 165 | 9 | ESI+:411 |

**[Table 101]**

| PEx | PSyn | Data |
|---|---|---|
| 166 | 39 | ESI+:386 |
| 167 | 39 | ESI+:394(-Boc) |
| 168 | 52 | NMR2:1.35(6H,d,J=6.1Hz),1.64(9H,s),3.89(3H,s),4.55-4. 63(1H,m),6.77-6.78(1H,m),7.24-7.25(1H,m),7.31-7.35(1 H,m),7.39-7.41(1H,m),8.03-8.06(1H,m),8.49-8.50(1H,m) |
| 169 | 9 | NMR2:1.61(9H,s),3.89(3H,s),6.72-6.73(1H,m),7.26-7.42 (3H,m),8.06-8.08(1H,m),8.28-8.29(1H,m) |
| 170 | 37 | ESI+:516 |
| 171 | 5 | ESI+:595 |
| 172 | 49 | ESI+:366 |
| 173 | 37 | ESI+:352 |
| 174 | 5 | ESI+:431 |
| 175 | 9 | ESI+:454 |
| 176 | 49 | ESI+:394 |
| 177 | 37 | ESI+:380 |
| 178 | 5 | ESI+:459 |
| 179 | 49 | ESI+:386 |
| 180 | 37 | ESI+:372 |
| 181 | 5 | ESI+:451 |
| 182 | 39 | ESI+:380(-Boc) |
| 183 | 49 | ESI+:380 |
| 184 | 37 | ESI+:366 |
| 185 | 5 | ESI+:445 |
| 186 | 49 | NMR2:0.25-0.29(2H,m),0.54-0.59(2H,m),1.10-1.13(1H, m),1.33(6H,d,J=6.0Hz),3.11-3.14(2H,m),3.80(3H,s),4.54-4.64(2H,m),6.39-6.42(1H,m),6.62-6.63(1H,m),6.78-6.79 (1H,m),6.88-6.89(1H,m),7.06-7.07(1H,m),7.17-7.20(1H, m),7.27-7.28(1H,m),7.93-7.94(1H,m),8.35(1H,brs) |
| 187 | 9 | NMR2:1.33(3H,d,J=6.2Hz),1.48(3H,t,J=7.2Hz),3.74-3.80 (5H,m),4.52-4.61(3H,m),6.79-6.80(1H,m),6.96-6.97(1H, m),7.29-7.30(2H,m),7.39-7.40(1H,m),8.48(1H,bs),8.64(1 H,d,J=1.3Hz),9.05(1H,d,J=1.3Hz) |
| 188 | 9 | ESI+:529 |
| 189 | 9 | NMR2:1.33(3H,d,J=6.3Hz),1.47(3H,t,J=7.2Hz),3.40(3H, s),3.44-3.67(2H,m),3.82(3H,s),4.46-4.71(3H,m), 6.79(1 H,m),6.86(1H,m),7.16(1H,m),7.22-7.37(2H,m), 7.42(1H, dd,J=5.7,8.7Hz),8.32(1H,d,J=8.7Hz),8.50-8.65(2H,m) |
| 190 | 49 | ESI+:388 |
| 191 | 57 | ESI+:674 |
| 192 | 50 | ESI+:649 |
| 193 | 5 | NMR2:0.22-0.27(2H,m),0.40-0.46(2H,m),1.14-1.20(1H, m),1.34(6H,d,J=6.1Hz),1.52(9H,s),3.80-3.83(5H,m),4.53-4.61(1H,m),6.67-6.69(1H,m),6.78-6.79(1H,m),7,02-7.03 (1H,m),7.14-7.15(1H,m),7.26-7.35(2H,m),7.60-7.63(1H, m),8.17-8.18(1H,m),8.39(1H,brs) |

**[Table 102]**

| Ex | Syn | Data |
|---|---|---|
| 1 | 1 | NMR2:1.26(3H,d,J=6.4Hz),3.30(3H,s),3.45-3.56(2H,m),3.7 7(3H,s),4.75-4.83(1H,m),6.57(1H,d,J=2.4Hz),7.04(1H,t,J=2. 4Hz),7.34-7.40(1H,m),7.50-7.54(1H,m),7.60(1H,d,J=2.4Hz), 7.69(1H,dd,J=3.2,8.8Hz),8.18(1H,brs),8.31(1H,d,J=8.8Hz), 8.44(1H,brs),8.69(1H,d,J=3.2Hz) ESI+:494 |
| 2 | 2 | NMR1:1.31(6H,d,J=6.0Hz),2.82(3H,d,J=4.7Hz),3.77(3H,s), 4.73-4.80(1H,m),6.57(1H,d,J=2.2Hz),6.99-7.01(1H,m),7.35-7.37(1H,m),7.48-7.50(1H,m),7.60(1H,d,J=2.2Hz),7.68(1H,d d,J=2.8,8.7Hz),8.30(1H,d,J=8.6Hz),8.69(1H,d,J=2.9Hz),9.0 2-9.07(1H,m),10.88(1H,s) ESI+:478 |
| 3 | 3 | NMR1:1.31(6H,d,J=6.0Hz),2.83(3H,br),3.77(3H,s),4.77(1H, m),6.57(1H,d,J=2.3Hz),6.98(1H,dd,J=2.2,2.2Hz),7.34(1H,b r),7.47(1H,br),7.60(1H,d,J=2.3Hz),7.67(1H,dd,J=8.8,2.8Hz), 8.27(1H,d,J=8.8Hz),8.66(1H,d,J=2.8Hz),9.31(1H,br),10.89 (1H,s) ESI+:478 |
| 4 | 4 | ESI+:534 |
| 5 | 5 | ESI+:445 |
| 6 | 6 | ESI+:426 |
| 7 | 7 | NMR2:1.31(3H,d,J=6.4Hz),2.33(1H,t,J=4.8Hz),3.08(3H,d,J =4.8Hz),3.70-3.83(5H,m),4.52-4.62(1H,m),6.75-6.85(2H, m),7.00-7.11(1H,m),7.26-7.32(2H,m),7.37-7.51(2H,m),8.15 (1H,d,J=8.8Hz),8.57(1H,d,J=2.8Hz),8.80(1H,brs) |
| 8 | 8 | ESI+:467 |
| 9 | 9 | NMR1:1.32(3H,d,J=6.0Hz),3.77(3H,s),4.70-4.85(1H,m),6.5 7(1H,m),7.00(1H,m),7.35(1H,m),7.47(1H,m),7.62(1H,m),7. 68(1H,m),8.17(1H,brs),8.30(1H,d,J=8.8Hz),8.43(1H,brs),8.6 7(1H,m) |
| 10 | 10 | NMR1:1.31(6H,d,J=6.0Hz),3.07(6H,s),3.77(3H,s),4.72-4.81 (1H,m),6.57(1H,d,J=2.2Hz),6.98-7.01(1H,m),7.33-7.36(1H, m),7.46-7.50(1H,m),7.60(1H,d,J=2.2Hz),7.67(1H,dd,J=2.8, 8.7Hz),8.31(1H,d,J=8.8Hz),8.67(1H,d,J=2.8Hz),10.88(1H,s) ESI+:492 |
| 11 | 11 | ESI+:467 |
| 12 | 12 | ESI+:460 |
| 13 | 13 | ESI+:478 |
| 14 | 14 | ESI+:461 |
| 15 | 15 | ESI+:464 |
| 16 | 16 | ESI+:521 |

**[Table 103]**

| Ex | Syn | Data |
|---|---|---|
| 17 | 17 | NMR1:1.56-1.86(2H,m),2.17-2.33(4H,m),2.83(3H,d,J=4.7H z),3.69-3.79(5H,m),4.98(1H,t,J=5.6Hz),6.56(1H,d,J=2.2Hz), 6.88-6.91(1H,m),7.31-7.35(1H,m),7.40-7.44(1H,m),7.60(1 H,d,J=2.2Hz),7.65-7.71(1H,m),8.29-8.33(1H,m),8.68(1H,d,J =2.8Hz),9.01-9.08(1H,m),10.85(1H,s) ESI+:520 |
| 18 | 18 | NMR1:1.31(6H,d,J=6.0Hz),2.84(3H,d,J=4.7Hz),3.78(3H,s), 4.71-4.80(1H,m),6.58(1H,d,J=2.2Hz),7.08-7.10(1H,m),7.45-7.47(1H,m),7.50-7.53(1H,m),7.60(1H,d,J=2.2Hz),8.80(1H,d, J=1.3Hz),9.0 (1H,d,J=1.4Hz),9.30-9.36(1H,m),10.86(1H,s) ESI+:479 |
| 19 | 19 | ESI+:508 |
| 20 | 20 | ESI+:466 |
| 21 | 21 | ESI+:480 |
| 22 | 22 | ESI+:518 |
| 23 | 23 | ESI+:535 |
| 24 | 24 | NMR2:0.08-0.13(2H,m),0.42-0.48(2H,m),0.90-0.99(1H,m), 1.33(3H,d,J=6.2Hz),1.60-1.64(1H,m),1.8 8-2.01(2H,m),2.02-2.07(1H,m),2.41(1H,m),3.20-3.31(2H,m),3.68(2H,d,J=7.1H z),3.73-3.82(2H,m),3.82(3H,s),3.92-3.96(2H,m),4.52-4.57(1 H,m),6.79-6.81(1H,d,J=2.2Hz),6.96-6.99(1H,m),7.29-7.32(2 H,m),7.36-7.38(1H,m),8.01(1H,br),8.37(1H,br),8.49(1H,br) ESI+:551 |
| 25 | 25 | ESI+:479 |
| 26 | 26 | ESI+:522 |
| 27 | 27 | APCI/ESI+:518 |
| 28 | 28 | ESI+:465 |
| 29 | 29 | NMR2:0.11-0.17(2H,m),0.42-0.49(2H,m),0.95-1.03(1H,m), 1.35(3H,d,J=6.3Hz),2.20(6H,s),3.05(2H,s),3.41(3H,s),3.51(1 H,dd,J=4.1,10.4Hz),3.60(1H,dd,J=6.0,10.4Hz),3.73(2H,d,J= 7.1Hz),3.82(3H,s),4.62(1H,ddq,J=4.1,6.0,6.3Hz),6.80(1H,d,J =2.3Hz),6.94-6.97(1H,m),7.26-7.28(1H,m),7.29(1H,d,J=2.3 Hz),7.34-7.36(1H,m),8.14(1H,br),8.30(1H,d,J=1.3Hz),8.49 (1H,br) ESI+:538 |
| 30 | 30 | APCI/ESI+:526 |
| 31 | 31 | ESI+:482 |
| 32 | 32 | ESI+:495 |
| 33 | 33 | ESI+:490 |
| 34 | 34 | ESI+:504 |
| 35 | 35 | ESI+:556 |
| 36 | 36 | ESI+:492 |
| 37 | 37 | ESI+:563 |
| 38 | 38 | ESI+:493 |

**[Table 104]**

| Ex | Syn | Data |
|---|---|---|
| 39 | 39 | ESI+:521 |
| 40 | 40 | ESI+:524 |
| 41 | 20 | ESI+:612 |
| 42 | 20 | ESI+:609 |
| 43 | 24 | ESI+:508 |
| 44 | 24 | ESI+:505 |
| 45 | 20 | ESI+:458 |
| 46 | 26 | ESI+:519 |
| 47 | 20 | ESI+:524 |
| 48 | 21 | ESI+:502 |
| 49 | 21 | ESI+:496 |
| 50 | 21 | ESI+:492 |
| 51 | 21 | ESI+:494 |
| 52 | 21 | ESI+:482 |
| 53 | 21 | ESI+:524 |
| 54 | 20 | ESI+:463 |
| 55 | 20 | ESI+:566 |
| 56 | 20 | ESI+:481 |
| 57 | 29 | ESI+:509 |
| 58 | 20 | ESI+:493 |
| 59 | 20 | ESI+:529 |
| 60 | 25 | ESI+:515 |
| 61 | 28 | ESI+:501 |
| 62 | 25 | ESI+:483,485 |
| 63 | 20 | ESI+:465 |
| 64 | 20 | ESI+:493 |
| 65 | 25 | ESI+:479 |
| 66 | 22 | ESI+:507 |
| 67 | 2 | NMR2:1.38(6H,d,J=6.0Hz),3.20(3H,s),3.31(3H,s),3.81(3H, s),4.58-4.66(1H,m),6.80(1H,brd,J=2Hz),6.90(1H,t,2.4Hz),7. 24-7.26(1H,m),7.29(1H,brd,J=2.4Hz),7.31-7.33(1H,m),8.41 (1H,brs),8.61(1H,d,J=1.2Hz),8.91(1H,d,J=1.2Hz) ESI+:493 |
| 68 | 1 | ESI+:465 |
| 69 | 2 | ESI+:433 |
| 70 | 1 | ESI+:516 |
| 71 | 2 | ESI+:544 |
| 72 | 22 | ESI+:533 |
| 73 | 22 | ESI+:528 |
| 74 | 22 | ESI+:535 |
| 75 | 22 | ESI+:528 |
| 76 | 22 | ESI+:523 |
| 77 | 22 | ESI+:479 |

**[Table 105]**

| Ex | Syn | Data |
|---|---|---|
| 78 | 22 | ESI+:495 |
| 79 | 20 | ESI+:513 |
| 80 | 22 | ESI+:481 |
| 81 | 22 | ESI+:491 |
| 82 | 29 | ESI+:508 |
| 83 | 2 | ESI+:467 |
| 84 | 1 | ESI+:405 |
| 85 | 5 | ESI+:459 |
| 86 | 5 | ESI+:419 |
| 87 | 22 | ESI+:493 |
| 88 | 22 | ESI+:528 |
| 89 | 23 | ESI+:509 |
| 90 | 23 | ESI+:505 |
| 91 | 22 | ESI+:505 |
| 92 | 22 | ESI+:519 |
| 93 | 2 | ESI+:435 |
| 94 | 23 | ESI+:548 |
| 95 | 2 | ESI+:511 |
| 96 | 22 | ESI+:509 |
| 97 | 22 | ESI+:528 |
| 98 | 2 | NMR1:1.31(6H,d,J=6.4Hz),3.09(3H,s),3.36(3H,s),3.78(3H, s),4.72-4.78(1H,m),6.58(1H,d,J=2.4Hz),7.09(1H,t,2Hz),7.4 4-7.47(1H,m),7.50-7.53(1H,m),7.60(1H,d,J=2.4Hz),8.81(1 H,d,J=1.2Hz),9.01(1H,d,J=1.2Hz),10.86(1H,brs) ESI+:493 |
| 99 | 2 | ESI+:469 |
| 100 | 23 | ESI+:555,557 |
| 101 | 23 | ESI+:588 |
| 102 | 4 | NMR1:0.66-0.81(4H,m),1.31(6H,d,J=5.6Hz),2.89-2.96(1H, m),3.77(3H,s),4.70-4.79(1H,m),6.57(1H,d,J=2.4Hz),7.08(1 H,brt,J=2Hz),7.45(1H,brt,J=2Hz),7.51(1H,brt,J=2Hz),7.60(1 H,d,J=2,4Hz),8.78(1H,d,J=1.2Hz),8.87(1H,d,J=1.2Hz),9.61 (1H,brs),10.85(1H,brs) ESI+:505 |
| 103 | 22 | ESI+:532 |
| 104 | 22 | ESI+:532 |
| 105 | 22 | ESI+:532 |
| 106 | 23 | ESI+:529 |
| 107 | 23 | ESI+:534 |
| 108 | 23 | ESI+:534 |

**[Table 1061**

| Ex | Syn | Data |
|---|---|---|
| 109 | 2 | NMR2:1.36(6H,d,J=6.0Hz),3.20(3H,s),3.32(3H,s),3.81(3H, s),4.57-4.64(1H,m),6.76-6.80(2H,m),7.09-7.11(1H,m),7.23-7.25(1H,m),7.29(1H,d,J=2.4Hz),7.43(1H,d,J=2.8Hz),7.45(1 H,d,J=2.8Hz),8.14(1H,d,J=1Hz),8.16(1H,d,J=1Hz),8.40(1H, brs),8.61(1H,d,J=2.8Hz) ESI+:492 |
| 110 | 23 | ESI+:521 |
| 111 | 23 | ESI+:521 |
| 112 | 23 | ESI+:521 |
| 113 | 23 | ESI+:491 |
| 114 | 25 | ESI+:574 |
| 115 | 23 | ESI+:507 |
| 116 | 23 | NMR1:0.00-0.15(2H,m),0.28-0.45(2H,m),0.80-1.00(1H,m), 1.36(3H,t,J=7.0Hz),1.47-1.73(4H,m),3.07-3.42(3H,m),3.60 (2H,d,J=7.0Hz),3.72-3.84(5H,m),4.14(2H,q,J=7.0Hz),6.58(1 H,d,J=2.3Hz),7.05(1H,s),7.44(1H,s),7.51(1H,s),7.61(1H,d,J= 2.3Hz),8.34(1H,s),8.53(1H,s),10.88(1H,s) ESI+:521 |
| 117 | 4 | NMR1:0.65-0.76(4H,m),1.31(6H,d,J=6.0Hz),2.87-2.95(1H, m),3.77(3H,s),4.72-4.81(1H,m),6.57(1H,d,J=2.3Hz),6.99-7.0 1(1H,m),7.34-7.38(1H,m),7.47-7.50(1H,m),7.60(1H,d,J=2.2 Hz),7.67(1H,dd,J=2.9,8.8Hz),8.31(1H,d,J=8.8Hz),8.68(1H, d,J=2.9Hz),9.15-9.18(1H,m),10.88(1H,s) ESI+:504 |
| 118 | 23 | NMR1:0.00-0.08(2H,m),0.28-0.41(2H,m),0.80-1.00(1H,m), 1.47-1.70(4H,m),3.09-3.46(3H,m),3.60(2H,d,J=7.6Hz),3.72-3.80(5H,m),5.22(2H,s),6.58(1H,d,J=2.3Hz),7.16(1H,dd,J=2. 2,2.2Hz),7.32-7.52(6H,m),7.61(1H,d,J=2.3Hz),7.64(1H,dd,J =1.9,1.9Hz),8.34(1H,d,J=1.2Hz),8.53(1H,d,J=1.2Hz),10.90 (1H,s) ESI+:583 |
| 119 | 23 | ESI+:539 |
| 120 | 25 | ESI+:549 |
| 121 | 2 | NMR1:1.30(6H,d,J=6.0Hz),2.82-2.86(3H,m),3.78(3H,s),4.7 2-4.80(1H,m),6.57(1H,d,J=2.2Hz),6.95-6.98(1H,m),7.32-7.3 4(1H,m),7.45-7.48(1H,m),7.60(1H,d,J=2.3Hz),7.67(1H,dd,J =2.9,8.7Hz),8.16(1H,d,J=8.7Hz),8.64(1H,d,J=2.9Hz),9.46(1 H,brs),10.88(1H,brs) ESI+:478 |

**[Table 107]**

| Ex | Syn | Data |
|---|---|---|
| 122 | 4 | NNM1:0.68-0.80(4H,m),1.30(6H,d,J=6.0Hz),2.89-2.96(1H, m),3.77(3H,s),4,71-4,81(1H,m),6.56(1H,d,J=2,2Hz),6.95-6.9 8(1H,m),7.30-7.34(1H,m),7.44-7.48(1H,m),7.60(1H,d,J=2.3 Hz),7.66(1H,dd,J=2.9,8.7Hz),8.16(1H,d,J=8.7Hz),8.63(1H, d,J=2.8Hz),9.59(1H,br),10.87(1H,s) ESI+:504 |
| 123 | 4 | ESI+:534 |
| 124 | 6 | NMR1:1.30(6H,d,J=6.0Hz),3.77(3H,s),4.62-4.67(2H,m),4.7 2-4.81(1H,m),5.78(1H,br),6.57(1H,d,J=2.2Hz),6.98-7.00(1 H,m),7.34-7.36(1H,m),7,46-7.49(1H,m),7.60(1H,d,J=2.2H z),7.64(1H,dd,J=2.8,8.7Hz),8.26(1H,d,J=8.7Hz),8.66(1H,d,J =2.8Hz),10.88(1H,s) ESI+:451 |
| 125 | 28 | ESI+:549 |
| 126 | 28 | ESI+:535 |
| 127 | 23 | ESI+:565 |
| 128 | 4 | NMR1:1.31(6H,d,J=6.0Hz),3.27-3.41(2H,m),3.51-3.57(2H, m),3.77(3H,s),4.73-4.82(2H,m),6.57(1H,d,J=2.2Hz),6.99-7.0 2(1H,m),7.35-7.38(1H,m),7.47-7.50(1H,m),7.60(1H,d,J=2.2 Hz),7.68(1H,dd,J=2.9,8.8Hz),8.32(1H,d,J=8.7Hz),8.69(1H, d,J=2.9Hz),8.92-8.98(1H,m),10.88(1H,s) ESI+:508 |
| 129 | 2 | NMR2:1.33(3H,d,J=6.4Hz),3.08(3H,d,J=5.6Hz),3.40(3H,s), 3.48-3.61(2H,m),3.78(3H,s),4.56-4.64(1H,m),6.79(1H,d,J= 2.0Hz),6.84(1H,t,J=2.0Hz),7.09-7.13(1H,m,J=1.6Hz),7.26-7. 32(2H,m),7.35-7.46(2H,m),8.16(1H,d,J=8.4Hz),8.55(1H,br s),8.59(1H,d,J=2.4Hz) APCI/ESI+:508 |
| 130 | 2 | NMR2:1.33(3H,d,J=6.4Hz),3.20(3H,s),3.32(3H,s),3.40(3H, s),3.47-3.61(2H,m),3.79(3H,s),4.56-4.64(1H,m),6.78(1H,d,J =2.0Hz),6.82-6.86(1H,m),7.11-7.15(1H,m),7.26-7.33(2H, m),7.43(1H,dd,J=3.2,8.8Hz),8.12-8.17(1H,m),8.49(1H,brs), 8.58-8.63(1H,m) ESI+:522 |
| 131 | 4 | ESI+:508 |
| 132 | 23 | ESI+:551 |
| 133 | 23 | ESI+:551 |
| 134 | 23 | ESI+:569 |
| 135 | 1 | ESI+:494 |
| 136 | 23 | ESI+:539 |
| 137 | 7 | ESI+:520 |

**[Table 108]**

| Ex | Syn | Data |
|---|---|---|
| 138 | 2 | NMR1:1.31(6H,d,J=6.0Hz),2.82(3H,d,J=4.7Hz),3.78(3H,s), 4.75(1H,m),6.58(1H,d,J=2.3Hz),7.10(1H,dd,J=2.2,2.2Hz),7. 47(1H,dd,J=2.2,2.2Hz),7.52(1H,dd,J=2.2,2.2Hz),7.61(1H,d,J =2.3Hz),8.84(1H,d,J=1.3Hz),9.04(1H,d,J=1.3Hz),9.08(1H,q, J=4.7Hz),10.88(1H,s) ESI+:479 |
| 139 | 2 | NMR1:1.14(3H.t.J=7.1Hz),1.31(6H,d,J=6.0Hz),3.26-3.45(2 H,m),3.78(3H,s),4.75(1H,m),6.58(1H,d,J=2.3Hz),7.10(1H,d d,J=2.2,2.2Hz),7.47(1H,dd,J=2.2,2.2Hz),7.52(1H,dd,J=2.2,2. 2Hz),7.61(1H,d,J=2.3Hz),8.84(1H,d,J=1.3Hz),9.04(1H,d,J= 1.3Hz),9.16(1H,t,J=5.7Hz),10.88(1H,s) ESI+:493 |
| 140 | 1 | ESI+:465 |
| 141 | 25 | ESI+:551 |
| 142 | 23 | ESI+:557 |
| 143 | 2 | NMR1:1.26(3H,d,J=6.1Hz),2.82(3H,d,J=4.3Hz),3.30(3H,s), 3.45-3.60(2H,m),3.78(3H,s),4.74-4.83(1H,m),6.58(1H,d,J= 2.2Hz),7.14(1H,s),7.47(1H,s),7.56(1H,s),7.61(1H,d,J=2.2H z),8.84(1H,d,J=1.2Hz),9.04(1H,d,J=1.2Hz),9.09(1H,q,J=4.3 Hz),10.89(1H,s) ESI+:509 |
| 144 | 23 | ESI+:563 |
| 145 | 2 | NMR2:1.26-1.38(6H,m),3.41(3H,s),3.48-3.64(4H,m),3.76(3 H,s),4.57-4.65(1H,m),6.80(1H,d,J=2.0Hz),6.95-6.98(1H,m), 7.14-7.20(1H,m),7.25-7.32(2H,m),7.38-7.42(1H,m),8.62(1 H,d,J=1.6Hz),8.83(1H,brs),8.99(1H,d,J=1.2Hz) ESI+:523 |
| 146 | 2 | NMR1:1.15(3H,t,7.2Hz),1.31(6H,d,J=6.0Hz),3,32(2H,m),3. 77(3H,s),4.77(1H,m),6.57(1H,d,J=2.3Hz),6.98(1H,dd,J=2.2, 2.2Hz),7.34(1H,dd,J=2.2,2.2Hz),7.47(1H,dd,J=2.2,2.2Hz),7. 60(1H,d,J=2.3Hz),7.67(1H,dd,J=8.6,2.8Hz),8.27(1H,d,J=8.6 Hz),8.67(1H,d,J=2.8Hz),9.40(1H,d,5.8Hz),10.89(1H,s) ESI+:492 |
| 147 | 1 | ESI+:495 |
| 148 | 1 | NMR1:1.31(6H,d,J=6.0Hz),3.77(3H,s),4.77(1H,m),6.57(1H, d,J=2.3Hz),6.98(1H,dd,J=2.2,2.2Hz),7.34(1H,dd,J=2.2,2.2H z),7.47(1H,dd,J=2.2,2.2Hz),7.60(1H,d,J=2.3Hz),7.67(1H,dd, J=8.6,2.8Hz),8.27(1H,d,J=8.6Hz),8.28(1H,br),8.66(1H,d,J= 2.8Hz),8.70(1H,br),10.89(1H,s) ESI+:464 |

**[Table 109]**

| Ex | Syn | Data |
|---|---|---|
| 149 | 23 | ESI+:534 |
| 150 | 4 | ESI+:522 |
| 151 | 4 | NMR1:0.60-0.83(4H,m),0.98-1.17(1H,m),1.31(6H,d,J=6.2H z),3.77(3H,s),4.77(1H,m),6.57(1H,d,J=2.3Hz),6.98(1H,s),7.3 4(1H,s),7.47(1H,s),7.60(1H,d,J=2.3Hz),7.67(1H,dd,J=8.6,2. 8Hz),8.27(1H,d,J=8.6Hz),8.67(1H,d,J=2.8Hz),9.47(1H,d,3.4 Hz),10.89(1H,s) ESI+:504 |
| 152 | 23 | ESI+:571 |
| 153 | 23 | ESI+:543 |
| 154 | 2 | NMR2:1.33(3H,d,J=6.0Hz),3.07(3H,d,J=4.8Hz),3.40(3H,s), 3.48-3.62(2H,m),3.78(3H,s),4.56-4.65(1H,m),6.78(1H,d,J= 2.0Hz),6.84-6.88(1H,m),7.12-7.36(5H,m),8.25(1H,d,J=8.8H z),8.58-8.67(2H,m) ESI+:508 |
| 155 | 7 | NMR2:1.31(3H,m,J=6.4Hz),2.39(1H,t,J=6.4Hz),3.07(3H,d,J =5.2Hz),3.72-3.84(5H,m),4.53-4.62(1H,m),6.79(1H,d,J=2.0 Hz),6.82-6.86(1H,m),7.11-7.15(1H,m),7.21-7.35(4H,m),7.42 (1H,dd,J=2.8,8.8Hz),8.24(1H,d,J=8.8Hz),8.60(1H,d,J=2.8H z),8.76(1H,brs) ESI+:494 |
| 156 | 24 | ESI+:555 |
| 157 | 29 | ESI+:536 |
| 158 | 24 | ESI+:537 |
| 159 | 24 | ESI+:525 |
| 160 | 2 | NMR2:1.23-1.38(6H,m),3.40(3H,s),3.48-3.64(2H,m),3.80(3 H,s),4.57-4.65(1H,m),6.78(1H,d,J=2.0Hz),6.85-6.89(1H,m), 7.13-7.37(4H,m),7.44(1H,dd,J=3.2,8.8Hz),8.26(1H,d,J=8.8H z),8.55-8.65(2H,m) ESI+:522 |
| 161 | 7 | ESI+:508 |
| 162 | 7 | ESI+:480 |
| 163 | 2 | ESI+:464 |
| 164 | 1 | ESI+:450 |
| 165 | 2 | ESI+:478 |
| 166 | 22 | ESI+:518 |
| 167 | 2 | ESI+:495 |
| 168 | 2 | NMR1:1.14(3H,t,J=7.2Hz),1.31(6H,d,J=6.0Hz),3.28-3.36(2 H,m),3.77(3H,s),4.73-4.80(1H,m),6.57(1H,d,J=2.3Hz),6.99-7.02(1H,m),7.35-7.38(1H,m),7.48-7.50(1H,m),7.60(1H,d,J= 2.3Hz),7.68(1H,dd,J=2.8,8.7Hz),8.31(1H,d,J=8.8Hz),8.69(1 H,d,J=2.9Hz),9.09-9.14(1H,m),10.89(1H,s) ESI+:492 |

**[Table 110]**

| Ex | Syn | Data |
|---|---|---|
| 169 | 24 | ESI+:534 |
| 170 | 2 | NMR2:3.07(3H,d,J=5.2Hz),3.74(3H,s),4.52-4.86(5H,m),6.7 7(1H,d,J=2.0Hz),6.90-6.96(1H,m),7.17-7.51(5H,m),8.24(1 H,d,J=8.8Hz),8.59(1H,d,J=2.8Hz),9.02(1H,brs) |
| 171 | 2 | NMR2:1.28(3H,t,J=7.2Hz),2.05(3H,d,J=8.8Hz),3.48-3.62(2 H,m),3.70(3H,s),4.47-4.89(5H,m),6.72-6.82(1H,m),6.85-7.0 0(1H,m),7.20-7.33(2H,m),7.34-7.49(2H,m),8.23(1H,d,J=8.8 Hz),8.58(1H,d,J=2.8Hz),9.36(1H,brs) ESI+:528 |
| 172 | 1 | ESI+:500 |
| 173 | 1 | ESI+:481 |
| 174 | 2 | ESI+:509 |
| 175 | 2 | ESI+:521 |
| 176 | 27 | ESI+:475 |
| 177 | 24 | ESI+:537 |

### Industrial Applicability

The compound of the formula (I) or a salt thereof has a GK activating action, and can be used as an active ingredient of a pharmaceutical composition for preventing and/or treating diabetes, particularly type 2 diabetes mellitus. Further, it can be used as an active ingredient of a pharmaceutical composition for preventing and/or treating nephropathy, retinopathy, neuropathy, peripheral circulatory disorder, cerebrovascular disorder, ischemic heart disease, and arteriosclerosis that are complications of diabetes mellitus. It can further be used as an active ingredient of a pharmaceutical composition for preventing and/or treating obesity or metabolic syndrome by suppressing overeating.

### Sequence Listing Free Text

Under the number title <223> in the following sequence listing, description of "Artificial Sequence" is provided. Specifically, the base sequences as set forth as SEQ ID NO: 1 and 2 in the sequence listing are artificially synthesized primer sequences.

## Claims

1. A compound of the formula (I) or a salt thereof. 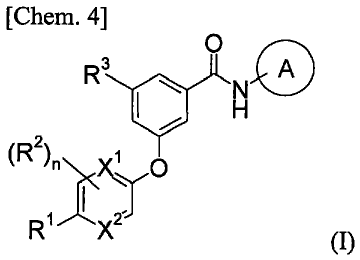 [the symbols in the formula have the following meanings:
Ring A: nitrogen-containing heteroaryl which may be substituted,
X¹ and X²: the same as or different from each other, each representing C(H), C(R²), or N,
R¹: -N(halogeno-lower alkyl)-C(O)-R¹¹, -N(lower alkylene-cycloalkyl)-C(O)-R¹¹, -N(lower alkylene-cycloalkyl)-CO₂R¹¹, -N(lower alkylene-cycloalkyl)-C(O)N(R⁰)(R¹¹), -N(lower alkylene-cycloalkyl)-S(O)₂-R¹¹, or R¹²,
R⁰: the same as or different from each other, each representing -H, or lower alkyl,
R¹¹: lower alkyl, halogeno-lower alkyl, lower alkylene-OR⁰, lower alkylene-O-lower alkylene-OR⁰, lower alkylene-OC(O)-lower alkyl, lower alkylene-CN, lower alkylene-N(R⁰)₂, lower alkylene-cycloalkyl, cycloalkyl, or a heterocyclic group,
provided that the cycloalkyl and the heterocyclic group in R¹¹ may be each substituted,
R¹²: 1,2,4-oxadiazol-3-yl, 1,2,4-oxadiazol-5-yl, or 1,3,4-oxadiazol-2-yl, each of which may be substituted with a group selected from Group G,
provided that when both of X¹ and X² are not N, 1,2,4-oxadiazol-3-yl and 1,2,4-oxadiazol-5-yl in R¹² are substituted with a group selected from Group G,
Group G: lower alkylene-CN, lower alkylene-OR⁰, lower alkylene-N(R⁰)₂, lower alkylene-C(O)N(R⁰)₂, -C(O)N(R⁰)₂, -C(O)N(R⁰)-lower alkylene-OR⁰, -C(O)N(R⁰)-cycloalkyl, -C(O)N(R⁰)-heterocyclic group, -C(O)-heterocyclic group, -N(R⁰)₂, -S(O)ₚ-lower alkyl, and cycloalkyl,
provided that the cycloalkyl and the heterocyclic group in Group G may be substituted,
R²: the same as or different from each other, each representing halogen, lower alkyl, -O-lower alkyl, or -O-halogeno-lower alkyl,
n and p: the same as or different from each other, each representing 0, 1, or 2,
R³: -H, halogen, lower alkyl, halogeno-lower alkyl, -N(R⁰)C(O)-lower alkyl, -N(lower alkylene-cycloalkyl)-C(O)-lower alkyl, or -O-R³¹, and
R³¹: -H, lower alkyl, halogeno-lower alkyl, lower alkylene-OR⁰, lower alkylene-N(R⁰)₂, lower alkylene-S(O)₂-lower alkyl, lower alkylene-C(O)N(R⁰)-S(O)₂-lower alkyl, lower alkylene-cycloalkyl, lower alkylene-aryl, cycloalkyl, or heterocyclic group,
provided that the cycloalkyl, aryl, and heterocyclic groups in R³¹ may be each substituted].

2. The compound according to claim 1, wherein R¹ is -N(lower alkylene-cycloalkyl)-C(O)-R¹¹ or R¹².

3. The compound according to claim 2, wherein Ring A is pyrazol-3-yl, thiazol-2-yl or 1,2,4-thiadiazol-5-yl, each of which may be substituted with lower alkyl which may be substituted with a hydroxyl group.

4. The compound according to claim 3, wherein R³ is -O-lower alkyl, -O-halogeno-lower alkyl, -O-lower alkylene-OR⁰, -O-(cycloalkyl which may be substituted with lower alkylene-OR⁰), -O-lower alkylene-aryl, or -N(lower alkylene-cycloalkyl)-C(O)-lower alkyl.

5. The compound according to claim 4, wherein X¹ is C(H) and X² is N or wherein X¹ is N and X² is N.

6. The compound according to claim 5, wherein n is 0.

7. The compound according to claim 6, wherein R¹ is 1,2,4-oxadiazol-3-yl, 1,2,4-oxadiazol-5-yl, or 1,3,4-oxadiazol-2-yl, each of which is substituted with a group selected from the group consisting of lower alkylene-OR⁰, -C(O)N(R⁰)₂, -C(O)N(R⁰)-lower alkylene-OR⁰, and -C(O)N(R⁰)-cycloalkyl.

8. The compound according to claim 6, wherein R¹ is - N(cyclopropylmethyl)-C(O)-lower alkylene-O-lower alkylene-OR⁰, - N(cyclopropylmethyl)-C(O)-lower alkylene-N(R⁰)₂, or -N(cyclopropylmethyl)-C(O)-(heterocyclic group which may be substituted with a group selected from the group consisting of lower alkyl, halogeno-lower alkyl, -O-R⁰, -O-halogeno-lower alkyl, and oxo).

9. The compound or a salt thereof according to claim 1, which is selected from the group consisting of:
3-(5-{3-isopropoxy-5-[(1-methyl-1H-pyrazol-3-yl)carbamoyl]phenoxy}pyrazin-2-yl)-N,N-dimethyl-1,2,4-oxadiazole-5-carboxamide,
N-(cyclopropylmethyl)-N-(5-{3-isopropoxy-5-[(1-methyl-1H-pyrazol-3-yl)carbamoyl]phenoxy}pyrazin-2-yl)-2-oxoimidazolidine-1-carboxamide,
N-(cyclopropylmethyl)-N-(5-{3-isopropoxy-5-[(1-methyl-1H-pyrazol-3-yl)carbamoyl]phenoxy}pyrazin-2-yl)pyrazine-2-carboxamide,
N-(cyclopropylmethyl)-N-(5- {3-isopropoxy-5-[(1-methyl-1H-pyrazol-3-yl)carbamoyl]phenoxy}pyrazin-2-yl)tetrahydro-2H-pyran-4-carboxamide,
5-(5-{3-isopropoxy-5-[(1-methyl-1H-pyrazol-3-yl)carbamoyl]phenoxy}pyrazin-2-yl)-N,N-dimethyl-1,3,4-oxadiazole-2-carboxamide,
3-[(5-{(cyclopropylmethyl) [(2-methoxyethoxy)acetyl] amino}pyrazin-2-yl)oxy]-5-isopropoxy-N-(1-methyl-1H-pyrazol-3-yl)benzamide,
(2R)-N-(cyclopropylmethyl)-N-(5-{3-isopropoxy-5-[(1-methyl-1H-pyrazol-3-yl)carbamoyl]phenoxy}pyrazin-2-yl)tetrahydrofuran-2-carboxamide, 5-(5-{3-isopropoxy-5-[(1-methyl-1H-pyrazol-3-yl)carbamoyl]phenoxy}pyridin-2-yl)-1,2,4-oxadiazole-3-carboxamide,
5-(5-{3-isopropoxy-5-[(1-methyl-1H-pyrazol-3-yl)carbamoyl]phenoxy}pyridin-2-yl)-N-methyl-1,2,4-oxadiazole-3-carboxamide,
5-(5-{3-isopropoxy-5-[(1-methyl-1H-pyrazol-3-yl)carbamoyl]phenoxy}pyridin-2-yl)-N,N-dimethyl-1,2,4-oxadiazole-3-carboxamide,
N-(5-{3-[acetyl(cyclopropylmethyl)amino]-5-[(1-methyl-1H-pyrazol-3-yl)carbamoyl]phenoxy}pyrazin-2-yl)-N-(cyclopropylmethyl)tetrahydro-2H-pyran-4-carboxamide,
5-(5-{3-isopropoxy-5-[(1-methyl-1H-pyrazol-3-yl)carbamoyl]phenoxy}pyridin-2-yl)-N-methyl-1,3,4-oxadiazole-2-carboxamide,
N-(2-hydroxyethyl)-5-(5-{3-isopropoxy-5-[(1-methyl-1H-pyrazol-3-yl)carbamoyl]phenoxy}pyridin-2-yl)-1,2,4-oxadiazole-3-carboxamide,
3-[5-(3-{[(2S)-1-methoxypropan-2-yl]oxy}-5-[(1-methyl-1H-pyrazol-3-yl)carbamoyl]phenoxy)pyridin-2-yl]-N,N-dimethyl-1,2,4-oxadiazole-5-carboxamide,
5-(5-{3-isopropoxy-5-[(1-methyl-1H-pyrazol-3-yl)carbamoyl]phenoxy}pyrazin-2-yl)-N-methyl-1,2,4-oxadiazole-3-carboxamide,
N-(cyclopropylmethyl)-N-[5-(3-{[(2S)-1-hydroxypropan-2-yl]oxy}-5-[(1-methyl-1H-pyrazol-3-yl)carbamoyl]phenoxy)pyrazin-2-yl]tetrahydro-2H-pyran-4-carboxamide,
5-[5-(3-{[(2S)-1-methoxypropan-2-yl]oxy}-5-[(1-methyl-1H-pyrazol-3-yl)carbamoyl]phenoxy)pyridin-2-yl]-1,2,4-oxadiazole-3-carboxamide,
5-[5-(3-{[(2S)-1-hydroxypropan-2-yl]oxy}-5-[(1-methyl-1H-pyrazol-3-yl)carbamoyl]phenoxy)pyridin-2-yl]-1,2,4-oxadiazole-3-carboxamide,
3-[(5-{(cyclopropylmethyl)[(2-methoxyethoxy)acetyl]amino}pyrazin-2-yl)oxy]-5-{[(2S)-1-hydroxypropan-2-yl]oxy}-N-(1-methyl-1H-pyrazol-3-yl)benzamide,
3-({5-[(cyclopropylmethyl)(N,N-dimethylglycyl)amino]pyrazin-2-yl}oxy)-5-{[(2S)-1-methoxypropan-2-yl]oxy}-N-(1-methyl-1H-pyrazol-3-yl)benzamide, and
(2R)-N-(cyclopropylmethyl)-N-[5-(3-{[(25)-1-hydroxypropan-2-yl]oxy}-5-[(1-methyl-1H-pyrazol-3-yl)carbamoyl]phenoxy)pyrazin-2-yl]tetrahydrofuran-2-carboxamide.

10. A pharmaceutical composition comprising:
the compound or a salt thereof according to claim 1; and
pharmaceutically acceptable excipients.

11. The pharmaceutical composition according to claim 10, which is a GK activator.

12. The pharmaceutical composition according to claim 10, which is a drug for preventing and/or treating diabetes.

13. The pharmaceutical composition according to claim 12, which is a drug for preventing and/or treating type 2 diabetes mellitus.

14. The pharmaceutical composition according to claim 10, which is a drug for preventing and/or treating obesity.

15. The pharmaceutical composition according to claim 10, which is a drug for preventing and/or treating metabolic syndrome.

16. Use of the compound or a pharmaceutically acceptable salt thereof according to claim 1 for the preparation of a GK activator, or a drug for preventing and/or treating diabetes, obesity, or metabolic syndrome.

17. Use of the compound or a pharmaceutically acceptable salt thereof according to claim 1 for preventing and/or treating diabetes, obesity, or metabolic syndrome.

18. A method for preventing and/or treating diabetes, obesity, or metabolic syndrome, comprising administering to a patient a therapeutically effective amount of the compound or a salt thereof according to claim 1.

19. The compound or a pharmaceutically acceptable salt thereof according to claim 1 for preventing and/or treating diabetes, obesity, or metabolic syndrome.


SEQUENCE LISTING

<110> Astellas Pharma Inc.

<120> Benzamide Compound

<130> A10044

<150> JP2009-281350
<151> 2009-12-11

<160> 2

<210> 1
<211> 28
<212> DNA
<213> Artificial

<220>
<223> Description of artificial sequence:primer
<220>
<223> Inventor: Hayakawa, Masahiko; Yoshimura, Seiji; Sasuga, Daisuke;
Inventor: Koike, Takanori; Nigawara, Takahiro;
Inventor: Okumura, Mitsuaki; Maki, Keisuke
<400> 1

TAGAATTCAT GGCGATGGAT GTCACAAG 28

<210> 2
<211> 27
<212> DNA
<213> Artificial

<220>

<223> Description of artificial sequence:primer

<400> 2

ATCTCGAGTC ACTGGCCCAG CATACAG 27